# EUROPÄISCHE PATENTSCHRIFT

(11) **EP 1 791 825 B1**
(45) Veröffentlichungstag und Bekanntmachung des Hinweises auf die Patenterteilung: **17.09.2008**
(21) Anmeldenummer: 05787301.0
(22) Anmeldetag: 14.09.2005
(51) Int. Cl.: C07D 333/28, C07D 333/16, C07D 307/56, C07D 249/08, C07D 233/54, C07D 231/12, C07D 213/89, C07D 213/61, C07D 213/30, C07D 209/12, C07D 207/44, A01N 43/56, A01N 43/50, A01N 43/40, A01N 43/38

(54) **BENZOYLSUBSTITUIERTE SERIN-AMIDE**
BENZOYL-SUBSTITUTED SERINE AMIDES
SERINE-AMIDES SUBSTITUES PAR BENZOYLE

(30) Priorität: 16.09.2004 DE 102004045300
(43) Veröffentlichungstag der Anmeldung: 06.06.2007
(73) Patentinhaber: BASF SE, 67056 Ludwigshafen (DE)
(72) Erfinder: WITSCHEL, Matthias, 67098 Bad Dürkheim (DE); STELZER, Frank, 68309 Mannheim (DE); KÜHN, Toralf, 68199 Mannheim (DE); PARRA RAPADO, Liliana, 77654 Offenburg (DE); HUPE, Eike, 67067 Ludwigshafen (DE); ZAGAR, Cyrill, 68167 Mannheim (DE); REINHARD, Robert, 67065 Ludwigshafen (DE); SIEVERNICH, Bernd, 67454 Hassloch (DE); EHRHARDT, Thomas, 67346 Speyer (DE)
(86) Internationale Anmeldenummer: PCT/EP2005/009855
(87) Internationale Veröffentlichungsnummer: WO 2006/029828

(56) Entgegenhaltungen:
- WO-A-03/066576
- WO-A-20/05061443
- WO-A-20/05061464

## Beschreibung

Die vorliegende Erfindung betrifft benzoylsubstituierte Serin-Amide der Formel I 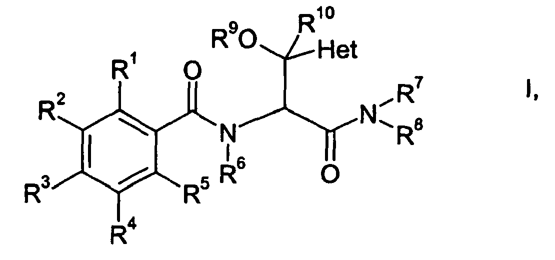 in der die Variablen die folgenden Bedeutungen haben:
- Het: mono- oder bicyclisches Heteroaryl mit 5 bis 10 Ringgliedern enthaltend 1 bis 4 Heteroatome aus der Gruppe Stickstoff, Sauerstoff und Schwefel, das partiell oder vollständig halogeniert sein kann und/oder 1 bis 3 Reste aus der Gruppe Cyano, Nitro, C₁-C₆-Alkyl, C₁-C₆-Halogenalkyl, Hydroxy, C₁-C₆-Alkoxy, C₁-C₆-Halogenalkoxy, Hydroxycarbonyl, C₁-C₆-Alkoxycarbonyl, Hydroxycarbonyl-C₁-C₆-alkoxy, C₁-C₆-Akoxycarbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkyl-sulfonylamino, Aminocarbonylamino, (C₁-C₆-Alkylamino)carbonylamino, Di-(C₁-C₆-alkyl)-aminocarbonylamino, Aryl und Aryl(C₁-C₆-alkyl) tragen kann;
- R¹: Halogen, Cyano, C₁-C₆-Alkyl, C₁-C₆-Halogenalkyl oder C₁-C₆-Halogenalkoxy;
- R², R³, R⁴, R⁵: Wasserstoff, Halogen, Cyano, C₁-C₆-Alkyl, C₁-C₆-Halogenalkyl, C₁-C₆-Alkoxy oder C₁-C₆-Halogenalkoxy;
- R⁶, R⁷: Wasserstoff, Hydroxy oder C₁-C₆-Alkoxy;
- R⁸: C₁-C₆-Alkyl, C₁-C₄-Cyanoalkyl oder C₁-C₆-Halogenalkyl;
- R⁹: Wasserstoff, C₁-C₆-Alkyl, C₃-C₆-Cycloalkyl, C₃-C₆-Alkenyl, C₃-C₆-Alkinyl, C₃-C₆-Halogenalkenyl, C₃-C₆-Halogenalkinyl, Formyl, C₁-C₆-Alkylcarbonyl, C₃-C₆-Cyclo-alkylcarbonyl, C₂-C₆-Alkenylcarbonyl, C₂-C₆-Alkinylcarbonyl, C₁-C₆-Alkoxycarbo-nyl, C₃-C₆-Alkenyloxycarbonyl, C₃-C₆-Alkinyloxycarbonyl, C₁-C₆-Alkylaminocar-bonyl, C₃-C₆-Alkenylaminocarbonyl, C₃-C₆-Alkinylaminocarbonyl, C₁-C₆-Alkyl-sulfonylaminocarbonyl, Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkylamino)-imino-C₁-C₆-alkyl oder Tri-C₁-C₄-alkylsilyl,
wobei die genannten Alkyl-, Cycloalkyl- und Alkoxyreste partiell oder voll-ständig halogeniert sein können und/oder eine bis drei der folgenden Grup-pen tragen können: Cyano, Hydroxy, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Di-(C₁-C₄-alkyl)-amino, C₁-C₄-Alkyl-C₁-C₆-alkoxy-carbonyl-amino, C₁-C₄-Alkylcarbonyl, Hydroxycarbonyl, C₁-C₄-Alkoxycarbonyl, Aminocarbonyl, C₁-C₄-Alkylamino-carbonyl, Di-(C₁-C₄-alkyl)-aminocarbonyl oder C₁-C₄-Alkylcarbonyloxy;
Phenyl, Phenyl-C₁-C₆-alkyl, Phenylcarbonyl, Phenylcarbonyl-C₁-C₆-alkyl, Pheno-xycarbonyl, Phenylaminocarbonyl, Phenylsulfonylaminocarbonyl, N-(C₁-C₆-Alkyl)-N-(phenyl)-aminocarbonyl, Phenyl-C₁-C₆-alkylcarbonyl, Heterocyclyl, Hete-rocyclyl-C₁-C₆-alkyl, Heterocyclylcarbonyl, Heterocyclylcarbonyl-C₁-C₆-alkyl, He-terocyclyloxycarbonyl, Heterocyclylaminocarbonyl, Heterocyclylsulfonyl-aminocarbonyl, N-(C₁-C₆-Alkyl)-N-(heterocyclyl)-aminocarbonyl, oder Heterocyc-lyl-C₁-C₆-alkylcarbonyl,
wobei der Phenyl- und der Heterocyclyl-Rest der 17 letztgenannten Substi-tuenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder
SO₂R¹¹;
- R¹⁰: Wasserstoff oder C₁-C₆-Alkyl;
- R¹¹: C₁-C₆-Alkyl, C₁-C₆-Halogenalkyl oder Phenyl, wobei der Phenylrest partiell oder vollständig halogeniert sein kann und/oder eine bis drei der folgenden Gruppen tragen kann: C₁-C₆-Alkyl, C₁-C₆-Halogen-alkyl oder C₁-C₆-Alkoxy;
sowie deren landwirtschaftlich brauchbaren Salze.

Außerdem betrifft die Erfindung Verfahren und Zwischenprodukte zur Herstellung von Verbindungen der Formel I, Mittel welche diese enthalten sowie die Verwendung dieser Derivate oder diese enthaltende Mittel zur Schadpflanzenbekämpfung.

Benzoylsubstituierte Serinamide mit pharmazeutischer Wirksamkeit, welche in β-Position einen Tetrazolylrest tragen, werden u.a. in JP 03/294253 beschrieben.

Weiterhin sind aus der Literatur, beispielsweise aus WO 03/066576, herbizid wirksame Phenylalaninderivate, welche in β-Position unsubstituiert sind oder ggf. durch Halogen substituierte Alkyl-, Alkenyl- oder Alkinylreste tragen, bekannt.

Die herbiziden Eigenschaften der bisher bekannten Verbindungen bzw. die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die benzoylsubstituierten Serin-Amide der Formel I sowie deren herbizide Wirkung gefunden.

Ferner wurden herbizide Mittel gefunden, welche die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I enthalten je nach Substitutionsmuster zwei oder mehr Chiralitätszentren und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di-(2-hydroxyeth-1-yl)-ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹¹ oder als Reste an Phenyl-, Aryl- Heteroaryl- oder Hetrocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Alkylsilyl-, Alkenyl-, Alkinyl-, Cyanoalkyl-, Halogenalkyl-, Halogenalkenyl-, Halogenalkinyl-, Alkoxy-, Halogenalkoxy-, Alkoxyalkyl-, Alkoxyalkoxyalkyl, Alkylcarbonyl-, Alkenylcarbonyl-, Alkinylcarbonyl-, Alkoxycarbonyl-, Alkenyloxycarbonyl-, Alkinyloxycarbonyl-, Alkylamino-, Alkylsulfonylamino-, Halogenalkylsulfonylamino-, Alkylalkoxycarbonylamino-, Alkylaminocarbonyl-, Alkenylaminocarbonyl-, Alkinylaminocarbonyl-, Alkylsulfonylaminocarbonyl, Dialkylaminocarbonyl-, N-Alkenyl-N-alkylaminocarbonyl-, N-Alkinyl-N-alkylamino-carbonyl-, N-Alkoxy-N-alkylamino-carbonyl-, N-Alkenyl-N-alkoxyaminocarbonyl-, N-Alkinyl-N-alkoxyaminocarbonyl-, Dialkylaminothiocarbonyl-, Alkylcarbonylalkyl-, Alkoximinoalkyl-, N-(Alkylamino)-iminoalkyl, N-(Dialkylamino)-iminoalkyl, Phenylalkyl-, Phenylcarbonylalkyl-, N-Alkyl-N-phenylaminocarbonyl-, Phenylalkylcarbonyl-, Arylalkyl-, Heterocyclylalkyl, Heterocyclylcarbonylalkyl-, N-Alkyl-N-heterocyclylaminocarbonyl-, Heterocyclylalkylcarbonyl-, Alkylthio-und Alkylcarbonyloxy-Teile können geradkettig oder verzweigt sein.

Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl sowie die Alkylteile von Tri-C₁-C₄-alkylsilyl, C₁-C₄-Alkylcarbonyloxy, C₁-C₄-alkyl-C₁-C₄-alkoxycarbonylamino, C₁-C₆-Alkyliminooxy-C₁-C₄-alkyl, C₁-C₄-alkoxy-C₁-C₄-alkoxy-C₁-C₄-alkyl und Aryl(C₁-C₄-alkyl): z.B. Methyl, Ethyl, n-Propyl, 1-Methylethyl, n-Butyl, 1-Methylpropyl, 2-Methylpropyl und 1,1-Dimethylethyl;
- C₁-C₆-Alkyl sowie die Alkylteile von C₁-C₆-Alkylsulfonylamino, C₁-C₆-Alkylsulfonylaminocarbonyl, N-(C₃-C₆-Alkenyl)-N-(C₁-C₆-alkyl)-aminocarbonyl, (C₃-C₆-Alkinyl)-N-(C₁-C₆-alkyl)-aminocarbonyl, N-(C₁-C₆-Alkoxy)-N-(C₁-C₆-alkyl)-aminocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkyl-amino)-imino-C₁-C₆-alkyl, Phenyl-C₁-C₆-alkyl, Phenylcarbonyl-C₁-C₆-alkyl, N-(C₁-C₆-Alkyl)-N-phenylaminocarbonyl, Heterocyclyl-C₁-C₆-alkyl, Hetrocyclyl-carbonyl-C₁-C₆-alkyl und N-(C₁-C₆-Alkyl)-N-heterocyclylaminocarbonyl: C₁-C₄-Alkyl, wie voranstehend genannt, sowie z.B. n-Pentyl, 1-Methyl-butyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethyl-butyl, 2-Ethylbutyl, 1,1,2-Trimethylpropyl, 1-Ethyl-1-methylpropyl und 1-Ethyl-3-methylpropyl;
- C₁-C₄-Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylcarbonyl sowie die Alkylcarbonylreste von C₁-C₆-Alkylcarbonyl-C₁-C₆-alkyl, Phenyl-C₁-C₆-alkylcarbonyl und Heterocyclyl-C₁-C₆-alkylcarbonyl: C₁-C₄-Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl;
- C₃-C₆-Cycloalkyl sowie die Cycloalkylteile von C₃-C₆-Cycloalkylcarbonyl: monocyclischer, gesättigter Kohlenwasserstoff mit 3 bis 6 Ringgliedern, wie Cyclopropyl, Cyclobutyl, Cyclopentyl und Cyclohexyl;
- C₃-C₆-Alkenyl sowie die Alkenylteile von C₃-C₆-Alkenyloxycarbonyl, C₃-C₆-Alkenylaminocarbonyl, N-(C₃-C₆-Alkenyl)-N-(C₁-C₆-alkyl)aminocarbonyl und N-(C₃-C₆-Alkenyl)-N-(C₁-C₆-alkoxy)aminocarbonyl: z.B. 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
- C₂C₆-Alkenyl sowie die Alkenylteile von C₂-C₆-Alkenylcarbonyl: C₃-C₆-Alkenyl wie voranstehend genannt sowie Ethenyl;
- C₃-C₆-Alkinyl sowie die Alkinylteile von C₃-C₆-Alkinyloxycarbonyl, C₃-C₆-Alkinylaminocarbonyl, N-(C₃-C₆-Alkinyl)-N-(C₁-C₆-alkyl)-aminocarbonyl, N-(C₃-C₆-Alkinyl)-N-(C₁-C₆-alkoxyaminocarbonyl: z.B. 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
- C₂-C₆-Alkinyl sowie die Alkinylteile von C₂-C₆-Alkinylcarbonyl: C₃-C₆-Alkinyl wie voranstehend genannt sowie Ethinyl;
- C₁-C₄-Cyanoalkyl: z.B. Cyanomethyl, 1-Cyanoeth-1-yl, 2-Cyanoeth-1-yl, 1-Cyano-prop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyano-but-2-yl, 2-Cyanobut-2-yl, 1-Cyanobut-3-yl, 2-Cyanobut-3-yl, 1-Cyano-2-methyl-prop-3-yl, 2-Cyano-2-methyl-prop-3-yl, 3-Cyano-2-methyl-prop-3-yl und 2-Cyanomethyl-prop-2-yl;
- C₁-C₄-Halogenalkyl: ein C₁-C₄-Alkylrest wie vorstehend genannt, der partiell oder vollständig durch Fluor, Chlor, Brom und/oder lod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, Brommethyl, lodmethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl sowie die Halogenalkylteile von C₁-C₆-Halogenalkylsulfonyl-amino: C₁-C₄-Halogenalkyl wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₃-C₆-Halogenalkenyl: ein C₃-C₆-Alkenylrest, wie voranstehend genannt, der partiell oder vollständig durch Fluor, Chlor, Brom und/oder Iod substituiert ist, z.B. 2-Chlor-prop-2-en-1-yl, 3-Chlorprop-2-en-1-yl, 2,3-Dichlorprop-2-en-1-yl, 3,3-Dichlorprop-2-en-1-yl, 2,3,3-Trichlor-2-en-1-yl, 2,3-Dichlorbut-2-en-1-yl, 2-Bromprop-2-en-1-yl, 3-Bromprop-2-en-1-yl, 2,3-Dibromprop-2-en-1-yl, 3,3-Dibromprop-2-en-1-yl, 2,3,3-Tribrom-2-en-1-yl oder 2,3-Dibrombut-2-en-1-yl;
- C₃-C₆-Halogenalkinyl: ein C₃-C₆-Alkinylrest, wie voranstehend genannt, der partiell oder vollständig durch Fluor, Chlor, Brom und/oder Iod substituiert ist, z.B. 1,1-Difluor-prop-2-in-1-yl, 3-Iod-prop-2-in-1-yl, 4-Fluorbut-2-in-1-yl, 4-Chlorbut-2in-1-yl, 1,1-Difluorbut-2-in-1-yl, 4-Iodbut-3-in-1-yl, 5-Fluorpent-3-in-1-yl, 5-Iodpent-4-in-1-yl, 6-Fluorhex-4-in-1-yl oder 6-Iodhex-5-in-1-yl;
   C₁-C₄-Alkoxy sowie die Alkoxyteile von Hydroxycarbonyl-C₁-C₄-alkoxy, C₁-C₄-Alkoxycarbonyl-C₁-C₄-alkoxy, C₁-C₄-Alkoxy-C₁-C₄-alkoxy-C₁-C₄-alkyl und C₁-C₄-alkyl-C₁-C₄-alkoxycarbonylamino: z.B. Methoxy, Ethoxy, Propoxy, 1-Methyl-ethoxy, Butoxy, 1-Methylpropoxy, 2-Methylpropoxy und 1,1-Dimethylethoxy;
- C₁-C₆-Alkoxy sowie die Alkoxyteile von Hydroxycarbonyl-C₁-C₆-alkoxy, C₁-C₆-Alkoxycarbonyl-C₁-C₆-alkoxy, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl und C₁-C₆-Alkoxyimino-C₁-C₆-alkyl: C₁-C₄-Alkoxy wie voranstehend genannt, sowie z.B. Pentoxy, 1-Methyl-butoxy, 2-Methylbutoxy, 3-Methoxylbutoxy, 1,1-Dimethyl-propoxy, 1,2-Dimethyl-propoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Di-methylbutoxy,1,2-Dimethyl-butoxy, 1,3-Dimethylbutoxy, 2,2-Dimethylbutoxy, 2,3-Dimethylbutoxy, 3,3-Dimethyl-butoxy, 1-Ethylbutoxy, 2-Ethylbutoxy, 1,1,2-Tri-methylpropoxy, 1,2,2-Trimethyl-propoxy, 1-Ethyl-1-methylpropoxy und 1-Ethyl-2-methylpropoxy;
- C₁-C₄-Halogenalkoxy: ein C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy: C₁-C₄-Halogenalkoxy wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy und Dodecafluorhexoxy;
- C₁-C₆-Alkoxy-C₁-C₄-alkyl: durch C₁-C₆-Alkoxy wie vorstehend genannt substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methyl-propoxy)methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)-ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)-propyl, 3-(Butoxy)propyl, 3-(1-Methylpropoxy)propyl, 3-(2-Methylpropoxy)propyl, 3-(1,1-Dimethylethoxy)propyl, 2-(Methoxy)-butyl, 2-(Ethoxy)butyl, 2-(Propoxy)-butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)-butyl, 3-(Propoxy)butyl, 3-(1-Methylethoxy)butyl, 3-(Butoxy)-butyl, 3-(1-Methyl-propoxy)-butyl, 3-(2-Methylpropoxy)butyl, 3-(1,1-Dimethylethoxy)butyl, 4-(Methoxy)-butyl, 4-(Ethoxy)butyl, 4-(Propoxy)butyl, 4-(1-Methylethoxy)butyl, 4-(Butoxy)butyl, 4-(1-Methylpropoxy)butyl, 4-(2-Methylpropoxy)butyl und 4-(1,1-Dimethylethoxy)-butyl;
- C₁-C₄-Alkoxycarbonyl sowie die Alkoxycarbonylteile von C₁-C₄-Alkoxycarbonyl-C₁-C₄-alkoxy, C₁-C₄-Alkoxy-C₁-C₄-alkoxycarbonyl und Di-(C₁-C₄-alkyl)-amino-C₁-C₄-alkoxycarbonyl: z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- C₁-C₆-Alkoxycarbonyl sowie die Alkoxycarbonylteile von C₁-C₆-Alkoxycarbonyl-C₁-C₆-alkoxy: C₁-C₄-Alkoxycarbonyl, wie voranstehend genannt, sowie z.B. Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methylpropoxycarbonyl oder 1-Ethyl-2-methyl-propoxycarbonyl;
- C₁-C₄-Alkylthio: z.B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylamino sowie die Alkylaminoreste von N(C₁-C₆-Alkylamino)imino-C₁-C₆-alkyl: z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
- Di(C₁-C₄-alkyl)amino: z.B. N,N-Dimethylamino, N,N-Diethylamino, N,N-Dipropylamino, N,N-Di-(1-methylethyl)-amino, N,N-Dibutylamino, N,N-Di-(1-methylpropyl)amino, N,N-Di-(2-methylpropyl)-amino, N,N-Di-(1,1-dimethylethyl)-amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethyl-ethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)-amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl-)amino, N-(1-Methylethyl)-N-(1-methylpropyl)amino, N-(1-Methylethyl)-N-(2-methyl-propyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethyl-ethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
- Di(C₁-C₆-alkyl)amino sowie die Dialkylaminoreste von N-(Di-C₁-C₆-alkylamino)-imino-C₁-C₆-alkyl: Di(C₁-C₄-alkyl)amino wie voranstehend genannt sowie: z.B. N,N-Dipentylamino, N,N-Dihexylamino, N-Methyl-N-pentylamino, N-Ethyl-N-pentylamino, N-Methyl-N-hexylamino und N-Ethyl-N-hexylamino;(C₁-C₄-Alkylamino)carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- (C₁-C₄-Alkylamino)carbonyl sowie die (C₁-C₄-Alkylamino)carbonyl-Teile von (C₁-C₄-Alkylamino)carbonylamino: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- Di(C₁-C₄-alkyl)aminocarbonyl sowie die Di(C₁-C₄-alkyl)aminocarbonyl-Teile von Di(C₁-C₄-alkyl)aminocarbonylamino: z.B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)-aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)-aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl oder N-(1,1-Dimethylethyl)-N-(2-methylpropyl)aminocarbonyl;
- (C₁-C₆-Alkylamino)carbonyl sowie die (C₁-C₆-Alkylamino)carbonyl-Teile von (C₁-C₆-Alkylamino)carbonylamino: (C₁-C₄-Alkylamino)carbonyl, wie voranstehend genannt, sowie z.B. Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl;
- Di(C₁-C₆-alkyl)aminocarbonyl sowie die Di(C₁-C₆-alkyl)aminocarbonyl-Teile von Di(C₁-C₆-alkyl)aminocarbonylamino: Di(C₁-C₄-alkyl)aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-aminocarbonyl, N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-(2-methylpentyl)-aminocarbonyl, N-Methyl-N-(3-methylpentyl)-aminocarbonyl, N-Methyl-N-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)- amino-carbonyl, N-Methyl-N-(3,3-dimethylbutyl)-aminocarbonyl, N-Methyl-N-(1-ethyl-butyl)-aminocarbonyl, N-Methyl-N-(2-ethylbutyl)-aminocarbonyl, N-Methyl-N-(1,1,2-trimethylpropyl)-aminocarbonyl, N-Methyl-N-(1,2,2-trimethylpropyl)-amino-carbonyl, N-Methyl-N-(1-ethyl-1-methylpropyl)-aminocarbonyl, N-Methyl-N-(1-ethyl-2-methylpropyl)-aminocarbonyl, N-Ethyl-N-pentylaminocarbonyl, N-Ethyl-N-(1-methylbutyl)-aminocarbonyl, N-Ethyl-N-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl-N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl)-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl)-aminocarbonyl, N-Ethyl-N-(1,3-dimethylbutyl)-aminocarbonyl, N-Ethyl-N-(2,2-dimethylbutyl)- aminocarbonyl, N-Ethyl-N-(2,3-dimethylbutyl)-aminocarbonyl, N-Ethyl-N-(3,3-dimethylbutyl)-aminocarbonyl, N-Ethyl-N-(1-ethylbutyl)-amino-carbonyl, N-Ethyl-N-(2-ethylbutyl)-aminocarbonyl, N-Ethyl-N-(1,1,2-trimethyl-propyl)-aminocarbonyl, N-Ethyl-N-(1,2,2-trimethylpropyl)-aminocarbonyl, N-Ethyl-N- (1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexyl-aminocarbonyl, N-Butyl-N-hexylaminocarbonyl, N-Pentyl-N-hexylaminocarbonyl oder N,N-Dihexylamino-carbonyl;
- Di(C₁-C₆-alkyl)aminothiocarbonyl: z.B. N,N-Dimethylaminothiocarbonyl, N,N-Diethylaminothiocarbonyl, N,N-Di-(1-methylethyl)aminothiocarbonyl, N,N-Dipropyl-aminothiocarbonyl, N,N-Dibutylaminothiocarbonyl, N,N-Di-(1-methylpropyl)-aminothiocarbonyl, N,N-Di-(2-methylpropyl)-aminothiocarbonyl, N,N-Di-(1,1-dimethylethyl)-aminothiocarbonyl, N-Ethyl-N-methylaminothiocarbonyl, N-Methyl-N-propylaminothiocarbonyl, N-Methyl-N-(1-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)aminothiocarbonyl, N-(1,1-Dimethylethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2-methylpropyl)-aminothiocarbonyl, N-Ethyl-N-(1,1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylamino-thiocarbonyl, N-(1,1-Dimethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-aminothiocarbonyl, N-(1,1-Dimethylethyl)-N-(1-methylpropyl)-aminothiocarbonyl, N-(1,1-Dimethylethyl)-N-(2-methylpropyl)-aminothiocarbonyl, N-Methyl-N-pentylaminothiocarbonyl, N-Methyl-N-(1-methylbutyl)-aminothio-carbonyl, N-Methyl-N-(2-methylbutyl)-aminothiocarbonyl, N-Methyl-N-(3-methylbutyl)-aminothiocarbonyl, N-Methyl-N-(2,2-dimethylpropyl)-aminothio-carbonyl, N-Methyl-N-(1-ethylpropyl)-aminothiocarbonyl, N-Methyl-N-hexyl-aminothiocarbonyl, N-Methyl-N- (1,1-dimethylpropyl)-aminothiocarbonyl, N-Methyl-N-(1,2-dimethylpropyl)-aminothiocarbonyl, N-Methyl-N-(1-methylpentyl)-aminothiocarbonyl, N-Methyl-N-(2-methylpentyl)-aminothiocarbonyl, N-Methyl-N-(3-methylpentyl)-aminothiocarbonyl, N-Methyl-N-(4-methylpentyl)-aminothio-carbonyl, N-Methyl-N- (1,1-dimethylbutyl)-aminothiocarbonyl, N-Methyl-N-(1,2-dimethylbutyl)-aminothiocarbonyl, N-Methyl-N-(1,3-dimethylbutyl)-aminothiocarbonyl, N-Methyl-N-(2,2-dimethylbutyl)- aminothiocarbonyl, N-Methyl-N-(2,3-dimethylbutyl)-aminothiocarbonyl, N-Methyl-N-(3,3-dimethylbutyl)-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1,2-trimethylpropyl)-aminothiocarbonyl, N-Methyl-N-(1,2,2-trimethylpropyl)-aminothiocarbonyl, N-Methyl-N-(1-ethyl-1-methylpropyl)-aminothiocarbonyl, N-Methyl-N-(1-ethyl-2-methylpropyl)-aminothiocarbonyl, N-Ethyl-N-pentyl-aminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)- aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethyl-propyl)-aminothiocarbonyl, N-Ethyl-N-(1,2-dimethylpropyl)- aminothiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-amino-thiocarbonyl, N-Ethyl-N-(1,2-dimethylbutyl)- aminothiocarbonyl, N-Ethyl-N-(1,3-dimethylbutyl)-aminothiocarbonyl, N-Ethyl-N-(2,2-dimethylbutyl)-aminothio-carbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl)-aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-N-(1,2,2-trimethylpropyl)-aminothiocarbonyl, N-Ethyl-N-(1-ethyl-1-methylpropyl)-aminothiocarbonyl, N-Ethyl-N-(1-ethyl-2-methylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexyl-aminothiocarbonyl, N-Butyl-N-hexylaminothiocarbonyl, N-Pentyl-N-hexyl-aminothiocarbonyl oder N,N-Dihexylaminothiocarbonyl;
- Heterocyclyl, sowie die Heterocyclylteile von Heterocyclyl-C₁-C₆-alkyl, Heterocyclylcarbonyl, Heterocyclylcarbonyl-C₁-C₆-alkyl, Heterocyclyloxycarbonyl, Heterocyclylaminocarbonyl, Heterocyclylsulfonylaminocarbonyl, N-(C₁-C₆-Alkyl)-N-(heterocyclyl)-aminocarbonyl und Heterocyclyl-C₁-C₆-alkylcarbonyl: ein gesättigter, partiell ungesättigter oder aromatischer 5- oder 6-gliedriger heterocyclischer Ring, der ein bis vier gleiche oder verschiedene Heteroatome, ausgewählt aus der Gruppe Sauerstoff, Schwefel oder Stickstoff, enthält, und über C oder N gebunden sein kann, z.B.
   C-gebundene, 5-gliedrige, gesättigte Ringe wie Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, Tetrahydropyrazol-3-yl, Tetrahydro-pyrazol-4-yl, Tetra-hydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxa-zol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 1,3,2-Dioxathiolan-4-yl;
   N-gebundene, 5-gliedrige, gesättigte Ringe wie: Tetrahydropyrrol-1-yl, Tetra-hydropyrazol-1-yl, Tetrahydroisoxazol-2-yl, Tetrahydroisothiazol-2-yl, Tetra-hydroimidazol-1-yl, Tetrahydrooxazol-3-yl, Tetrahydrothiazol-3-yl;
   C-gebundene, 5-gliedrige, partiell ungesättigte Ringe wie: 2,3-Dihydrofuran-2-yl, 2,3-Dihydrofuran-3-yl, 2,5-Dihydrofuran-2-yl, 2,5-Di-hydrofuran-3-yl, 4,5-Dihydrofuran-2-yl, 4,5-Dihydrofuran-3-yl, 2,3-Dihydro-thien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1 H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1 H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydro-oxazol-2-yl, 4,5-Dihydrooxazol-4-yl, 4,5-Dihydrooxazol-5-yl, 2,5-Dihydro-oxazol-2-yl, 2,5-Dihydrooxazol-4-yl, 2,5-Dihydrooxazol-5-yl, 2,3-Dihydro-oxazol-2-yl, 2,3-Dihydrooxazol-4-yl, 2,3-Dihydrooxazol-5-yl, 4,5-Dihydro-thiazol-2-yl, 4,5-Dihydrothiazol-4-yl, 4,5-Dihydrothiazol-5-yl, 2,5-Dihydro-thiazol-2-yl, 2,5-Dihydrothiazol-4-yl, 2,5-Dihydrothiazol-5-yl, 2,3-Dihydro-thiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxa-thiol-4-yl, 1,3-Oxathiol-5-yl, 1,2,3-Δ²-Oxadiazolin-4-yl, 1,2,3-Δ²-Oxadiazolin-5-yl, 1,2,4-Δ⁴-Oxadiazolin-3-yl, 1,2,4-Δ⁴-Oxadiazolin-5-yl, 1,2,4-Δ²-Oxadia-zolin-3-yl, 1,2,4-Δ²-Oxadiazolin-5-yl, 1,2,4-Δ³-Oxadiazolin-3-yl, 1,2,4-Δ³-Oxadiazolin-5-yl, 1,3,4-Δ²-Oxadiazolin-2-yl, 1,3,4-Δ²-Oxadiazolin-5-yl, 1,3,4-Δ³-Oxadiazolin-2-yl, 1,3,4-Oxadiazolin-2-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1,2,4-Δ²-Thiadiazolin-3-yl, 1,2,4-Δ²-Thiadiazolin-5-yl, 1,3,4-Δ²-Thiadiazolin-2-yl, 1,3,4-Δ²-Thiadiazolin-5-yl, 1,3,4-Δ³-Thiadiazolin-2-yl, 1,3,4-Thiadiazolin-2-yl, 1,2,3-Δ²-Triazolin-4-yl, 1,2,3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2,4-Δ³-Triazolin-5-yl, 1,2,4-Δ¹-Triazolin-2-yl, 1,2,4-Triazolin-3-yl, 3H-1,2,4-Dithiazol-5-yl, 2H-1,3,4-Dithiazol-5-yl, 2H-1,3,4-Oxathiazol-5-yl;
   N-gebundene, 5-gliedrige, partiell ungesättigte Ringe wie: 2,3-Dihydro-1H-pyrrol-1-yl, 2,5-Dihydro-1H-pyrrol-1-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, 1,2,4-Δ⁴-Oxadiazolin-2-yl, 1,2,4-Δ²-Oxadiazolin-4-yl, 1,2,4-Δ³-Oxadiazolin-2-yl, 1,3,4-Δ²-Oxadiazolin-4-yl, 1,2,4-Δ⁵-Thiadiazolin-2-yl, 1,2,4-Δ³-Thiadiazolin-2-y1, 1,2,4-Δ²-Thiadiazolin-4-yl, 1,3,4-Δ²-Thiadiazolin-4-yl, 1,2,3-Δ²-Triazolin-1-yl, 1,2,4-Δ²-Triazolin-1-yl, 1,2,4-Δ²-Triazolin-4-yl, 1,2,4-Δ³-Triazolin-1-yl, 1,2,4-Δ¹-Triazolin-4-yl;
   C-gebundene, 5-gliedrige, aromatische Ringe wie: 2-Furyl, 3-Furyl, 2-Thienyl, 3-Thienyl, Pyrrol-2-yl, Pyrrol-3-yl, Pyrazol-3-yl, Pyrazol-4-yl, Isoxazol-3-yl, Isoxa-zol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl, 1,2,4-Triazol-3-yl, Tetrazol-5-yl;
   N-gebundene, 5-gliedrige, aromatische Ringe wie: Pyrrol-1-yl, Pyrazol-1-yl, Imidazol-1-yl, 1,2,3-Triazol-1-yl, 1,2,4-Triazohl-yl, Tetrazol-1-yl;
   C-gebundene, 6-gliedrige, gesättigte Ringe wie: Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl;
   N-gebundene, 6-gliedrige, gesättigte Ringe wie: Piperidin-1-yl, Hexahydropyrimi-din-1-yl, Hexahydropyrazin-1-yl, Hexahydro-pyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl;
   C-gebundene, 6-gliedrige, partiell ungesättigte Ringe wie: 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydro-pyran-6-yl, 2H-3,4-Dihydrothiopyran-5-yl, 2H-3,4-Dihydrothiopyran-4-yl, 2H-3,4-Dihydropyran-3-yl, 2H-3,4-Dihydropyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetra-hydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydro-thiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydro-pyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl-, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydro-pyridin-3-yl, 1,2-Dihydropyridin-4-yl, 1,2-Dihydropyridin-5-yl, 1,2-Dihydro-pyridin-6-yl, 3,4-Dihydropyridin-2-yl, 3,4-Dihydropyridin-3-yl, 3,4-Dihydro-pyridin-4-yl, 3,4-Dihydropyridin-5-yl, 3,4-Dihydropyridin-6-yl, 2,5-Dihydro-pyridin-2-yl, 2,5-Dihydropyridin-3-yl, 2,5-Dihydropyridin-4-yl, 2,5-Dihydro-pyridin-5-yl, 2,5-Dihydropyridin-6-yl, 2,3-Dihydropyridin-2-yl, 2,3-Dihydro-pyridin-3-yl, 2,3-Dihydropyridin-4-yl, 2,3-Dihydropyridin-5-yl, 2,3-Dihydro-pyridin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Di-hydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetra-hydropyridazin-5-yl, 1,2,5,6-Tetrahydropyridazin-6-yl, 1,2,3,6-Tetrahydro-pyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydro-pyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-bihydropyrimidin-2-yl, 3,4-Dihydropyrimidin-4-yl, 3,4-Dihydropyrimidin-5-yl oder 3,4-Dihydropyrimidin-6-yl;
   N-gebundene, 6-gliedrige, partiell ungesättigte Ringe wie: 1,2,3,4-Tetrahydro-pyridin-1-yl, 1,2,5,6-Tetrahydropyridin-1-yl, 1,4-Dihydro-pyridin-1-yl, 1,2-Dihydropyridin-1-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-oxazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihdro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl;
   C-gebundene, 6-gliedrige, aromatische Ringe wie: Pyridin-2-yl, Pyridin-3-yl, Pyridin-4-yl, Pyridazin-3-y1, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1,2,4-Triazin-5-yl, 1,2,4-Triazin-6-yl, 1,2,4,5-Tetrazin-3-yl;
   wobei mit einem ankondensierten Phenylring oder mit einem C₃-C₆-Carboxyclus oder mit einem weiteren 5- bis 6-gliedrigen Heterocyclus ein bicyclisches Ringsystem ausgebildet werden kann.
- Aryl sowie der Arylteil von Aryl(C₁-C₄-alkyl): ein- bis dreikerniger aromatischer Carbocyclus mit 6 bis 14 Ringgliedern, wie z.B. Phenyl, Naphthyl und Anthracenyl;
- mono- oder bicyclisches Heteroaryl mit 5 bis 10 Ringgliedern enthaltend 1 bis 4 Heteroatome aus der Gruppe Stickstoff, Sauerstoff und Schwefel:
   mono- oder bicyclisches aromatisches Heterocyclyl mit 5 bis 10 Ringgliedern, welches neben Kohlenstoffatomen 1 bis 4 Stickstoffatome, oder 1 bis 3 Stickstoffatome und ein Sauerstoff- oder ein Schwefelatom, oder ein Sauerstoff- oder ein Schwefelatom enthält, z.B.
   Monocyclen wie Furyl (z.B. 2-Furyl, 3-Furyl), Thienyl (z.B. 2-Thienyl, 3-Thienyl), Pyrrolyl (z.B. Pyrrol-2-yl, Pyrrol-3-yl), Pyrazolyl (z.B. Pyrazol-3-yl, Pyrazol-4-yl), Isoxazolyl (z.B. Isoxazol-3-yl, Isoxazol-4-yl, Isoxazol-5-yl), Isothiazolyl (z.B. Isothiazol-3-yl, Isothiazol-4-yl, Isothiazol-5-yl), Imidazolyl (z.B. Imidazol-2-yl, Imidazol-4-yl), Oxazolyl (z.B. Oxazol-2-yl, Oxazol-4-yl, Oxazol-5-yl), Thiazolyl (z.B. Thiazol-2-yl, Thiazol-4-yl, Thiazol-5-yl), Oxadiazolyl (z.B. 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl), Thiadiazolyl (z.B. 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl), Triazolyl (z.B. 1,2,3-Triazol-4-yl, 1,2,4-Triazol-3-yl), Tetrazol-5-yl, Pyridyl (z.B. Pyridin-2-yl, Pyridin-3-yl, Pyridin-4-yl), Pyrazinyl (z.B. Pyridazin-3-yl, Pyridazin-4-yl), Pyrimidinyl (z.B. Pyrimidin-2-yl, Pyrimidin-4-yl, Pyrimidin-5-yl), Pyrazin-2-yl, Triazinyl (z.B. 1,3,5-Triazin-2-yl, 1,2,4-Triazin-3-yl, 1,2,4-Triazin-5-yl, 1,2,4-Triazin-6-yl), Tetrazinyl (z.B. 1,2,4,5-Tetrazin-3-yl); sowie
   Bicyclen wie die benzanellierten Derivate der vorgenannten Monocyclen, z.B. Chinolinyl, Isochinolinyl, Indolyl, Benzthienyl, Benzofuranyl, Benzoxazolyl, Benzthiazolyl, Benzisothiazolyl, Benzimidazolyl, Benzopyrazolyl, Benzthiadiazolyl, Benzotriazolyl.

Alle Phenyl- und Arylringe bzw. Heterocyclyl- und Heteroarylreste sowie alle Phenylkomponenten in Phenyl-C₁-C₆-alkyl, Phenylcarbonyl, Phenylcarbonyl-C₁-C₆-alkyl, Phenoxycarbonyl, Phenylaminocarbonyl, Phenylsulfonylaminocarbonyl, N-(C₁-C₆-Alkyl)-N-phenylaminocarbonyl und Phenyl-C₁-C₆-alkylcarbonyl, alle Arylkomponenten in Aryl(C₁-C₄-alkyl), alle Heteroarylkomponenten in mono- oder bicyclischem Heteroaryl und alle Heterocyclylkomponenten in Heterocyclyl-C₁-C₆-alkyl, Heterocyclylcarbonyl, Heterocyclylcarbonyl-C₁-C₆-alkyl, Heterocyclyloxycarbonyl, Heterocyclylaminocarbonyl, Heterocyclylsulfonylaminocarbonyl, N-(C₁-C₆-Alkyl)-N-heterocyclylaminocarbonyl und Heterocyclyl-C₁-C₆-alkylcarbonyl sind, soweit nicht anders angegeben, vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

In einer besonderen Ausführungsform haben die Variablen der benzoylsubstituierten Serin-Amide der Formel I folgende Bedeutungen, wobei diese für sich allein betrachtet als auch in Kombination miteinander besondere Ausgestaltungen der Verbindungen der Formel I darstellen:

Bevorzugt sind die benzoylsubstituierten Serin-Amide der Formel I, in der
- Het: mono- oder bicyclisches Heteroaryl mit 5 bis 10 Ringgliedern enthaltend 1 bis 4 Heteroatome aus der Gruppe Stickstoff, Sauerstoff und Schwefel, das partiell oder vollständig halogeniert sein kann und/oder 1 bis 3 Reste aus der Gruppe Nitro, C₁-C₄-Alkyl, C₁-C₄-Halogenalkyl, Hydroxy, C₁-C₄-Alkoxy, C₁-C₄-Halogenalkoxy, Hydroxycarbonyl, C₁-C₄-Alkoxycarbonyl, Hydroxycarbonyl-C₁-C₄-alkoxy, C₁-C₄-Alkoxycarbonyl-C₁-C₄-alkoxy, Amino, C₁-C₄-Alkylamino, Di(C₁-C₄-alkyl)amino, C₁-C₄-Alkylsulfonylamino, C₁-C₄-Halogenalkylsulfonylamino, Aminocarbonylamino, (C₁-C₄-Alkylamino)-carbonylamino und Di-(C₁-C₄-alkyl)-aminocarbonylamino tragen kann;
besonders bevorzugt mono- oder bicyclisches Heteroaryl ausgewählt aus der Gruppe Furyl, Thienyl, Pyrrolyl, Pyrazolyl, Isoxazolyl, Isothiazolyl, Imidazolyl, Oxazolyl, Thiazolyl, Oxadiazolyl, Thiadiazolyl, Triazolyl, Tetrazolyl, Pyridyl, Pyridazinyl, Pyrimidinyl, Pyrazinyl, Triazinyl, Tertrazinyl, Chinolinyl Isochinolinyl, Indolyl, Benzthienyl, Benzfuranyl, Benzoxazolyl, Benzthiazolyl, Benzisothiazolyl, Benzimidazolyl, Benzopyrazolyl, Benzthiadiazolyl und Benztriazolyl,
wobei die genannten Heteroaryle partiell oder vollständig halogeniert sein können und/oder 1 bis 3 Reste aus der Gruppe Nitro, C₁-C₄-Alkyl, C₁-C₄-Halogenalkyl, Hydroxy, C₁-C₄-Alkoxy, C₁-C₄-Halogenalkoxy, Hydroxycarbonyl, C₁-C₄-Alkoxycarbonyl, Hydroxycarbonyl-C₁-C₄-alkoxy, C₁-C₄-Alkoxycarbonyl-C₁-C₄-alkoxy, Amino, C₁-C₄-Alkylamino, Di(C₁-C₄-alkyl)-amino, C₁-C₄-Alkylsulfonylamino, C₁-C₄-Halogenalkylsulfonylamino, Aminocarbonylamino, (C₁-C₄-Alkylamino)carbonylamino und Di-(C₁-C₄-alkyl)-aminocarbonylamino tragen können;
insbesondere bevorzugt mono- oder bicyclisches Heteroaryl ausgewählt aus der Gruppe Furyl, Thienyl, Pyrazolyl, Imidazolyl, Thiazolyl, Triazolyl, Tetrazolyl, Pyridyl, Pyrimidinyl, Chinoliny und Indolyl,
wobei die genannten Heteroaryle partiell oder vollständig halogeniert sein können und/oder 1 bis 3 Reste aus der Gruppe Nitro, C₁-C₄-Alkyl, C₁-C₄-Halogenalkyl, Hydroxy, C₁-C₄-Alkoxy, C₁-C₄-Halogenalkoxy, Hydroxycarbonyl, C₁-C₄-Alkoxycarbonyl, Hydroxycarbonyl-C₁-C₄-alkoxy, C₁-C₄-Alkoxycarbonyl-C₁-C₄-alkoxy, Amino, C₁-C₄-Alkylamino, Di(C₁-C₄-alkyl)-amino, C₁-C₄-Alkylsulfonylamino, C₁-C₄-Halogenalkylsulfonylamino, Aminocarbonylamino, (C₁-C₄-Alkylamino)carbonylamino und Di-(C₁-C₄-alkyl)-aminocarbonylamino tragen können;
außerordentlich bevorzugt mono- oder bicyclisches Heteroaryl ausgewählt aus der Gruppe Thienyl, Thiazolyl, Tetrazolyl, Pyridyl und Indolyl,
wobei die genannten Heteroaryle partiell oder vollständig halogeniert sein können und/oder 1 bis 2 Reste aus der Gruppe Nitro, C₁-C₄-Alkyl, C₁-C₄-Halogenalkyl, Hydroxy, C₁-C₄-Alkoxy, C₁-C₄-Halogenalkoxy, Hydroxycarbonyl, C₁-C₄-Alkoxycarbonyl, Hydroxycarbonyl-C₁-C₄-alkoxy, C₁-C₄-Alkoxycarbonyl-C₁-C₄-alkoxy, Amino, C₁-C₄-Alkylamino, Di(C₁-C₄-alkyl)-amino, C₁-C₄-Alkylsulfonylamino, C₁-C₄-Halogenalkylsulfonylamino, Aminocarbonylamino, (C₁-C₄-Alkylamino)carbonylamino und Di-(C₁-C₄-alkyl)-aminocarbonylamino tragen können;
bedeutet.

Besonders bevorzugt sind die benzoylsubstituierten Serin-Amide der Formel I, in der Het für Het-1 bis Het-6 steht, 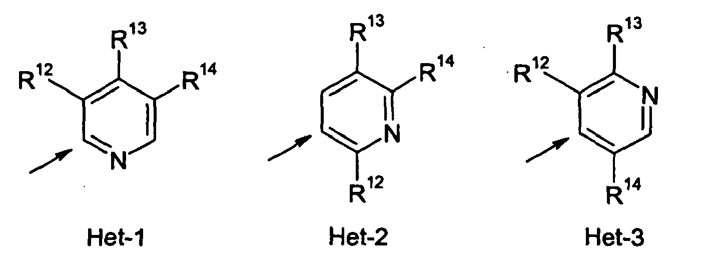 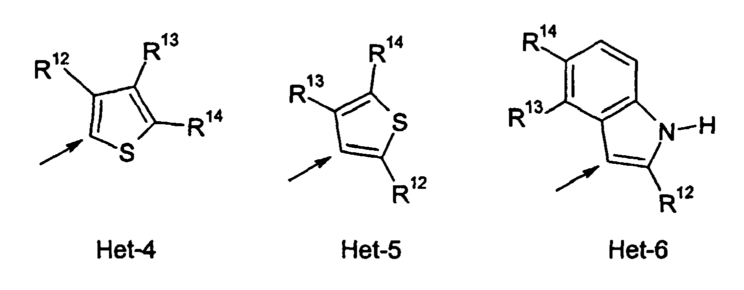 wobei der Pfeil die Verknüpfungsposition anzeigt und
- R¹²: Wasserstoff, Halogen, C₁-C₆-Alkyl oder C₁-C₆-Halogenalkyl;
bevorzugt Wasserstoff, Halogen, C₁-C₄-Alkyl oder C₁-C₄-Halogenalkyl;
insbesondere bevorzugt Wasserstoff, Halogen oder C₁-C₄-Alkyl;
besonders bevorzugt Wasserstoff, Fluor, Chlor oder Methyl;
- R¹³: Wasserstoff, Halogen, C₁-C₆-Alkyl oder C₁-C₆-Halogenalkyl;
bevorzugt Wasserstoff, Halogen, C₁-C₄-Alkyl oder C₁-C₄-Halogenalkyl;
insbesondere bevorzugt Wasserstoff, Halogen oder C₁-C₄-Alkyl;
besonders bevorzugt Wasserstoff, Fluor, Chlor oder Methyl;
- R¹⁴: Wasserstoff, Halogen oder C₁-C₄-Alkyl;
bevorzugt Wasserstoff, oder Halogen;
insbesondere bevorzugt Wasserstoff oder Fluor;
bedeuten.

Bevorzugt sind die benzoylsubstituierten Serin-Amide der Formel I, in der
- R¹: Halogen, C₁-C₄-Alkyl oder C₁-C₆-Halogenalkyl;
besonders bevorzugt Halogen oder C₁-C₆-Halogenalkyl;
insbesondere bevorzugt Halogen oder C₁-C₄-Halogenalkyl;
außerordentlich bevorzugt Fluor, Chlor oder CF₃;
bedeutet.

Ebenso bevorzugt sind die benzoylsubstituierten Serin-Amide der Formel I, in der
- R² und R³: unabhängig voneinander
Wasserstoff, Halogen, C₁-C₄-Alkyl oder C₁-C₆-Halogenalkyl;
sehr bevorzugt Wasserstoff, Halogen oder C₁-C₆-Halogenalkyl;
besonders bevorzugt Wasserstoff, Halogen oder C₁-C₄-Halogenalkyl;
insbesondere bevorzugt Wasserstoff, Fluor, Chlor oder CF₃;
außerordentlich bevorzugt Wasserstoff, Fluor oder Chlor; sehr außerordentlich bevorzugt Wasserstoff oder Fluor;
bedeuten.

Ebenso bevorzugt sind die benzoylsubstituierten Serin-Amide der Formel I, in der
- R⁴: Wasserstoff, Halogen, C₁-C₄-Alkyl oder C₁-C₄-Halogenalkyl;
besonders bevorzugt Wasserstoff, Halogen oder C₁-C₄-Alkyl;
insbesondere bevorzugt Wasserstoff oder Halogen; außerordentlich bevorzugt Wasserstoff;
bedeutet.

Ebenso bevorzugt sind die benzoylsubstituierten Serin-Amide der Formel I, in der
- R⁵: Wasserstoff, Halogen, C₁-C₄-Alkyl oder C₁-C₄-Halogenalkyl;
besonders bevorzugt Wasserstoff, Halogen oder C₁-C₄-Alkyl;
insbesondere bevorzugt Wasserstoff oder Halogen; außerordentlich bevorzugt Wasserstoff;
bedeutet.

Ebenso bevorzugt sind die benzoylsubstituierten Serin-Amide der Formel I, in der
- R⁶: Wasserstoff; und
- R⁷: Wasserstoff oder Hydroxy; besonders bevorzugt Wasserstoff;
bedeuten.

Ebenso bevorzugt sind die benzoylsubstituierten Serin-Amide der Formel I, in der
- R⁸: C₁-C₆-Alkyl oder C₁-C₆-Halogenalkyl;
besonders bevorzugt C₁-C₆-Alkyl;
insbesondere bevorzugt C₁-C₄-Alkyl; außerordentlich bevorzugt CH₃;
bedeutet.

Ebenso bevorzugt sind die benzoylsubstituierten Serin-Amide der Formel I, in der
- R⁹: Wasserstoff, C₁-C₆-Alkyl, C₃-C₆-Alkenyl, C₃-C₆-Alkinyl, C₁-C₆-Alkylcarbonyl, C₂-C₆-Alkenylcarbonyl, C₃-C₆-Cycloalkylcarbonyl, C₁-C₆-Alkoxycarbonyl, C₁-C₆-Alkylaminocarbonyl, C₁-C₆-Alkylsulfonylaminocarbonyl, Di-(C₁-C₆-alkyl)-amino-carbonyl, N-(C₁-C₆-Alkoxy)-N-(C₁-C₆-alkyl)-aminocarbonyl, Di-(C₁-C₆-alkyl)-aminothicarbonyl, C₁-C₆-Alkoxyimino-C₁-C₆-alkyl,
wobei die genannten Alkyl, Cycloalkyl- und Alkoxyreste partiell oder voll-ständig halogeniert sein können und/oder eine bis drei der folgenden Grup-pen tragen können: Cyano, Hydroxy, C₃-C₆-Cycloalkyl, C₁-C₄-Alkoxy, C₁-C₄-Alkylthio, Di-(C₁-C₄-alkyl)-amino, C₁-C₄-Alkylcarbonyl, Hydroxycarbonyl, C₁-C₄-Alkoxycarbonyl, Aminocarbonyl, C₁-C₄-Alkylaminocarbonyl, Di-(C₁-C₄-alkyl)-aminocarbonyl, oder C₁-C₄-Alkylcarbonyloxy;
Phenyl, Phenyl-C₁-C₆-alkyl, Phenylcarbonyl, Phenylcarbonyl-C₁-C₆-alkyl, Phenyl-sulfonylaminocarbonyl oder Phenyl-C₁-C₆-alkylcarbonyl,
wobei der Phenylrest der 6 letztgenannten Substituenten partiell oder voll-ständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder
SO₂R¹¹;
besonders bevorzugt Wasserstoff, C₁-C₆-Alkyl, C₃-C₆-Alkenyl, C₃-C₆-Alkinyl, C₁-C₆-Alkylcarbonyl, C₂-C₆-Alkenylcarbonyl, C₁-C₆-Alkoxycarbonyl, C₁-C₆-Alkyl-sulfonylaminocarbonyl, Di-(C₁-C₆-alkyl)-aminocarbonyl, N-(C₁-C₆-Alkoxy)-N-(C₁-C₆-alkyl)-aminocarbonyl oder Di-(C₁-C₆-alkyl)-aminothiocarbonyl,
wobei die genannten Alkyl- oder Alkoxyreste partiell oder vollständig halo-geniert sein können und/oder eine bis drei der folgenden Gruppen tragen können: Cyano, C₁-C₄-Alkoxy, C₁-C₄-Alkoxycarbonyl, C₁-C₄-Alkylaminocarbonyl, Di-(C₁-C₄-alkyl)-aminocarbonyl oder C₁-C₄-Alkylcarbonyloxy;
Phenyl-C₁-C₆-alkyl, Phenylcarbonyl, Phenylcarbonyl-C₁-C₆-alkyl, Phenylsulfonyl-aminocarbonyl oder Phenyl-C₁-C₆-alkylcarbonyl,
wobei der Phenylring der 5 letztgenannten Substituenten partiell oder voll-ständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder
SO₂R¹¹;
insbesondere bevorzugt Wasserstoff, C₁-C₆-Alkyl, C₃-C₆-Alkenyl, C₃-C₆-Alkinyl, C₁-C₆-Alkyl-carbonyl, C₂-C₆-Alkenylcarbonyl, C₁-C₆-Alkoxycarbonyl, Di-(C₁-C₆-alkyl)-amino-carbonyl, N-(C₁-C₆-Alkoxy)-N-(C₁-C₆-alkyl)-aminocarbonyl, Di-(C₁-C₆-alkyl)-aminothiocarbonyl, Phenyl-C₁-C₆-alkyl, Phenylcarbonyl, Phenylcarbo-nyl-C₁-C₆-alkyl oder Phenyl-C₁-C₆-alkylcarbonyl
wobei der Phenylring der 4 letztgenannten Substituenten partiell oder voll-ständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogen-alkoxy; oder
SO₂R¹¹;
bedeutet.

Ebenso bevorzugt sind die benzoylsubstituierten Serin-Amide der Formel I, in der
- R⁹: Wasserstoff, C₁-C₆-Alkyl, C₃-C₆-Alkenyl, C₃-C₆-Alkinyl, C₁-C₆-Alkylcarbonyl, C₂-C₆-Alkenylcarbonyl, C₃-C₆-Cycloalkylcarbonyl, C₁-C₆-Alkoxycarbonyl, C₁-C₆-Alkylaminocarbonyl, Di-(C₁-C₆-alkyl)-aminocarbonyl, N-(C₁-C₆-Alkoxy)-N-(C₁-C₆-alkyl)-aminocarbonyl, Di-(C₁-C₆-alkyl)-aminothiocarbonyl, C₁-C₆-Alkoxyimino-C₁-C₆-alkyl,
wobei die genannten Alkyl-, Cycloalkyl- oder Alkoxyreste partiell oder voll-ständig halogeniert sein können und/oder eine bis drei der folgenden Grup-pen tragen können: Cyano, Hydroxy, C₃-C₆-Cycloalkyl, C₁-C₄-Alkoxy, C₁-C₄-Alkylthio, Di-(C₁-C₄-alkyl)-amino, C₁-C₄-Alkylcarbonyl, Hydroxycarbonyl, C₁-C₄-Alkoxycarbonyl, Aminocarbonyl, C₁-C₄-Alkylaminocarbonyl, Di-(C₁-C₄-alkyl)-aminocarbonyl oder C₁-C₄-Alkylcarbonyloxy; oder
SO₂R¹¹;
bedeutet.

Ebenso bevorzugt sind die benzoylsubstituierten Serin-Amide der Formel I, in der
- R⁹: Wasserstoff, C₁-C₆-Alkyl, C₃-C₆-Alkenyl, C₃-C₆-Alkinyl, C₁-C₆-Alkylcarbonyl, C₁-C₆-Alkoxycarbonyl, C₁-C₆-Alkylaminocarbonyl, Di-(C₁-C₆-alkyl)-aminocarbonyl, N-(C₁-C₆-Alkoxy)-N-(C₁-C₆-alkyl)-amino-carbonyl,
wobei die genannten Alkyl-,und Alkoxyreste partiell oder vollständig halogeniert sein können und/oder eine bis drei der folgenden Gruppen tragen können: Cyano, C₁-C₄-Alkoxy, C₁-C₄-Alkylaminocarbonyl oder Di-(C₁-C₄-alkyl)-aminocarbonyl;
Phenyl-C₁-C₆-alkyl, Phenylcarbonyl, Phenylcarbonyl-C₁-C₆-alkyl, Phenylamino-carbonyl, N-(C₁-C₆-Alkyl)-N-(phenyl)-aminocarbonyl oder Heterocyclylcarbonyl,
wobei der Phenyl- und der Heterocyclylrest der 6 letztgenannten Substi-tuenten partiell oder vollständig halogeniert sein kann und/oder eine bis drei der folgenden Gruppen tragen kann: Cyano, C₁-C₄-Alkyl oder C₁-C₄-Halogenalkyl; oder
SO₂R¹¹;
besonders bevorzugt Wasserstoff, C₁-C₄-Alkyl, C₃-C₄-Alkenyl, C₃-C₄-Alkinyl, C₁-C₄-Alkylcarbonyl, C₁-C₄-Alkoxycarbonyl, C₁-C₄ Alkylaminocarbonyl, Di-(C₁-C₄-alkyl)-aminocarbonyl, N-(C₁-C₄-Alkoxy)-N-(C₁-C₄-alkyl)-aminocarbonyl, insbesondere bevorzugt Wasserstoff oder C₁-C₄-Alkyl;
wobei die genannten Alkyl-,und Alkoxyreste partiell oder vollständig halogeniert sein können und/oder eine bis drei der folgenden Gruppen tragen können: Cyano, C₁-C₄-Alkoxy, C₁-C₄-Alkylaminocarbonyl oder Di-(C₁-C₄-alkyl)-aminocarbonyl;
Phenyl-C₁-C₄-alkyl, Phenylcarbonyl, Phenylcarbonyl-C₁-C₄-alkyl, Phenylamino-carbonyl, N-(C₁-C₄-Alkyl)-N-(phenyl)-aminocarbonyl oder Heterocyclylcarbonyl,
wobei der Phenyl- und der Heterocyclylrest der 6 letztgenannten Substituenten partiell oder vollständig halogeniert sein kann und/oder eine bis drei der folgenden Gruppen tragen kann: Cyano, C₁-C₄-Alkyl oder C₁-C₄Halogenalkyl; oder
SO₂R¹¹;
außerordentlich bevorzugt Wasserstoff, C₁-C₄-Alkylcarbonyl, C₁-C₄-Alkylaminocarbonyl, Di-(C₁-C₄-alkyl)-aminocarbonyl, Phenylaminocarbonyl, N-(C₁-C₄-Alkyl)N-(phenyl)-aminocarbonyl, SO₂CH₃, SO₂CF₃ oder SO₂(C₆H₅);
bedeuten.

Ebenso bevorzugt sind die benzoylsubstituierten Serin-Amide der Formel I, in der
- R¹⁰: Wasserstoff oder C₁-C₄-Alkyl;
bevorzugt Wasserstoff oder CH₃; insbesondere bevorzugt Wasserstoff;
bedeutet.

Ebenso bevorzugt sind die benzoylsubstituierten Serin-Amide der Formel I, in der
- R¹¹: C₁-C₆-Alkyl, C₁-C₆-Halogenalkyl oder Phenyl,
wobei der Phenylrest partiell oder vollständig halogeniert sein kann und/oder durch C₁-C₄-Alkyl substituiert sein kann;
besonders bevorzugt C₁-C₄-Alkyl, C₁-C₄-Halogenalkyl oder Phenyl; insbesondere bevorzugt Methyl, Trifluormethyl oder Phenyl.
bedeutet.

Besonders bevorzugt sind die benzoylsubstituierten Serin-Amide der Formel I, in der
- Het: mono- oder bicyclisches Heteroaryl ausgewählt aus der Gruppe Thienyl, Thiazo-lyl, Tetrazolyl, Pyridyl und Indolyl,
wobei die genannten Heteroaryle partiell oder vollständig halogeniert sein können und/oder 1 bis 2 Reste aus der Gruppe Nitro, C₁-C₄-Alkyl, C₁-C₄-Halogenalkyl, Hydroxy, C₁-C₄-Alkoxy, C₁-C₄-Halogenalkoxy, Hydroxy-carbonyl, C₁-C₄-Alkoxycarbonyl, Hydroxycarbonyl-C₁-C₄-alkoxy, C₁-C₄-Alkoxycarbonyl-C₁-C₄-alkoxy, Amino, C₁-C₄-Alkylamino, Di(C₁-C₄-alkyl)-amino, C₁-C₄-Alkylsulfonylamino, C₁-C₄-Halogenalkylsulfonylamino, Ami-nocarbonylamino, (C₁-C₄-Alkylamino)carbonylamino, Di-(C₁-C₄-alkyl)-aminocarbonylamino tragen können;
- R¹: Fluor, Chlor oder CF₃;
- R² und R³: unabhängig voneinander Wasserstoff, Fluor oder Chlor;
- R⁴, R⁵, R⁶ und R⁷: Wasserstoff;
- R⁸: C₁-C₄-Alkyl,
besonders bevorzugt CH₃;
- R⁹: Wasserstoff, C₁-C₄-Alkylcarbonyl, C₁-C₄-Alkylaminocarbonyl, Di-(C₁-C₄-Alkyl)-aminocarbonyl, Phenylaminocarbonyl, N-(C1-C₄-alkyl)-N-(phenyl)-aminocarbonyl, SO₂CH₃ oder SO₂(C₆H₅); und
- R¹⁰: Wasserstoff
bedeuten.

Außerordentlich bevorzugt sind die Verbindungen der Formel I.a.1 (entspricht Formel I mit Het =Het-1, R¹= CF₃, R², R³, R⁴, R⁵, R⁶, R⁷ und R¹⁰ = H; R⁸ = CH₃), insbesondere die Verbindungen der Formel I.a.1.1 bis I.a.1.192 der Tabelle 1, wobei die Definitionen der Variablen R¹ bis R¹⁴ nicht nur in Kombination miteinander, sondern auch jeweils für sich allein betrachtet für die erfindungsgemäßen Verbindungen eine besondere Rolle spielen.

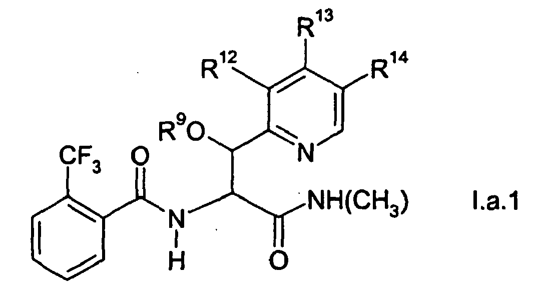

**Tabelle 1**

| Nr. | R⁹ | R¹² | R¹³ | R¹⁴ |
|---|---|---|---|---|
| I.a.1.1 | H | H | H | H |
| I.a.1.2 | H | H | H | F |
| I.a.1.3 | H | H | CH₃ | H |
| I.a.1.4 | H | H | CH₃ | F |
| I.a.1.5 | H | H | F | H |
| I.a.1.6 | H | H | F | F |
| I.a.1.7 | H | H | Cl | H |
| I.a.1.8 | H | H | Cl | F |
| I.a.1.9 | H | CH₃ | H | H |
| I.a.1.10 | H | CH₃ | H | F |
| I.a.1.11 | H | CH₃ | CH₃ | H |
| I.a.1.12 | H | CH₃ | CH₃ | F |
| I.a.1.13 | H | CH₃ | F | H |
| I.a.1.14 | H | CH₃ | F | F |
| I.a.1.15 | H | CH₃ | Cl | H |
| I.a.1.16 | H | CH₃ | Cl | F |
| I.a.1.17 | H | F | H | H |
| I.a.1.18 | H | F | H | F |
| I.a.1.19 | H | F | CH₃ | H |
| I.a.1.20 | H | F | CH₃ | F |
| I.a.1.21 | H | F | F | H |
| I.a.1.22 | H | F | F | F |
| I.a.1.23 | H | F | Cl | H |
| I.a.1.24 | H | F | Cl | F |
| I.a.1.25 | H | Cl | H | H |
| I.a.1.26 | H | Cl | H | F |
| I.a.1.27 | H | Cl | CH₃ | H |
| I.a.1.28 | H | Cl | CH₃ | F |
| I.a.1.29 | H | Cl | F | H |
| I.a.1.30 | H | Cl | F | F |
| I.a.1.31 | H | Cl | Cl | H |
| I.a.1.32 | H | Cl | Cl | F |
| I.a.1.33 | C(O)CH₃ | H | H | H |
| I.a.1.34 | C(O)CH₃ | H | H | F |
| I.a.1.35 | C(O)CH₃ | H | CH₃ | H |
| I.a.1.36 | C(O)CH₃ | H | CH₃ | F |
| I.a.1.37 | C(O)CH₃ | H | F | H |
| I.a.1.38 | C(O)CH₃ | H | F | F |
| I.a.1.39 | C(O)CH₃ | H | Cl | H |
| I.a.1.40 | C(O)CH₃ | H | Cl | F |
| I.a.1.41 | C(O)CH₃ | CH₃ | H | H |
| I.a.1.42 | C(O)CH₃ | CH₃ | H | F |
| I.a.1.43 | C(O)CH₃ | CH₃ | CH₃ | H |
| I.a.1.44 | C(O)CH₃ | CH₃ | CH₃ | F |
| I.a.1.45 | C(O)CH₃ | CH₃ | F | H |
| I.a.1.46 | C(O)CH₃ | CH₃ | F | F |
| I.a.1.47 | C(O)CH₃ | CH₃ | Cl | H |
| I.a.1.48 | C(O)CH₃ | CH₃ | Cl | F |
| I.a.1.49 | C(O)CH₃ | F | H | H |
| I.a.1.50 | C(O)CH₃ | F | H | F |
| I.a.1.51 | C(O)CH₃ | F | CH₃ | H |
| I.a.1.52 | C(O)CH₃ | F | CH₃ | F |
| I.a.1.53 | C(O)CH₃ | F | F | H |
| I.a.1.54 | C(O)CH₃ | F | F | F |
| I.a.1.55 | C(O)CH₃ | F | Cl | H |
| I.a.1.56 | C(O)CH₃ | F | Cl | F |
| I.a.1.57 | C(O)CH₃ | Cl | H | H |
| I.a.1.58 | C(O)CH₃ | Cl | H | F |
| I.a.1.59 | C(O)CH₃ | Cl | CH₃ | H |
| I.a.1.60 | C(O)CH₃ | Cl | CH₃ | F |
| I.a.1.61 | C(O)CH₃ | Cl | F | H |
| I.a.1.62 | C(O)CH₃ | Cl | F | F |
| I.a.1.63 | C(O)CH₃ | Cl | Cl | H |
| I.a.1.64 | C(O)CH₃ | Cl | Cl | F |
| I.a.1.65 | C(O)*tert*C₄H₉ | H | H | H |
| I.a.1.66 | C(O)*tert*C₄H₉ | H | H | F |
| I.a.1.67 | C(O)*tert*C₄H₉ | H | CH₃ | H |
| I.a.1.68 | C(O)*tert*C₄H₉ | H | CH₃ | F |
| I.a.1.69 | C(O)*tert*C₄H₉ | H | F | H |
| I.a.1.70 | C(O)*tert*C₄H₉ | H | F | F |
| I.a.1.71 | C(O)*tert*C₄H₉ | H | Cl | H |
| I.a.1.72 | C(O)*tert*C₄H₉ | H | Cl | F |
| I.a.1.73 | C(O)*tert*C₄H₉ | CH₃ | H | H |
| I.a.1.74 | C(O)*tert*C₄H₉ | CH₃ | H | F |
| I.a.1.75 | C(O)*tert*C₄H₉ | CH₃ | CH₃ | H |
| I.a.1.76 | C(O)*tert*C₄H₉ | CH₃ | CH₃ | F |
| I.a.1.77 | C(O)*tert*C₄H₉ | CH₃ | F | H |
| I.a.1.78 | C(O)*tert*C₄H₉ | CH₃ | F | F |
| I.a.1.79 | C(O)*tert*C₄H₉ | CH₃ | Cl | H |
| I.a.1.80 | C(O)*tert*C₄H₉ | CH₃ | Cl | F |
| I.a.1.81 | C(O)*tert*C₄H₉ | F | H | H |
| I.a.1.82 | C(O)*tert*C₄H₉ | F | H | F |
| I.a.1.83 | C(O)*tert*C₄H₉ | F | CH₃ | H |
| I.a.1.84 | C(O)*tert*C₄H₉ | F | CH₃ | F |
| I.a.1.85 | C(O)*tert*C₄H₉ | F | F | H |
| I.a.1.86 | C(O)*tert*C₄H₉ | F | F | F |
| I.a.1.87 | C(O)*tert*C₄H₉ | F | Cl | H |
| I.a.1.88 | C(O)*tert*C₄H₉ | F | Cl | F |
| I.a.1.89 | C(O)*tert*C₄H₉ | Cl | H | H |
| I.a.1.90 | C(O)*tert*C₄H₉ | Cl | H | F |
| I.a.1.91 | C(O)*tert*C₄H₉ | Cl | CH₃ | H |
| I.a.1.92 | C(O)*tert*C₄H₉ | Cl | CH₃ | F |
| I.a.1.93 | C(O)*tert*C₄H₉ | Cl | F | H |
| I.a.1.94 | C(O)*tert*C₄H₉ | Cl | F | F |
| I.a.1.95 | C(O)*tert*C₄H₉ | Cl | Cl | H |
| I.a.1.96 | C(O)*tert*C₄H₉ | Cl | Cl | F |
| I.a.1.97 | C(O)N(CH₃)₂ | H | H | H |
| I.a.1.98 | C(O)N(CH₃)₂ | H | H | F |
| I.a.1.99 | C(O)N(CH₃)₂ | H | CH₃ | H |
| I.a.1.100 | C(O)N(CH₃)₂ | H | CH₃ | F |
| I.a.1.101 | C(O)N(CH₃)₂ | H | F | H |
| I.a.1.102 | C(O)N(CH₃)₂ | H | F | F |
| I.a.1.103 | C(O)N(CH₃)₂ | H | Cl | H |
| I.a.1.104 | C(O)N(CH₃)₂ | H | Cl | F |
| I.a.1.105 | C(O)N(CH₃)₂ | CH₃ | H | H |
| I.a.1.106 | C(O)N(CH₃)₂ | CH₃ | H | F |
| I.a.1.107 | C(O)N(CH₃)₂ | CH₃ | CH₃ | H |
| I.a.1.108 | C(O)N(CH₃)₂ | CH₃ | CH₃ | F |
| I.a.1.109 | C(O)N(CH₃)₂ | CH₃ | F | H |
| I.a.1.110 | C(O)N(CH₃)₂ | CH₃ | F | F |
| I.a.1.111 | C(O)N(CH₃)₂ | CH₃ | Cl | H |
| I.a.1.112 | C(O)N(CH₃)₂ | CH₃ | Cl | F |
| I.a.1.113 | C(O)N(CH₃)₂ | F | H | H |
| I.a.1.114 | C(O)N(CH₃)₂ | F | H | F |
| I.a.1.115 | C(O)N(CH₃)₂ | F | CH₃ | H |
| I.a.1.116 | C(O)N(CH₃)₂ | F | CH₃ | F |
| I.a.1.117 | C(O)N(CH₃)₂ | F | F | H |
| I.a.1.118 | C(O)N(CH₃)₂ | F | F | F |
| I.a.1.119 | C(O)N(CH₃)₂ | F | Cl | H |
| I.a.1.120 | C(O)N(CH₃)₂ | F | Cl | F |
| I.a.1.121 | C(O)N(CH₃)₂ | Cl | H | H |
| I.a.1.122 | C(O)N(CH₃)₂ | Cl | H | F |
| I.a.1.123 | C(O)N(CH₃)₂ | Cl | CH₃ | H |
| I.a.1.124 | C(O)N(CH₃)₂ | Cl | CH₃ | F |
| I.a.1.125 | C(O)N(CH₃)₂ | Cl | F | H |
| I.a.1.126 | C(O)N(CH₃)₂ | Cl | F | F |
| I.a.1.127 | C(O)N(CH₃)₂ | Cl | Cl | H |
| I.a.1.128 | C(O)N(CH₃)₂ | Cl | Cl | F |
| I.a.1.129 | C(O)N(CH₃)(C₆H₅) | H | H | H |
| I.a.1.130 | C(O)N(CH₃)(C₆H₅) | H | H | F |
| I.a.1.131 | C(O)N(CH₃)(C₆H₅) | H | CH₃ | H |
| I.a.1.132 | C(O)N(CH₃)(C₆H₅) | H | CH₃ | F |
| I.a.1.133 | C(O)N(CH₃)(C₆H₅) | H | F | H |
| I.a.1.134 | C(O)N(CH₃)(C₆H₅) | H | F | F |
| I.a.1.135 | C(O)N(CH₃)(C₆H₅) | H | Cl | H |
| I.a.1.136 | C(O)N(CH₃)(C₆H₅) | H | Cl | F |
| I.a.1.137 | C(O)N(CH₃)(C₆H₅) | CH₃ | H | H |
| I.a.1.138 | C(O)N(CH₃)(C₆H₅) | CH₃ | H | F |
| I.a.1.139 | C(O)N(CH₃)(C₆H₅) | CH₃ | CH₃ | H |
| I.a.1.140 | C(O)N(CH₃)(C₆H₅) | CH₃ | CH₃ | F |
| I.a.1.141 | C(O)N(CH₃)(C₆H₅) | CH₃ | F | H |
| I.a.1.142 | C(O)N(CH₃)(C₆H₅) | CH₃ | F | F |
| I.a.1.143 | C(O)N(CH₃)(C₆H₅) | CH₃ | Cl | H |
| I.a.1.144 | C(O)N(CH₃)(C₆H₅) | CH₃ | Cl | F |
| I.a.1.145 | C(O)N(CH₃)(C₆H₅) | F | H | H |
| I.a.1.146 | C(O)N(CH₃)(C₆H₅) | F | H | F |
| I.a.1.147 | C(O)N(CH₃)(C₆H₅) | F | CH₃ | H |
| I.a.1.148 | C(O)N(CH₃)(C₆H₅) | F | CH₃ | F |
| I.a.1.149 | C(O)N(CH₃)(C₆H₅) | F | F | H |
| I.a.1.150 | C(O)N(CH₃)(C₆H₅) | F | F | F |
| I.a.1.151 | C(O)N(CH₃)(C₆H₅) | F | Cl | H |
| I.a.1.152 | C(O)N(CH₃)(C₆H₅) | F | Cl | F |
| I.a.1.153 | C(O)N(CH₃)(C₆H₅) | Cl | H | H |
| I.a.1.154 | C(O)N(CH₃)(C₆H₅) | Cl | H | F |
| I.a.1.155 | C(O)N(CH₃)(C₆H₅) | Cl | CH₃ | H |
| I.a.1.156 | C(O)N(CH₃)(C₆H₅) | Cl | CH₃ | F |
| I.a.1.157 | C(O)N(CH₃)(C₆H₅) | Cl | F | H |
| I.a.1.158 | C(O)N(CH₃)(C₆H₅) | Cl | F | F |
| I.a.1.159 | C(O)N(CH₃)(C₆H₅) | Cl | Cl | H |
| I.a.1.160 | C(O)N(CH₃)(C₆H₅) | Cl | Cl | F |
| I.a.1.161 | SO₂CH₃ | H | H | H |
| I.a.1.162 | SO₂CH₃ | H | H | F |
| I.a.1.163 | SO₂CH₃ | H | CH₃ | H |
| I.a.1.164 | SO₂CH₃ | H | CH₃ | F |
| I.a.1.165 | SO₂CH₃ | H | F | H |
| I.a.1.166 | SO₂CH₃ | H | F | F |
| I.a.1.167 | SO₂CH₃ | H | Cl | H |
| I.a.1.168 | SO₂CH₃ | H | Cl | F |
| I.a.1.169 | SO₂CH₃ | CH₃ | H | H |
| I.a.1.170 | SO₂CH₃ | CH₃ | H | F |
| I.a.1.171 | SO₂CH₃ | CH₃ | CH₃ | H |
| I.a.1.172 | SO₂CH₃ | CH₃ | CH₃ | F |
| I.a.1.173 | SO₂CH₃ | CH₃ | F | H |
| I.a.1.174 | SO₂CH₃ | CH₃ | F | F |
| I.a.1.175 | SO₂CH₃ | CH₃ | Cl | H |
| I.a.1.176 | SO₂CH₃ | CH₃ | Cl | F |
| I.a.1.177 | SO₂CH₃ | F | H | H |
| I.a.1.178 | SO₂CH₃ | F | H | F |
| I.a.1.179 | SO₂CH₃ | F | CH₃ | H |
| I.a.1.180 | SO₂CH₃ | F | CH₃ | F |
| I.a.1.181 | SO₂CH₃ | F | F | H |
| I.a.1.182 | SO₂CH₃ | F | F | F |
| I.a.1.183 | SO₂CH₃ | F | Cl | H |
| I.a.1.184 | SO₂CH₃ | F | Cl | F |
| I.a.1.185 | SO₂CH₃ | Cl | H | H |
| I.a.1.186 | SO₂CH₃ | Cl | H | F |
| I.a.1.187 | SO₂CH₃ | Cl | CH₃ | H |
| I.a.1.188 | SO₂CH₃ | Cl | CH₃ | F |
| I.a.1.189 | SO₂CH₃ | Cl | F | H |
| I.a.1.190 | SO₂CH₃ | Cl | F | F |
| I.a.1.191 | SO₂CH₃ | Cl | Cl | H |
| I.a.1.192 | SO₂CH₃ | Cl | Cl | F |

Ebenso außerordentlich bevorzugt sind die Verbindungen der Formel I.a.2, insbesondere die Verbindungen der Formel I.a.2.1 bis I.a.2.192, die sich von den entsprechenden Verbindungen der Formel I.a.1.1 bis I.a.1.192 dadurch unterscheiden, daß R² für Fluor steht.

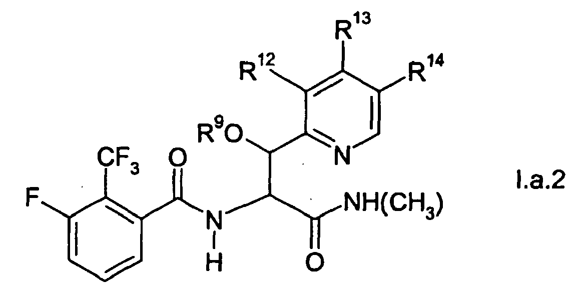

Ebenso außerordentlich bevorzugt sind die Verbindungen der Formel I.a.3, insbesondere die Verbindungen der Formel I.a.3.1 bis I.a.3.192, die sich von den entsprechenden Verbindungen der Formel I.a.1.1 bis I.a.1.192 dadurch unterscheiden, daß R³ für Fluor steht.

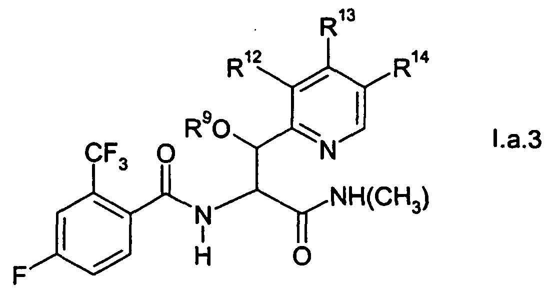

Ebenso außerordentlich bevorzugt sind die Verbindungen der Formel I.a.4, insbesondere die Verbindungen der Formel I.a.4.1 bis I.a.4.192, die sich von den entsprechenden Verbindungen der Formel I.a.1.1 bis I.a.1.192 dadurch unterscheiden, daß R⁴ für Fluor steht.

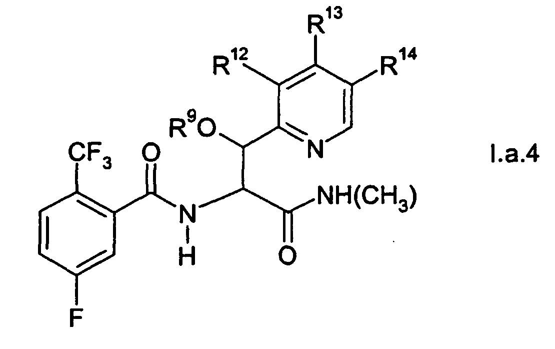

Ebenso außerordentlich bevorzugt sind die Verbindungen der Formel I.a.5, insbesondere die Verbindungen der Formel I.a.5.1 bis I.a.5.192, die sich von den entsprechenden Verbindungen der Formel I.a.1.1 bis I.a.1.192 dadurch unterscheiden, daß R² für Chlor steht.

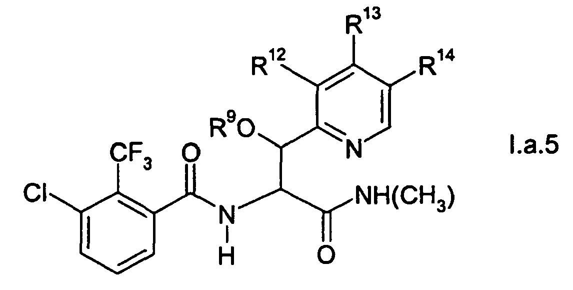

Ebenso außerordentlich bevorzugt sind die Verbindungen der Formel I.a.6, insbesondere die Verbindungen der Formel I.a.6.1 bis I.a.6.192, die sich von den entsprechenden Verbindungen der Formel I.a.1.1 bis I.a.1.192 dadurch unterscheiden, daß R³ für Chlor steht.

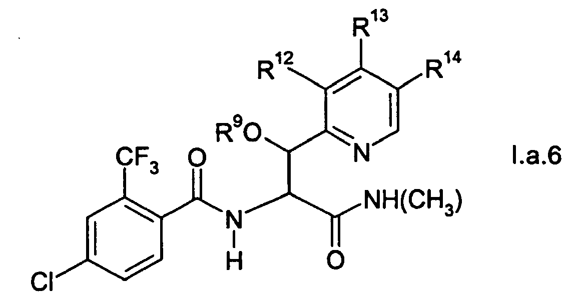

Ebenso außerordentlich bevorzugt sind die Verbindungen der Formel I.a.7, insbesondere die Verbindungen der Formel I.a.7.1 bis I.a.7.192, die sich von den entsprechenden Verbindungen der Formel I.a.1.1 bis I.a.1.192 dadurch unterscheiden, daß R³ und R⁴ für Fluor stehen.

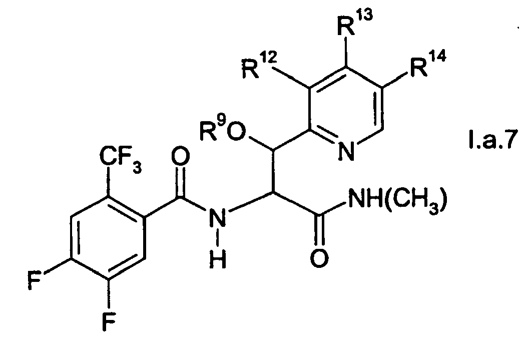

Ebenso außerordentlich bevorzugt sind die Verbindungen der Formel I.a.8, insbesondere die Verbindungen der Formel I.a.8.1 bis I.a.8.192, die sich von den entsprechenden Verbindungen der Formel I.a.1.1 bis I.a.1.192 dadurch unterscheiden, daß R¹ für Chlor und R² für CF₃ steht.

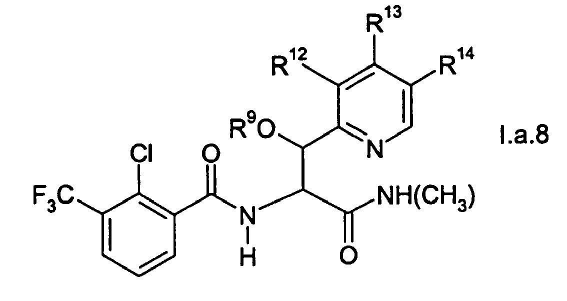

Ebenso außerordentlich bevorzugt sind die Verbindungen der Formel I.a.9, insbesondere die Verbindungen der Formel I.a.9.1 bis I.a.9.192, die sich von den entsprechenden Verbindungen der Formel I.a.1.1 bis I.a.1.192 dadurch unterscheiden, daß R¹ und R² für Chlor stehen.

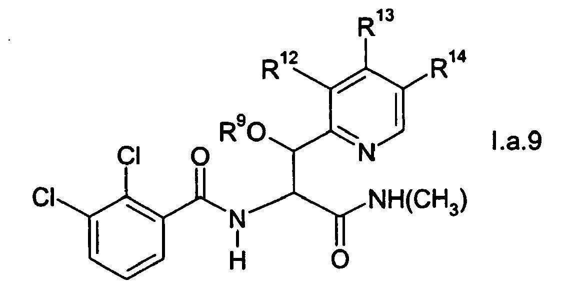

Ebenso außerordentlich bevorzugt sind die Verbindungen der Formel I.a.10, insbesondere die Verbindungen der Formel I.a.10. bis I.a.10.192, die sich von den entsprechenden Verbindungen der Formel I.a.1.1 bis I.a.1.192 dadurch unterscheiden, daß R¹ und R³ für Chlor stehen.

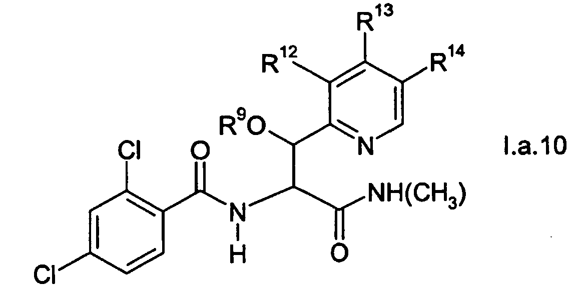

Ebenso außerordentlich bevorzugt sind die Verbindungen der Formel I.b.1, insbesondere die Verbindungen der Formel I.b.1.1 bis I.b.1.192, die sich von den entsprechenden Verbindungen der Formel I.a.1.1 bis I.a.1.192 dadurch unterscheiden, daß Het für Het-2 steht.

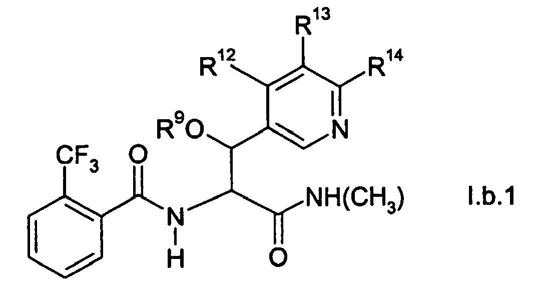

Ebenso außerordentlich bevorzugt sind die Verbindungen der Formel I.b.2, insbesondere die Verbindungen der Formel I.b.2.1 bis I.b.2.192, die sich von den entsprechenden Verbindungen der Formel I.a.1.1 bis I.a.1.192 dadurch unterscheiden, daß Het für Het-2 sowie R² für Fluor steht.

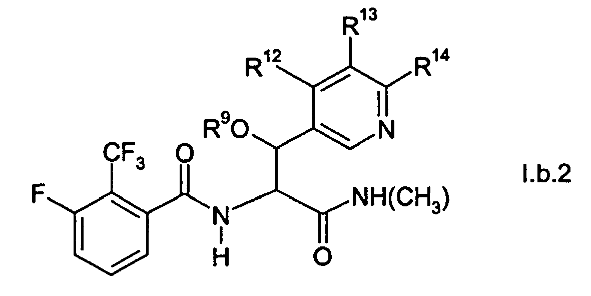

Ebenso außerordentlich bevorzugt sind die Verbindungen der Formel I.b.3, insbesondere die Verbindungen der Formel I.b.3.1 bis I.b.3.192, die sich von den entsprechenden Verbindungen der Formel I.a.1.1 bis I.a.1.192 dadurch unterscheiden, daß Het für Het-2 sowie R³ für Fluor steht.

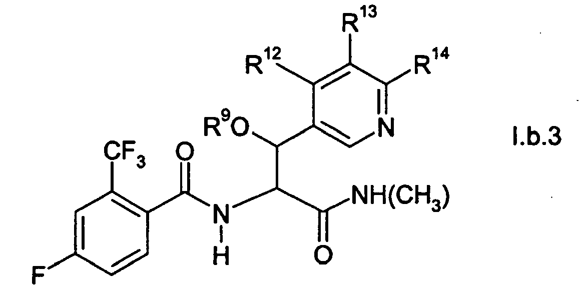

Ebenso außerordentlich bevorzugt sind die Verbindungen der Formel I.b.4, insbesondere die Verbindungen der Formel I.b.4.1 bis I.b.4.192, die sich von den entsprechenden Verbindungen der Formel I.a.1.1 bis I.a.1.192 dadurch unterscheiden, daß Het für Het-2 sowie R⁴ für Fluor steht.

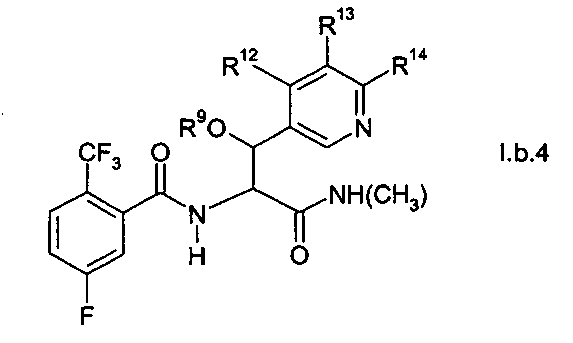

Ebenso außerordentlich bevorzugt sind die Verbindungen der Formel I.b.5, insbesondere die Verbindungen der Formel I.b.5.1 bis I.b.5.192, die sich von den entsprechenden Verbindungen der Formel I.a.1.1 bis I.a.1.192 dadurch unterscheiden, daß Het für Het-2 sowie R² für Chlor steht.

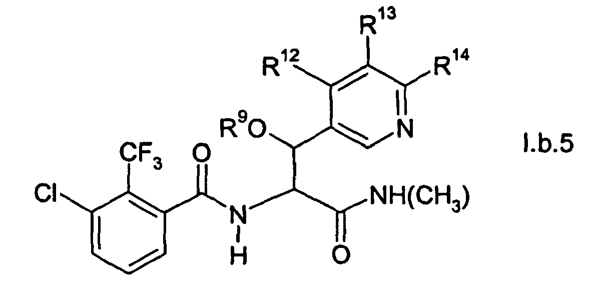

Ebenso außerordentlich bevorzugt sind die Verbindungen der Formel I.b.6, insbesondere die Verbindungen der Formel I.b.6.1 bis I.b.6.192, die sich von den entsprechenden Verbindungen der Formel I.a.1.1 bis I.a.1.192 dadurch unterscheiden, daß Het für Het-2 sowie R³ für Chlor steht.

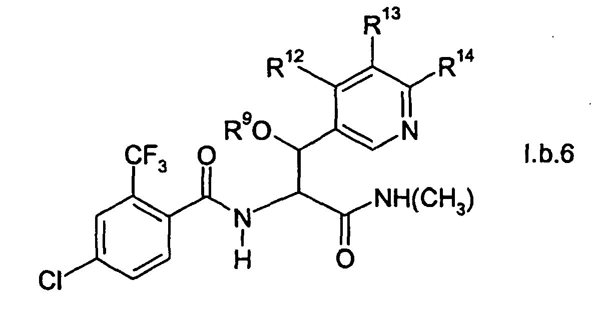

Ebenso außerordentlich bevorzugt sind die Verbindungen der Formel I.b.7. insbesondere die Verbindungen der Formel I.b.7.1 bis I.b.7.192, die sich von den entsprechenden Verbindungen der Formel I.a.1.1 bis I.a.1.192 dadurch unterscheiden, daß Het für Het-2 sowie R³ und R⁴ für Fluor stehen.

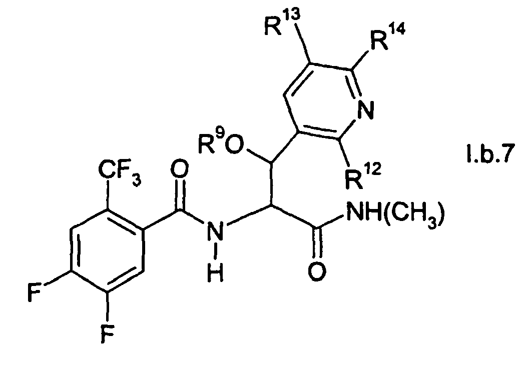

Ebenso außerordentlich bevorzugt sind die Verbindungen der Formel I.b.8, insbesondere die Verbindungen der Formel I.b.8.1 bis I.b.8.192, die sich von den entsprechenden Verbindungen der Formel I.a.1.1 bis I.a.1.192 dadurch unterscheiden, daß Het für Het-2 sowie R¹ für Chlor und R² für CF₃ steht.

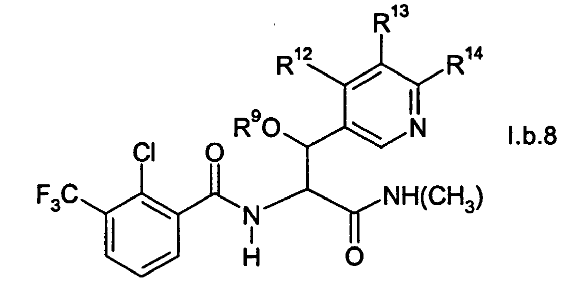

Ebenso außerordentlich bevorzugt sind die Verbindungen der Formel I.b.9, insbesondere die Verbindungen der Formel I.b.9.1 bis I.b.9.192. die sich von den entsprechenden Verbindungen der Formel I.a.1.1 bis I.a.1.1 92 dadurch unterscheiden, daß Het für Het-2 sowie R¹ und R² für Chlor stehen.

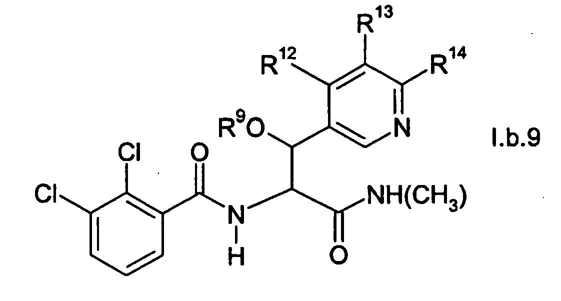

Ebenso außerordentlich bevorzugt sind die Verbindungen der Formel I.b.10, insbesondere die Verbindungen der Formel I.b.10.1 bis I.b.10.192, die sich von den entsprechenden Verbindungen der Formel I.a.1.1 bis I.a.1.192 dadurch unterscheiden, daß Het für Het-2 sowie R¹ und R³ für Chlor stehen.

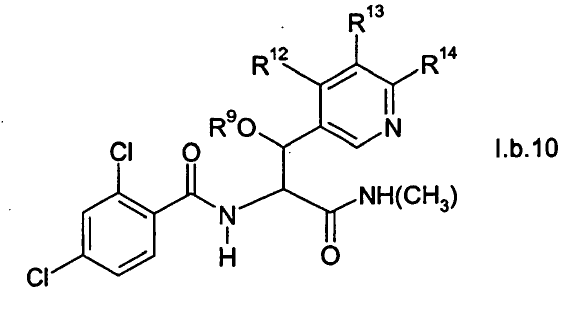

Ebenso außerordentlich bevorzugt sind die Verbindungen der Formel I.c.1, insbesondere die Verbindungen der Formel I.c.1.1 bis I.c.1.192, die sich von den entsprechenden Verbindungen der Formel I.a.1.1 bis I.a.1.192 dadurch unterscheiden, daß Het für Het-3 steht.

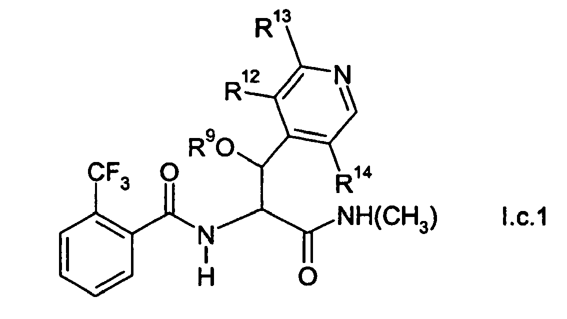

Ebenso außerordentlich bevorzugt sind die Verbindungen der Formel I.c.2. insbesondere die Verbindungen der Formel I.c.2.1 bis I.c.2.192, die sich von den entsprechenden Verbindungen der Formel I.a.1.1 bis I.a.1.192 dadurch unterscheiden, daß Het für Het-3 sowie R² für Fluor steht.

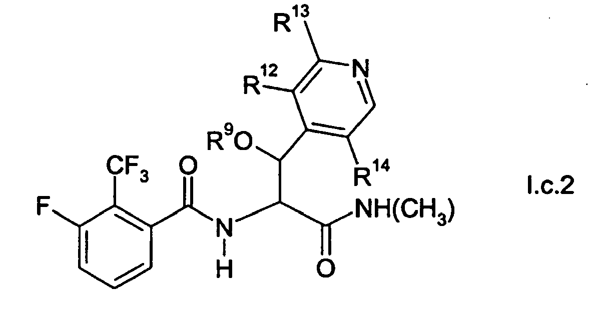

Ebenso außerordentlich bevorzugt sind die Verbindungen der Formel I.c.3, insbesondere die Verbindungen der Formel I.c.3.1 bis I.c.3.192, die sich von den entsprechenden Verbindungen der Formel I.a.1.1 bis I.a.1.192 dadurch unterscheiden, daß Het für Het-3 sowie R³ für Fluor steht.

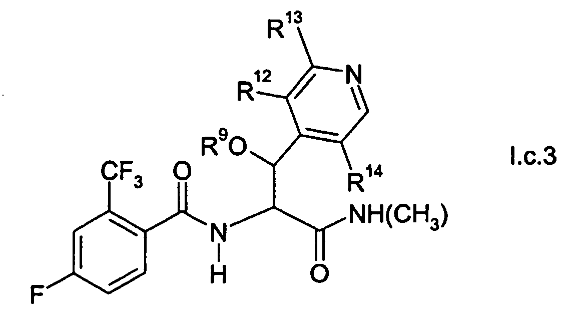

Ebenso außerordentlich bevorzugt sind die Verbindungen der Formel I.c.4, insbesondere die Verbindungen der Formel I.c.4.1 bis I.c.4.192, die sich von den entsprechenden Verbindungen der Formel I.a.1.1 bis I.a.1.192 dadurch unterscheiden, daß Het für Het-3 sowie R⁴ für Fluor steht.

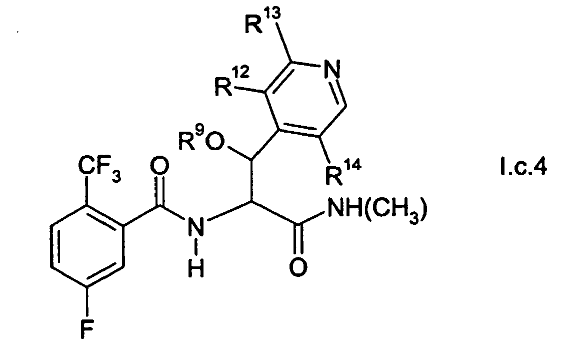

Ebenso außerordentlich bevorzugt sind die Verbindungen der Formel I.c.5. insbesondere die Verbindungen der Formel I.c.5.1 bis I.c.5.192, die sich von den entsprechenden Verbindungen der Formel I.a.1.1 bis I.a.1.192 dadurch unterscheiden, daß Het für Het-3 sowie R² für Chlor steht.

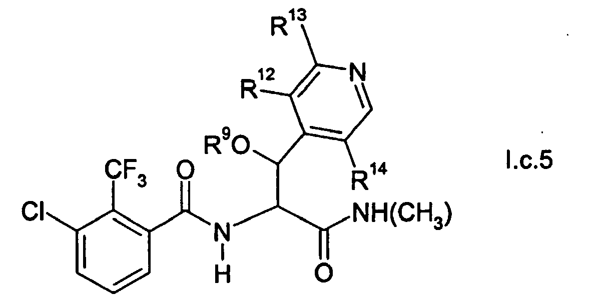

Ebenso außerordentlich bevorzugt sind die Verbindungen der Formel I.c.6, insbesondere die Verbindungen der Formel I.c.6.1 bis I.c.6.192, die sich von den entsprechenden Verbindungen der Formel I.a.1.1 bis I.a.1.192 dadurch unterscheiden, daß Het für Het-3 sowie R³ für Chlor steht.

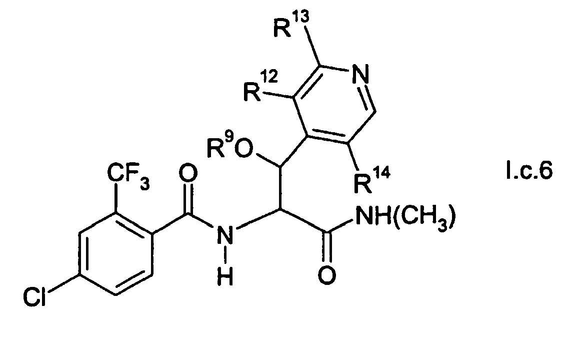

Ebenso außerordentlich bevorzugt sind die Verbindungen der Formel I.c.7, insbesondere die Verbindungen der Formel I.c.7.1 bis I.c.7.192, die sich von den entsprechenden Verbindungen der Formel I.a.1.1 bis I.a.1.192 dadurch unterscheiden, daß Het für Het-3 sowie R³ und R⁴ für Fluor stehen.

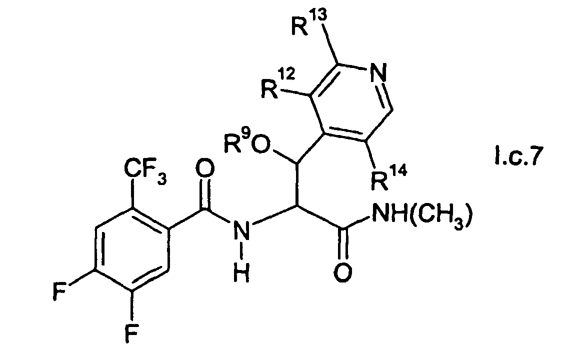

Ebenso außerordentlich bevorzugt sind die Verbindungen der Formel I.c.8, insbesondere die Verbindungen der Formel I.c.8.1 bis I.c.8.192, die sich von den entsprechenden Verbindungen der Formel I.a.1.1 bis I.a.1.192 dadurch unterscheiden, daß Het für Het-3 sowie R' für Chlor und R² für CF₃ steht.

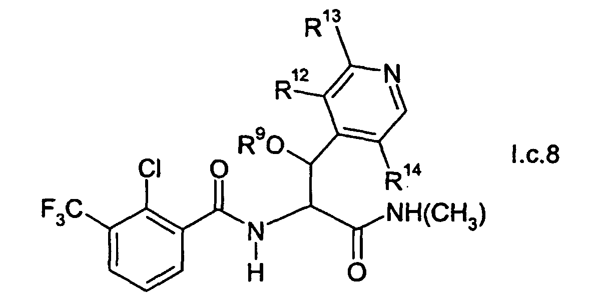

Ebenso außerordentlich bevorzugt sind die Verbindungen der Formel I.c.9. insbesondere die Verbindungen der Formel I.c.9.1 bis I.c.9.192, die sich von den entsprechenden Verbindungen der Formel I.a.1.1 bis I.a.1.192 dadurch unterscheiden, daß Het für Het-3 sowie R¹ und R² für Chlor stehen.

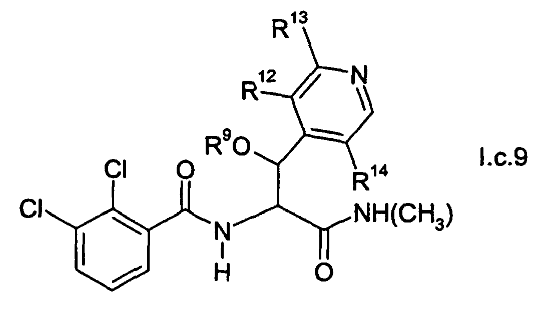

Ebenso außerordentlich bevorzugt sind die Verbindungen der Formel I.c.10, insbesondere die Verbindungen der Formel I.c.10.1 bis I.c.10.192, die sich von den entsprechenden Verbindungen der Formel I.a.1.1 bis I.a.1.192 dadurch unterscheiden, daß Het für Het-3 sowie R' und R³ für Chlor stehen.

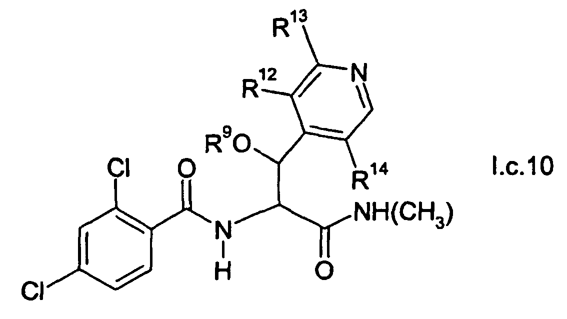

Ebenso außerordentlich bevorzugt sind die Verbindungen der Formel I.d.1, insbesondere die Verbindungen der Formel I.d.1.1 bis I.d.1.192, die sich von den entsprechenden Verbindungen der Formel I.a.1.1 bis I.a.1.192 dadurch unterscheiden, daß Het für Het-4 steht.

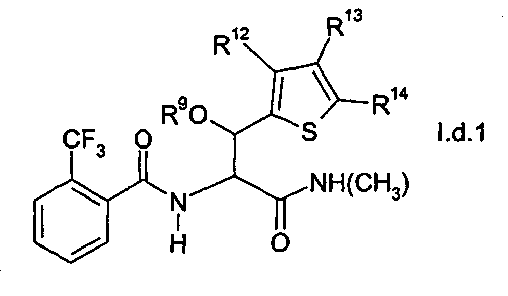

Ebenso außerordentlich bevorzugt sind die Verbindungen der Formel I.d.2, insbesondere die Verbindungen der Formel I.d.2.1 bis I.d.2.192, die sich von den entsprechenden Verbindungen der Formel I.a.1.1 bis I.a.1.192 dadurch unterscheiden, daß Het für Het-4 sowie R² für Fluor steht.

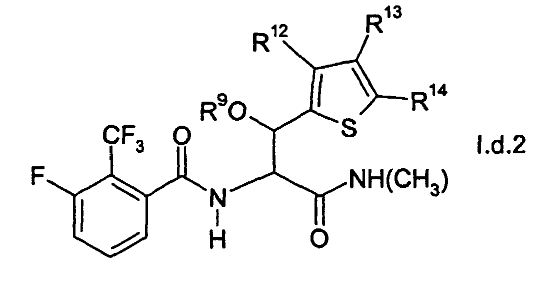

Ebenso außerordentlich bevorzugt sind die Verbindungen der Formel I.d.3, insbesondere die Verbindungen der Formel I.d.3.1 bis I.d.3.192, die sich von den entsprechenden Verbindungen der Formel I.a.1.1 bis I.a.1.192 dadurch unterscheiden, daß Het für Het-4 sowie R³ für Fluor steht.

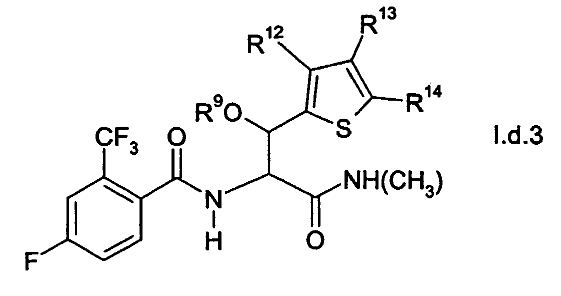

Ebenso außerordentlich bevorzugt sind die Verbindungen der Formel I.d.4, insbesondere die Verbindungen der Formel I.d.4.1 bis I.d.4.192, die sich von den entsprechenden Verbindungen der Formel I.a.1.1 bis I.a.1.192 dadurch unterscheiden, daß Het für Het-4 sowie R⁴ für Fluor steht.

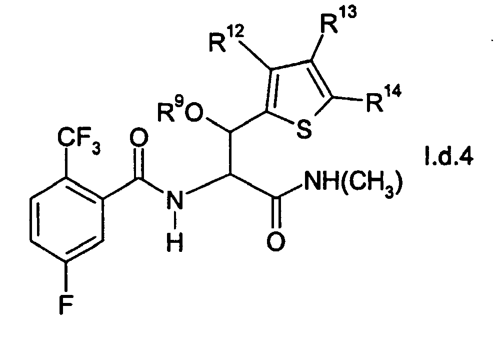

Ebenso außerordentlich bevorzugt sind die Verbindungen der Formel I.d.5, insbesondere die Verbindungen der Formel I.d.5.1 bis I.d.5.192, die sich von den entsprechenden Verbindungen der Formel I.a.1.1 bis I.a.1.192 dadurch unterscheiden, daß Het für Het-4 sowie R² für Chlor steht.

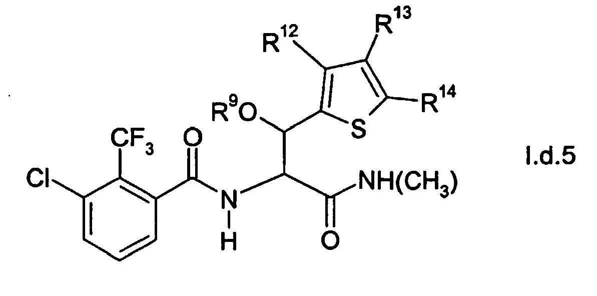

Ebenso außerordentlich bevorzugt sind die Verbindungen der Formel I.d.6, insbesondere die Verbindungen der Formel I.d.6.1 bis I.d.6.192, die sich von den entsprechenden Verbindungen der Formel I.a.1.1 bis I.a.1.192 dadurch unterscheiden, daß Het für Het-4 sowie R³ für Chlor steht.

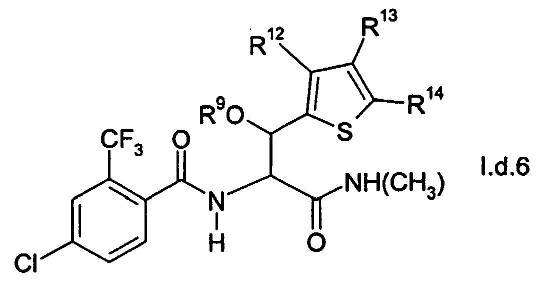

Ebenso außerordentlich bevorzugt sind die Verbindungen der Formel I.d.7. insbesondere die Verbindungen der Formel I.d.7.1 bis I.d.7.192, die sich von den entsprechenden Verbindungen der Formel I.a.1.1 bis I.a.1.192 dadurch unterscheiden, daß Het für Het-4 sowie R³ und R⁴ für Fluor stehen.

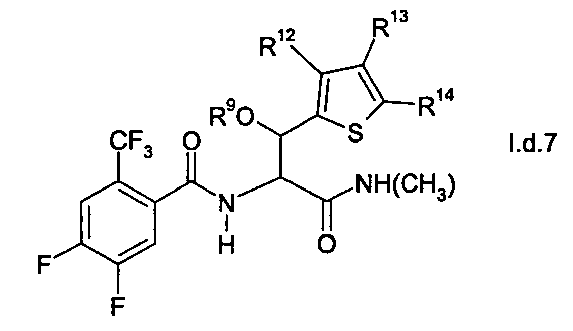

Ebenso außerordentlich bevorzugt sind die Verbindungen der Formel I.d.8, insbesondere die Verbindungen der Formel I.d.8.1 bis I.d.8.192, die sich von den entsprechenden Verbindungen der Formel I.a.1.1 bis I.a.1.192 dadurch unterscheiden, daß Het für Het-4 sowie R¹ für Chlor und R² für CF₃ steht.

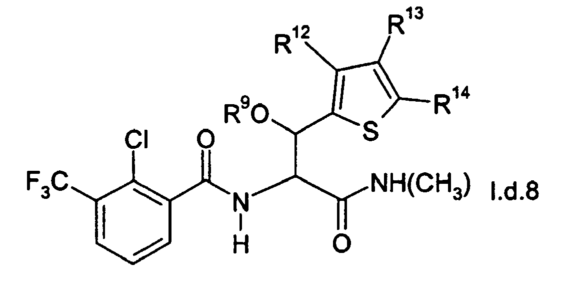

Ebenso außerordentlich bevorzugt sind die Verbindungen der Formel I.d.9, insbesondere die Verbindungen der Formel I.d.9.1 bis I.d.9.192, die sich von den entsprechenden Verbindungen der Formel I.a.1.1 bis I.a.1.192 dadurch unterscheiden, daß Het für Het-4 sowie R¹ und R² für Chlor stehen.

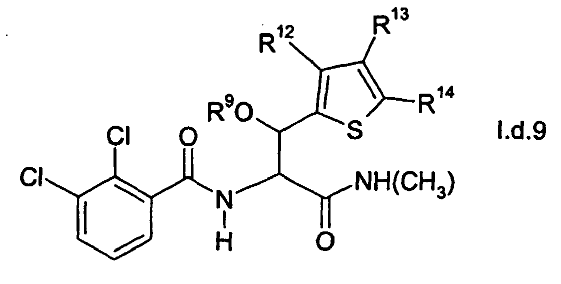

Ebenso außerordentlich bevorzugt sind die Verbindungen der Formel I.d.10, insbesondere die Verbindungen der Formel I.d.10.1 bis I.d.10.192, die sich von den entsprechenden Verbindungen der Formel I.a.1.1 bis I.a.1.192 dadurch unterscheiden, daß Het für Het-4 sowie R¹ und R³ für Chlor stehen.

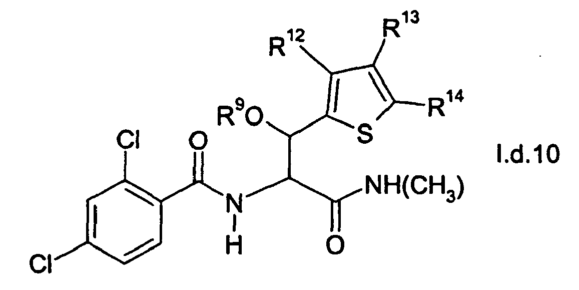

Ebenso außerordentlich bevorzugt sind die Verbindungen der Formel I.e.1, insbesondere die Verbindungen der Formel I.e.1.1 bis I.e.1.192, die sich von den entsprechenden Verbindungen der Formel I.a.1.1 bis I.a.1.192 dadurch unterscheiden, daß Het für Het-5 steht.

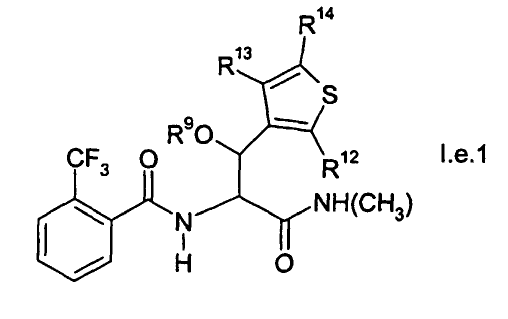

Ebenso außerordentlich bevorzugt sind die Verbindungen der Formel I.e.2, insbesondere die Verbindungen der Formel I.e.2.1 bis I.e.2.192, die sich von den entsprechenden Verbindungen der Formel I.a.1.1 bis I.a.1.192 dadurch unterscheiden, daß Het für Het-5 sowie R² für Fluor steht.

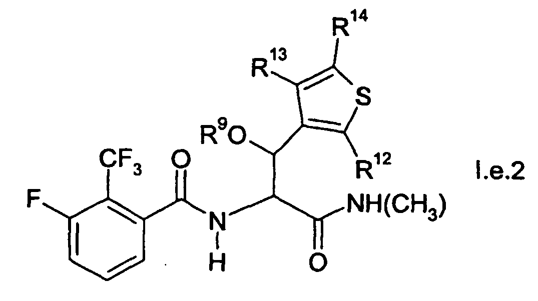

Ebenso außerordentlich bevorzugt sind die Verbindungen der Formel I.e.3, insbesondere die Verbindungen der Formel I.e.3.1 bis I.e.3.192, die sich von den entsprechenden Verbindungen der Formel I.a.1.1 bis I.a.1.192 dadurch unterscheiden, daß Het für Het-5 sowie R³ für Fluor steht.

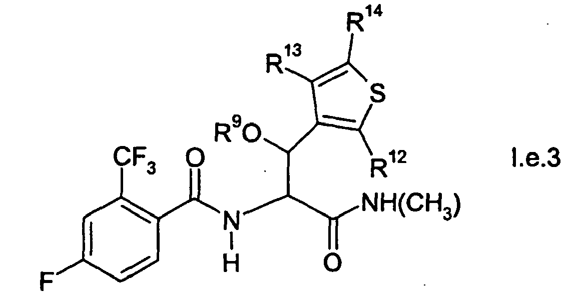

Ebenso außerordentlich bevorzugt sind die Verbindungen der Formel I.e.4, insbesondere die Verbindungen der Formel I.e.4.1 bis I.e.4.192, die sich von den entsprechenden Verbindungen der Formel I.a.1.1 bis I.a.1.192 dadurch unterscheiden, daß Het für Het-5 sowie R⁴ für Fluor steht.

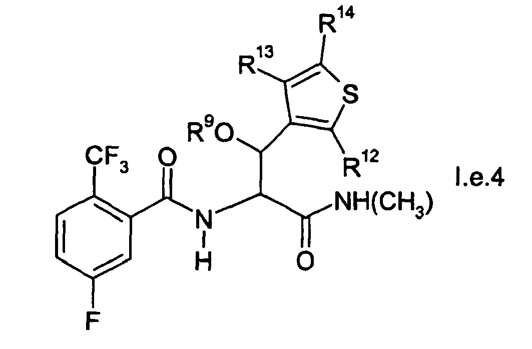

Ebenso außerordentlich bevorzugt sind die Verbindungen der Formel I.e.5, insbesondere die Verbindungen der Formel I.e.5.1 bis I.e.5.192, die sich von den entsprechenden Verbindungen der Formel I.a.1.1 bis I.a.1.192 dadurch unterscheiden, daß Het für Het-5 sowie R² für Chlor steht.

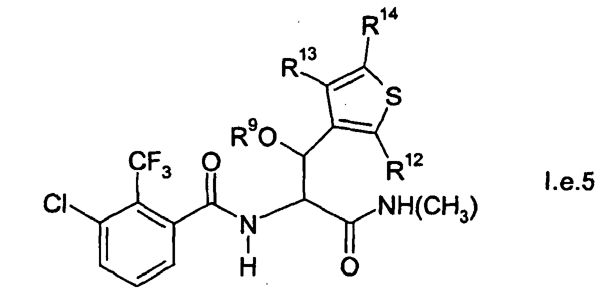

Ebenso außerordentlich bevorzugt sind die Verbindungen der Formel I.e.6, insbesondere die Verbindungen der Formel I.e.6.1 bis I.e.6.192, die sich von den entsprechenden Verbindungen der Formel I.a.1.1 bis I.a.1.192 dadurch unterscheiden, daß Het für Het-5 sowie R³ für Chlor steht.

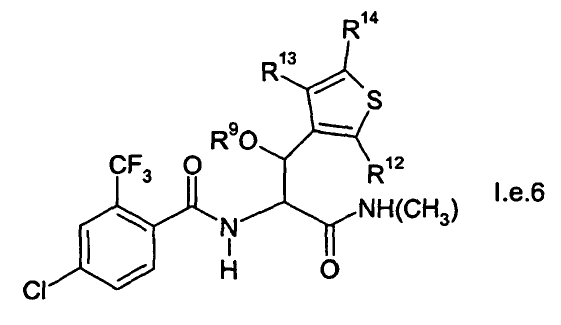

Ebenso außerordentlich bevorzugt sind die Verbindungen der Formel I.e.7, insbesondere die Verbindungen der Formel I.e.7.1 bis I.e.7.192, die sich von den entsprechenden Verbindungen der Formel I.a.1.1 bis I.a.1.192 dadurch unterscheiden, daß Het für Het-5 sowie R³ und R⁴ für Fluor stehen.

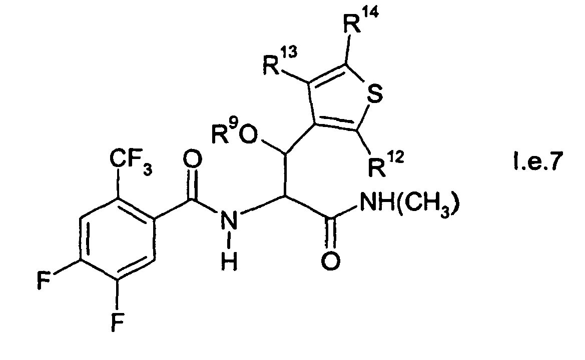

Ebenso außerordentlich bevorzugt sind die Verbindungen der Formel I.e.8, insbesondere die Verbindungen der Formel I.e.8.1 bis I.e.8.192, die sich von den entsprechenden Verbindungen der Formel I.a.1.1 bis I.a.1.192 dadurch unterscheiden, daß Het für Het-5 sowie R¹ für Chlor und R² für CF₃ steht.

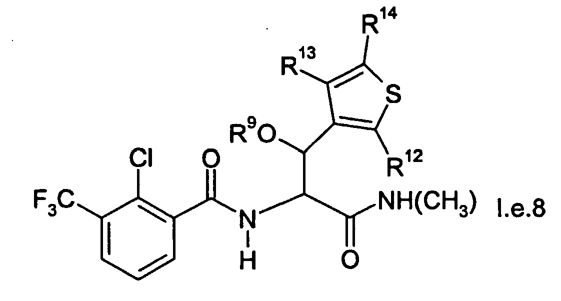

Ebenso außerordentlich bevorzugt sind die Verbindungen der Formel I.e.9, insbesondere die Verbindungen der Formel I.e.9.1 bis I.e.9.192, die sich von den entsprechenden Verbindungen der Formel I.a.1.1 bis I.a.1.192 dadurch unterscheiden, daß Het für Het-5 sowie R' und R² für Chlor stehen.

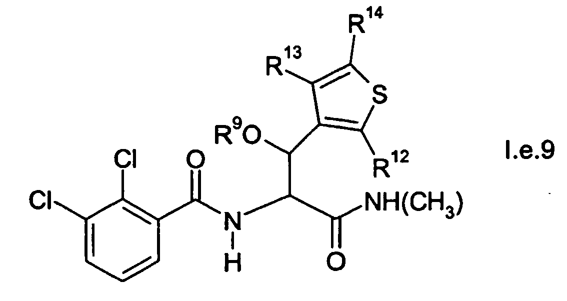

Ebenso außerordentlich bevorzugt sind die Verbindungen der Formel I.e.10, insbesondere die Verbindungen der Formel I.e.10.1 bis I.e.10.192, die sich von den entsprechenden Verbindungen der Formel I.a.1.1 bis I.a.1.192 dadurch unterscheiden, daß Het für Het-5 sowie R¹ und R³ für Chlor stehen.

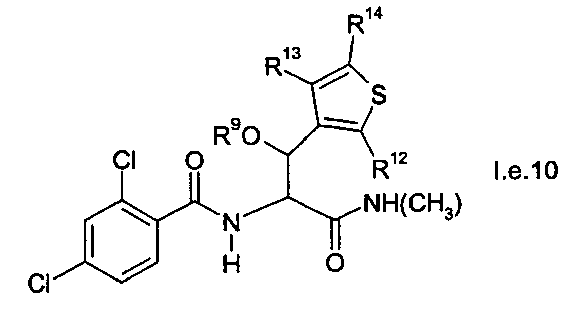

Ebenso außerordentlich bevorzugt sind die Verbindungen der Formel I.f.1, insbesondere die Verbindungen der Formel I.f.1.1 bis I.f.1.192, die sich von den entsprechenden Verbindungen der Formel I.a.1.1 bis I.a.1.192 dadurch unterscheiden, daß Het für Het-6 steht.

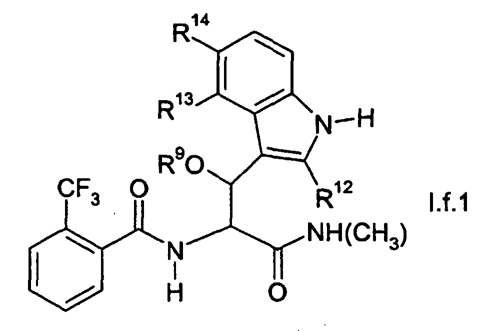

Ebenso außerordentlich bevorzugt sind die Verbindungen der Formel I.f.2, insbesondere die Verbindungen der Formel I.f.2.1 bis I.f.2.192, die sich von den entsprechenden Verbindungen der Formel I.a.1.1 bis I.a.1.192 dadurch unterscheiden, daß Het für Het-6 sowie R² für Fluor steht.

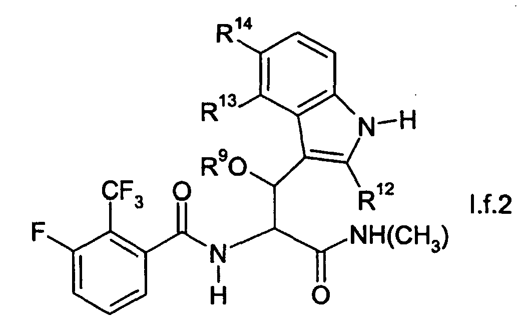

Ebenso außerordentlich bevorzugt sind die Verbindungen der Formel I.f.3, insbesondere die Verbindungen der Formel I.f.3.1 bis I.f.3.192, die sich von den entsprechenden Verbindungen der Formel I.a.1.1 bis I.a.1.192 dadurch unterscheiden, daß Het für Het-6 sowie R³ für Fluor steht.

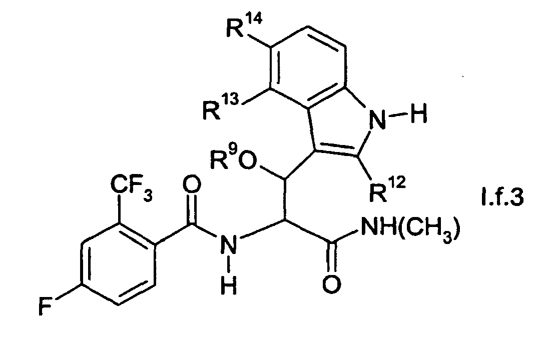

Ebenso außerordentlich bevorzugt sind die Verbindungen der Formel I.f.4, insbesondere die Verbindungen der Formel I.f.4.1 bis I.f.4.192, die sich von den entsprechenden Verbindungen der Formel I.a.1.1 bis I.a.1.192 dadurch unterscheiden, daß Het für Het-6 sowie R⁴ für Fluor steht.

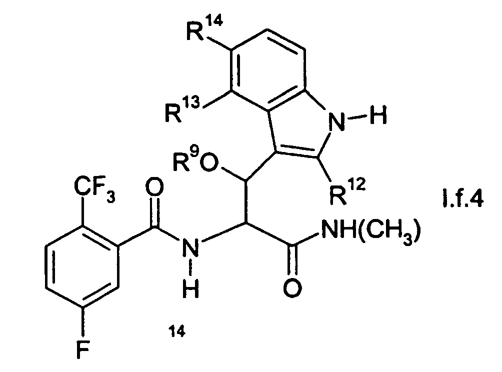

Ebenso außerordentlich bevorzugt sind die Verbindungen der Formel I.f.5, insbesondere die Verbindungen der Formel I.f.5.1 bis I.f.5.192, die sich von den entsprechenden Verbindungen der Formel I.a.1.1 bis I.a.1.192 dadurch unterscheiden, daß Het für Het-6 sowie R² für Chlor steht.

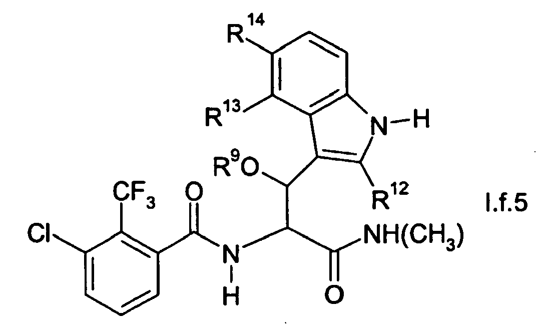

Ebenso außerordentlich bevorzugt sind die Verbindungen der Formel I.f.6, insbesondere die Verbindungen der Formel I.f.6.1 bis I.f.6.192, die sich von den entsprechenden Verbindungen der Formel I.a.1.1 bis I.a.1.192 dadurch unterscheiden, daß Het für Het-6 sowie R³ für Chlor steht.

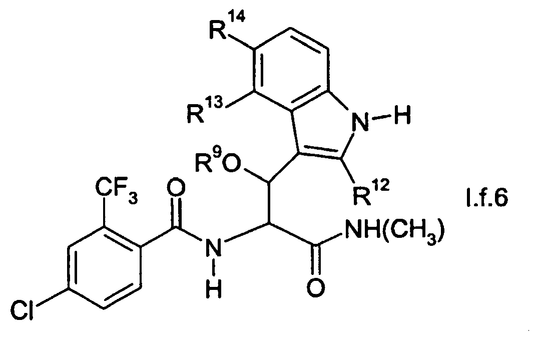

Ebenso außerordentlich bevorzugt sind die Verbindungen der Formel I.f.7, insbesondere die Verbindungen der Formel I.f.7.1 bis I.f.7.192, die sich von den entsprechenden Verbindungen der Formel I.a.1.1 bis I.a.1.192 dadurch unterscheiden, daß Het für Het-6 sowie R³ und R⁴ für Fluor stehen.

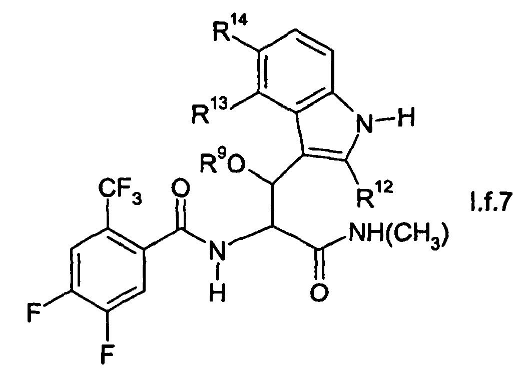

Ebenso außerordentlich bevorzugt sind die Verbindungen der Formel I.f.8, insbesondere die Verbindungen der Formel I.f.8.1 bis I.f.8.192, die sich von den entsprechenden Verbindungen der Formel I.a.1.1 bis I.a.1.192 dadurch unterscheiden, daß Het für Het-6 sowie R' für Chlor und R² für CF₃ steht.

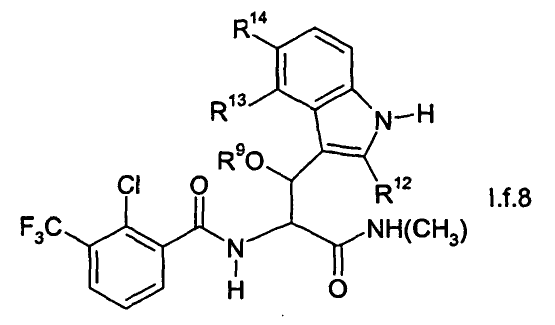

Ebenso außerordentlich bevorzugt sind die Verbindungen der Formel I.f.9, insbesondere die Verbindungen der Formel I.f.9.1 bis I.f.9.192, die sich von den entsprechenden Verbindungen der Formel I.a.1.1 bis I.a.1.192 dadurch unterscheiden, daß Het für Het-6 sowie R¹ und R² für Chlor stehen.

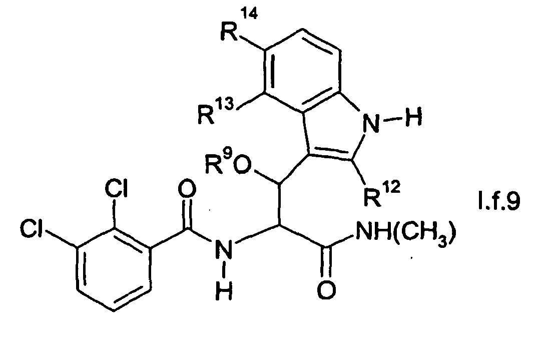

Ebenso außerordentlich bevorzugt sind die Verbindungen der Formel I.f.10, insbesondere die Verbindungen der Formel I.f.10.1 bis I.f.10.192, die sich von den entsprechenden Verbindungen der Formel I.a.1.1 bis I.a.1.192 dadurch unterscheiden, daß Het für Het-6 sowie R¹ und R³ für Chlor stehen.

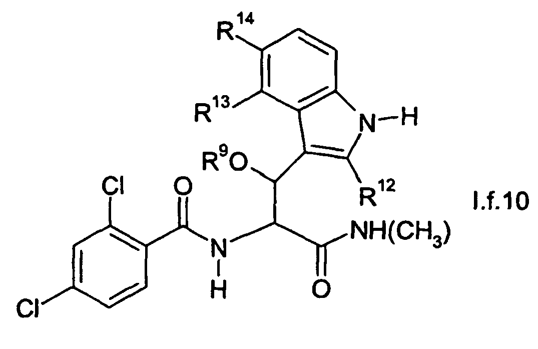

Die benzoylsubstituierten Serin-Amide der Formel I sind auf verschiedene Art und Weise erhältlich, beispielsweise nach folgenden Verfahren:

### Verfahren A

Serinderivate der Formel V werden zunächst mit Benzoesäure(derivate)n der Formel IV zu entsprechenden Benzoylderivaten der Formel III umgesetzt, welche anschließend mit Aminen der Formel II zu den gewünschten benzoylsubstituierten Serin-Amiden der Formel I reagieren:

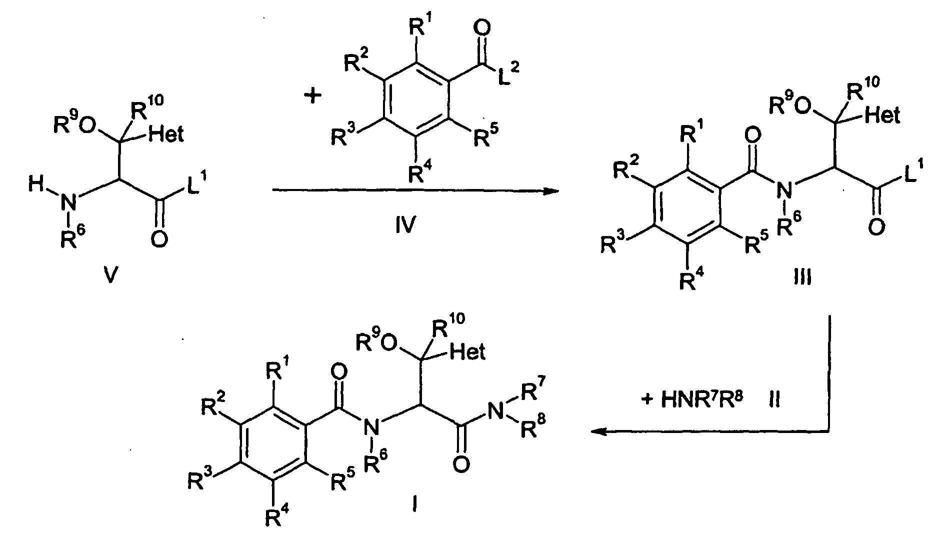

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₄-Alkylsulfonyl, Phosphoryl oder Isoureyl.

Die Umsetzung der Serinderivate der Formel V mit Benzoesäure(derivate)n der Formel IV, wobei L² für Hydroxy steht, zu Benzoylderivaten der Formel III erfolgt in Gegenwart eines Aktivierungsreagenz und einer Base üblicherweise bei Temperaturen von 0 °C bis zum Siedepunkt des Reaktionsgemisches, vorzugsweise 0°C bis 110°C, besonders bevorzugt bei Raumtemperatur, in einem inerten organischen Lösungsmittel [vgI. Bergmann, E. D.; et al., J Chem Soc 1951, 2673; Zhdankin, V. V.; et al., Tetrahedron Lett. 2000, 41 (28), 5299-5302; Martin, S. F. et al., Tetrahedron Lett.1998, 39 (12), 1517-1520; Jursic, B. S. et al., Synth Commun 2001, 31 (4), 555-564; Albrecht, M. et al., Synthesis 2001, (3), 468-472; Yadav, L. D. S. et al., Indian J. Chem B. 41(3),593-595(2002); Clark, J. E. et al., Sythesis (10),891-894 (1991)].

Geeignete Aktivierungsreagenzien sind Kondensationsmittel wie z.B. polystyrolgebundenes Dicyclohexylcarbodiimid, Diisopropylcarbodiimid, Carbonyldiimidazol, Chlorkohlensäureester wie Methylchloroformiat, Ethylchloroformiat, Isoropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin und Pyridin.

Die Basen werden im allgemeinen in äquimolar Mengen eingesetzt. Sie können aber auch im Überschuß oder gegebenenfalls als Lösungsmittel verwendet werden.

Die Edukte werden im allgemeinen in äquimolaren Mengen miteinander umgesetzt. Es kann vorteilhaft sein, IV in einem Überschuß bezogen auf V einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z. T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Umsetzung der Serinderivate der Formel V mit Benzoesäure(derivate)n der Formel IV, wobei L² für Halogen, C₁-C₆-Alkylcarbonyl, C₁-C₆-Alkoxycarbonyl, C₁-C₄-Alkylsulfonyl, Phosphoryl oder Isoureyl steht, zu Benzoylderivaten der Formel III erfolgt in Gegenwart einer Base üblicherweise bei Temperaturen von 0 °C bis zum Siedepunkt des Reaktionsgemisches, vorzugsweise 0°C bis 100°C, besonders bevorzugt bei Raumtemperatur in einem inerten organischen Lösungsmittel [vgI. Bergmann, E. D.; et al., J Chem Soc 1951, 2673; Zhdankin, V. V.; et al., Tetrahedron Lett. 2000, 41 (28), 5299-5302; Martin, S. F. et al., Tetrahedron Lett. 1998, 39 (12), 1517-1520; Jursic, B. S. et al., Synth Commun 2001, 31 (4), 555-564; Albrecht, M. et al., Synthesis 2001, (3), 468-472; Yadav, L. D. S. et al., Indian J. Chem B. 41 (3),593-595(2002); Clark, J. E. et al., Synthesis (10),891-894 (1991)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin und Pyridin.

Die Basen werden im allgemeinen in äquimolar Mengen eingesetzt. Sie können aber auch im Überschuß oder gegebenenfalls als Lösungsmittel verwendet werden.

Die Edukte werden im allgemeinen in äquimolaren Mengen miteinander umgesetzt. Es kann vorteilhaft sein, IV in einem Überschuß bezogen auf V einzusetzen.

Die Aufarbeitung und Isolierung der Produkte kann in an sich bekannter Weise erfolgen.

Natürlich können auch in analoger Weise zunächst die Serinderivate der Formel V mit Aminen der Formel II zu den entsprechenden Amiden umgesetzt werden, welche dann mit Benzoesäure(derivate)n der Formel IV zu den gewünschten benzoylsubstituierten Serin-Amiden der Formel I reagieren.

Die für die Herstellung der Benzoylderivate der Formel III benötigten Serinderivate der Formel V (z.B. mit L¹ = Hydroxy oder C₁-C₆-Alkoxy) sind, auch in enantiomeren- und diastereomerenreiner Form, in der Literatur bekannt oder können gemäß der zitierten Literatur hergestellt werden:
- durch Kondensation von Glycinenolat-Equivalenten mit Heterocyclylaldehyden oder Heterocyclyketonen [Blaser, D. et al., Liebigs Ann. Chem. 10, 1067-1078 (1991); Seethaler, T. et al., Liebigs Ann. Chem. 1, 11-17 (1991); Weltenauer, G. et al., Gazz. Chim. Ital. 81, 162 (1951); Dalla Croce, P. et al., Heterocycles 52(3), 1337-1344 (2000); Van der Werf, A. W. et al., J. Chem. Soc. Chem. Commun. 100, 682-683 (1991); Caddick, S. et al., Tetrahedron 57 (30), 6615-6626 (2001); Owa, T. et al., Chem. Lett. 1, 83-86 (1988); Alker, D. et al., Tetrahedron 54 (22), 6089-6098 (1998); Rousseau, J. F. et al., J. Org. Chem. 63 (8), 2731-2737 (1998); Saeed, A. et al., Tetrahedron 48 (12), 2507-2514 (1992); Dong, L. et al., J. Org. Chem. 67 (14), 4759-4770 (2002)].
- durch Aminohydroxylierung von 3-Heterocyclyl-substituierten Acrylsäure-Derivaten [Zhang, H. X. et al., Tetrahedron Asymmetr. 11(16), 3439-3447 (2000); Fokin, V. V. et al., Angew. Chem. Int. Edit. 40(18), 3455 (2001); Sugiyama, H. et al., Tetrahedron Lett. 43(19), 3489-3492 (2002); Bushey, M. L. et al., J. Org. Chem. 64(9), 2984-2985 (1999); Raatz, D. et al., Synlett (12), 1907-1910 (1999)].
- durch nukleophile Substitution von Abgangsgruppen in 2-Position von 3-Heterocyclyl-3-Hydroxy-Propionsäurederivaten [Owa, T. et al., Chem. Lett. (11), 1873-1874 (1988); Boger, D. L. et al., J. Org. Chem. 57(16), 4331-4333 (1992); Alcaide, B. et al., Tetrahedron Lett. 36(30), 5417-5420 (1995)].
- durch Kondensation von Heterocyclylaldehyden mit Nukleophilen unter Ausbildung von Oxazolinen sowie anschließender Hydrolyse [Evans, D. A. et al., Angew. Chem. Int. Edit. 40(10), 1884-1888 (2001); Ito, Y. et al., Tetrahedron Lett. 26(47), 5781-5784 (1985); Togni, A. et al., J. Organomet. Chem. 381(1), C21-5 (1990); Longmire, J. M. et al., Organometallics 17(20), 4374-4379 (1998); Suga, H. et al., J. Org. Chem. 58(26), 7397-7405 (1993)].
- durch oxidative Cyclisierung von 2-Acylamino-3-Heterocyclyl-Propionsäurederivaten zu Oxazolinen sowie anschließende Hydrolyse (JP10101655).
- durch Hetero-Diels-Alder-Reaktion von Vinyliminen mit Heterocyclyl-Aldehyden zu Tetrahydro-Oxazin und anschließende Hydrolyse [Bongini, A. et al., Tetrahedron Asym. 12(3),439-454 (2001)].

Die für die Herstellung der Benzoylderivate der Formel III benötigten Benzoesäure-(derivate) der Formel IV können käuflich erworben werden oder können analog zu literaturbekannten Vorschrift über eine Grignard-Reaktion aus dem entsprechenden Halogenid hergestellt werden [z.B. A. Mannschuk et al., Angew. Chem. 100, 299 (1988)].

Die Umsetzung der Benzoylderivate der Formel III mit L¹ = Hydroxy bzw. deren Salze mit Aminen der Formel 11 zu den gewünschten benzoylsubstituierten Serin-Amiden der Formel I erfolgt in Gegenwart eines Aktivierungsreagenz und gegebenenfalls in Gegenwart einer Base üblicherweise bei Temperaturen von 0 °C bis zum Siedepunkt des Reaktionsgemisches, vorzugsweise 0°C bis 100°C, besonders bevorzugt bei Raumtemperatur in einem inerten organischen Lösungsmittel. [vgI. Perich, J. W., Johns, R. B., J. Org. Chem. 53 (17), 4103-4105 (1988); Somlai, C. et al., Synthesis (3), 285-287 (1992) ; Gupta, A. et al., J. Chem. Soc. Perkin Trans. 2, 1911 (1990); Guan et al., J. Comb. Chem. 2, 297 (2000)].

Geeignete Aktivierungsreagenzien sind Kondensationsmittel wie z.B. polystyrolgebundenes Dicyclohexylcarbodiimid, Diisopropylcarbodiimid, Carbonyldiimidazol, Chlorkohlensäureester wie Methylchloroformiat, Ethylchloroformiat, Isoropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF, Methanol, Ethanol und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin, Ethyldiisopropylamin, N-methylmorpholin und Pyridin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein II in einem Überschuß bezogen auf III einzusetzen.

Die Aufarbeitung und Isolierung der Produkte kann in an sich bekannter Weise erfolgen.

Die Umsetzung der Benzoylderivate der Formel III mit L¹ = C₁-C₆-Alkoxy mit Aminen der Formel II zu den gewünschten benzoylsubstituierten Serin-Amiden der Formel I erfolgt üblicherweise bei Temperaturen von 0 °C bis zum Siedepunkt des Reaktionsgemisches, vorzugsweise 0°C bis 100°C, besonders bevorzugt bei Raumtemperatur in einem inerten organischen Lösungsmittel gegebenenfalls in Gegenwart einer Base [vgI. Kawahata, N. H. et al., Tetrahedron Lett. 43 (40), 7221-7223 (2002); Takahashi, K. et al., J. Org. Chem. 50 (18), 3414-3415 (1985); Lee, Y. et al., J. Am. Chem. Soc. 121 (36), 8407-8408 (1999)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF, Methanol, Ethanol und Wasser.

Es können auch Gemische der genannten Lösungsmittel verwendet werden.

Die Umsetzung kann gegebenenfalls in Gegenwart einer Base erfolgen. Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin, Ethyldiisopropylamin, N-methylmorpholin und Pyridin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein, II in einem Überschuß bezogen auf III einzusetzen.

Die Aufarbeitung und Isolierung der Produkte kann in an sich bekannter Weise erfolgen.

Die für die Herstellung der benzoylsubstituierten Serin-Amide der Formel I benötigten Amine der Formel II können käuflich erworben werden.

### Verfahren B

Benzoylderivate der Formel III mit R⁹ = Wasserstoff können auch erhalten werden, indem acylierte Glycin-Derivate der Formel VIII, wobei die Acylgruppe eine abspaltbare Schutzgruppe wie Benzyloxycarbonyl (vgl. VIIIa mit Σ = Benzyl) oder tert.-Butyloxycarbonyl (vgl. VIIIa mit Σ = tert-Butyl) sein kann, mit Heterocyclylcarbonyl-Verbindungenen VII zu entsprechenden Aldolprodukten VI kondensiert wird. Anschließend wird die Schutzgruppe abgespalten und die so entstandenen Serinderivate der Formel V mit R⁹ = Wasserstoff mit Benzoesäure(derivate)n der Formel IV acyliert.

Anlog kann auch ein acyliertes Glycin-Derivat der Formel VIII, wobei die Acylgruppe ein substituierter Benzoylrest (vgl. VIIIb) ist, unter Baseneinfluß mit einer Heterocyclylcarbonyl-Verbindung VII zum Benzoylderivat III mit R⁹ = Wasserstoff umgesetzt werden:

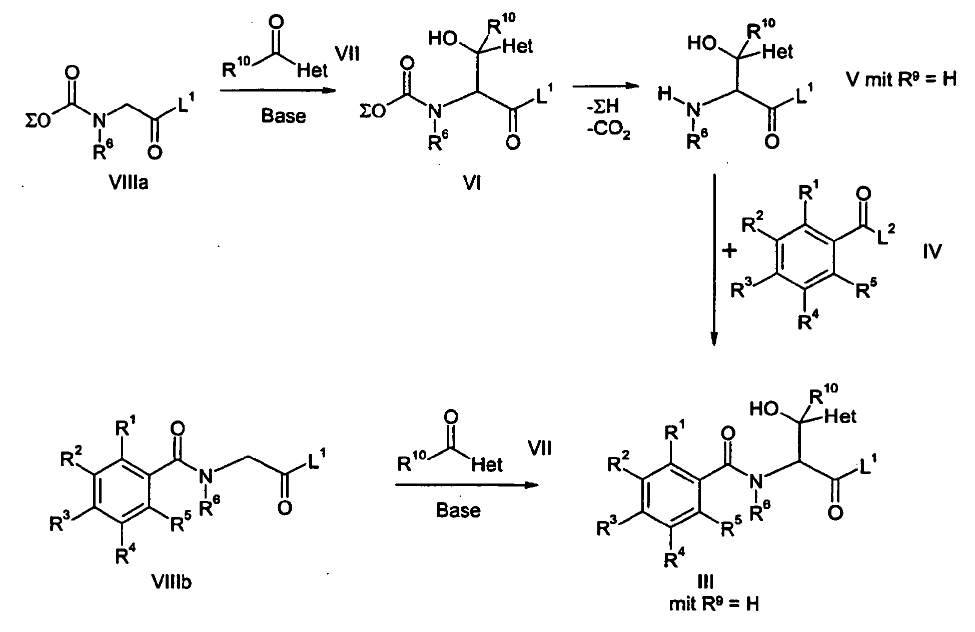

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₄-Alkylsulfonyl, Phosphoryl oder Isoureyl.

Die Umsetzung der Glycinderivate VIII mit Heterocyclyl-Verbindungen VII zum entsprechenden Aldolprodukt VI bzw. Benzoylderivat III mit R⁹ = Wasserstoff erfolgt üblicherweise bei Temperaturen von -100°C bis zum Siedepunkt der Reaktionsmischung, bevorzugt -80°C bis 20°C, insbesondere bevorzugt -80°C bis -20°C, in einem inerten organischen Lösungsmittel in Gegenwart einer Base [vgl. J.-F. Rousseau et al., J. Org. Chem. 63, 2731-2737 (1998)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, sowie Dimethylsulfoxid, Dimethylformamid und Dimethylacetamid, besonders bevorzugt Diethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydride wie Lithiumhydrid, Natriumhydrid, Kaliumhydrid und Calciumhydrid, Alkalimetallazide wie Lithiumhexamethyldisilazid, metallorganische Verbindungen, insbesondere Alkalimetallalkyle wie Methyllithium, Butyllithium und Phenyllithium, sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydrid, Lithiumhexamethyldisilazid und Lithiumdiisopropylamid.

Die Basen werden im allgemeinen in äquimolaren Mengen eingesetzt, sie können aber auch katalytisch, im Überschuß oder gegebenenfalls als Lösungsmittel verwendet werden.

Die Edukte werden im allgemeinen in äquimolaren Mengen miteinander umgesetzt. Es kann vorteilhaft sein, die Base und/oder die Heterocyclylcarbonyl-Verbindungen VII in einem Überschuß bezogen auf die Glycinderivate VIII einzusetzen.

Die Aufarbeitung und Isolierung der Produkte kann in an sich bekannter Weise erfolgen.

Die für die Herstellung der Verbindungen I benötigten Glycinderivate der Formel VIII können käuflich erworben werden, sind in der Literatur bekannt [z.B. H. Pessoa-Mahana et al., Synth. Comm. 32, 1437 (2002] oder können gemäß der zitierten Literatur hergestellt werden.

Die Abspaltung der Schutzgruppe zu Serinderivaten der Formel V mit R⁹ = Wasserstoff erfolgt nach literaturbekannten Methoden[vgl. J.-F. Rousseau et al., J. Org. Chem. 63, 2731-2737 (1998) ); J. M. Andres, Tetrahedron 56, 1523 (2000)];
im Fall von Σ = Benzyl durch Hydrogenolyse, bevorzugt durch Wasserstoff und Pd/C in Methanol;im Fall von Σ = tert.-Butyl durch Säure, bevorzugt Salzsäure in Dioxan.

Die Umsetzung der Serinderivate V mit R⁹= Wasserstoff mit Benzoesäure(derivate)n IV zu Benzoylderivaten III mit R⁹ = Wasserstoff erfolgt üblicherweise analog der unter Verfahren A genannten Umsetzung der Serinderivate der Formel V mit Benzoesäure(derivate)n der Formel IV zu Benzoylderivaten III.

Die Benzoylderivate der Formel III mit R⁹ = Wasserstoff lassen sich anschließend mit Aminen der Formel II analog zu Verfahren A zu den gewünschten benzoylsubstituierten Serin-Amiden der Formel I mit R⁹ = Wasserstoff umsetzen, welche dann mit Verbindungen der Formel IX zu benzoylsubstituierten Serin-Amiden der Formel I derivatisiert werden können [vgl. z.B. Yokokawa, F. et al., Tetrahedron Lett. 42 (34), 5903-5908 (2001); Arrault, A. et al., Tetrahedron Lett. 43( 22), 4041-4044 (2002)].

Ebenso können die Benzoylderivate der Formel III mit R⁹ = Wasserstoff zunächst mit Verbindungen der Formel IX zu weiteren Benzoylderivaten der Formel III derivatisiert werden [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)] und anschließend analog zu Verfahren A mit Aminen der Formel II zu den gewünschten benzoylsubstituierten Serin-Amiden der Formel I umgesetzt werden:

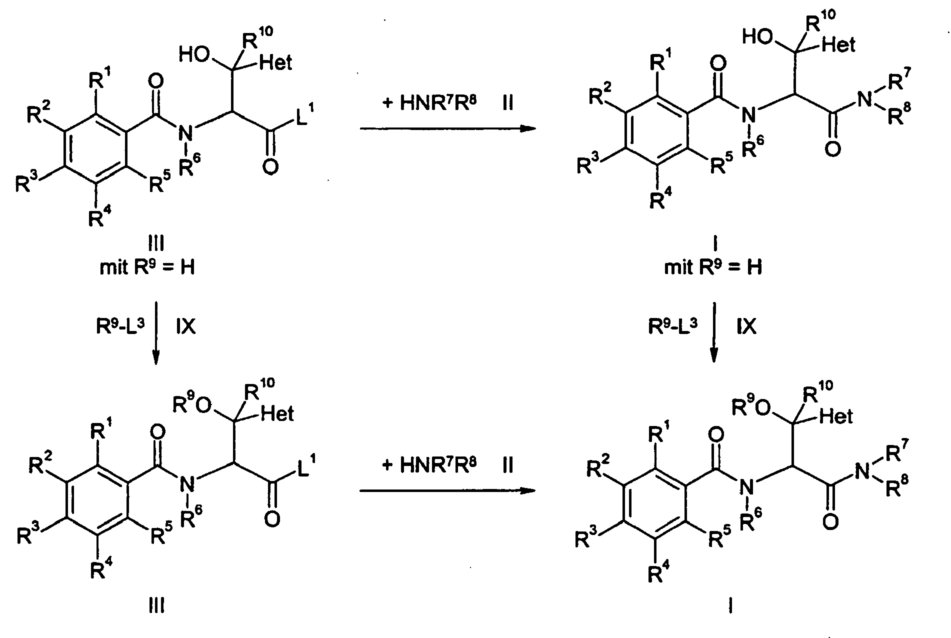

L¹ steht für eine nucleophil verdrängbare Abgangsgruppe, z.B. für Hydroxy oder C₁-C₆-Alkoxy.

L³ steht für eine nucleophil verdrängbare Abgangsgruppe, z.B. für Halogen, Hydroxy, oder C₁-C₆-Alkoxy.

Die Umsetzung der Benzoylderivate der Formel III (gegebenenfalls mit R⁹ = Wasserstoff) mit Aminen der Formel II zu benzoylsubstituierten Serin-Amiden der Formel I (gegebenenfalls mit R⁹ = Wasserstoff) erfolgt üblicherweise analog der unter Verfahren A geschilderten Umsetzung der Benzoylderivate der Formel III mit Aminen der Formel II.

Die Umsetzung der Benzoylderivate der Formel III mit R⁹ = Wasserstoff bzw. der benzoylsubstituierten Serin-Amide der Formel I mit R⁹ = Wasserstoff mit Verbindungen der Formel IX zu Benzoylderivaten der Formel III bzw. benzoylsubstituierten Serin-Amiden der Formel I erfolgt üblicherweise bei Temperaturen von 0°C bis 100°C, vorzugsweise 10°C bis 50°C, in einem inerten organischen Lösungsmittel in Gegenwart einer Base [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Dichlormethan, tert.-Butylmethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Natriumhydrid und Triethylamin.

Die Basen werden im allgemeinen in äquimolaren Mengen eingesetzt, sie können aber auchkatalytisch, im Überschuß oder gegebenenfalls als Lösungsmittel verwendet werden.

Die Edukte werden im allgemeinen in äquimolaren Mengen miteinander umgesetzt. Es kann vorteilhaft sein, die Base und/oder IX in einem Überschuß bezogen auf III bzw. I einzusetzen.

Die Aufarbeitung und Isolierung der Produkte kann in an sich bekannter Weise erfolgen.

Die benötigten Verbindungen der Formel VIII können käuflich erworben werden.

### Verfahren C

Benzoylderivate der Formel III mit R⁹ = Wasserstoff können auch erhalten werden, indem Aminomalonyl-Verbindungen der Formel XI zunächst mit Benzoesäure(derivate)n der Formel IV zu entsprechenden N-Acyl-Aminomalonyl-Verbindungen der Formel X acyliert werden und anschließend mit einer Heterocyclylcarbonyl-Verbindung der Formel VII unter Decarboxylierung kondensiert werden:

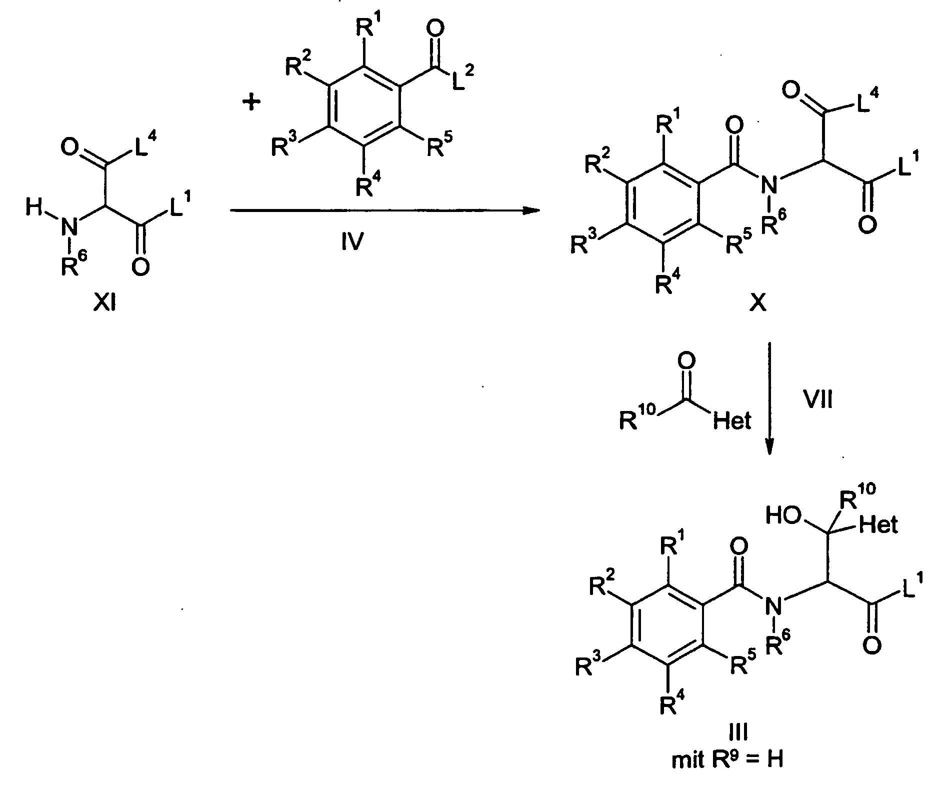

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₆-Alkylsulfonyl, Phosphoryl oder Isoureyl.

L⁴ steht für eine nucleophil verdrängbare Abgangsgruppe, z.B. für Hydroxy oder C₁-C₆-Alkoxy.

Die Acylierung der Aminomalonyl-Verbindungen der Formel XI mit Benzoesäure(derivate)n der Formel IV zu entsprechenden N-Acyl-Aminomalonyl-Verbindungen der Formel X erfolgt üblicherweise analog der unter Verfahren A genannten Umsetzung der Serinderivate der Formel V mit Benzoesäure(derivate)n der Formel IV zu den entsprechenden Benzoylderivaten der Formel III.

Die Umsetzung der N-Acyl-Aminomalonyl-Verbindungen der Formel X mit Heterocyclylcarbonylverbindungen der Formel VII zu Benzoylderivaten der Formel III mit R⁹ = Wasserstoff erfolgt üblicherweise bei Temperaturen von 0°C bis 100°C, vorzugsweise 10°C bis 50°C, in einem inerten organischen Lösungsmittel in Gegenwart einer Base [vgl. z.B. US 4904674; Hellmann, H. et al., Liebigs Ann. Chem. 631, 175-179 (1960)]

Falls L⁴ bei den N-Acyl-Aminomalonyl-Verbindungen der Formel X für C₁-C₆-Akoxy steht, ist es von Vorteil, L⁴ zunächst durch Esterverseifung [z.B. Hellmann, H. et al., Liebigs Ann. Chem. 631, 175-179 (1960)] in eine Hydroxygruppe zu überführen.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid,besonders bevorzugt Diethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin und Diisopropylethylamin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein, die Base in einem Überschuß bezogen auf X einzusetzen.

Die Aufarbeitung und Isolierung der Produkte kann in an sich bekannter Weise erfolgen.

Die so erhaltenen Benzoylderivate der Formel III mit R⁹ = Wasserstoff können anschließend gemäß den voranstehend genannten Verfahren A bzw. B zu den gewünschten benzoylsubstituierten Serin-Amiden der Formel I umgesetzt werden.

Die benötigten Aminomalonyl-Verbindungen der Formel XI können käuflich erworben werden bzw. sind in der Literatur bekannt [z.B. US 4904674; Hellmann, H. et al., Liebigs Ann. Chem. 631, 175-179 (1960)] oder können gemäß der zitierten Literatur hergestellt werden.

Die benötigten heteroyclischen Verbindungen der Formel VII können käuflich erworben werden.

### Verfahren D

Benzoylderivate der Formel III mit R⁹ und R¹⁰ = Wasserstoff können auch erhalten werden, indem Ketoverbindungen der Formel XIII zunächst mit Benzoesäure(derivate)n der Formel IV zu entsprechenden N-Acyl-Ketoverbindungen der Formel XII acyliert werden und anschließend die Ketogruppe reduziert wird [Girard A, Tetrahedron Lett. 37(44),7967-7970(1996); Nojori R., J. Am. Chem. Soc. 111(25),9134-9135(1989); Schmidt U., Synthesis (12),1248-1254 (1992); Bolhofer, A.; J. Am. Chem. Soc. 75, 4469 (1953)]:

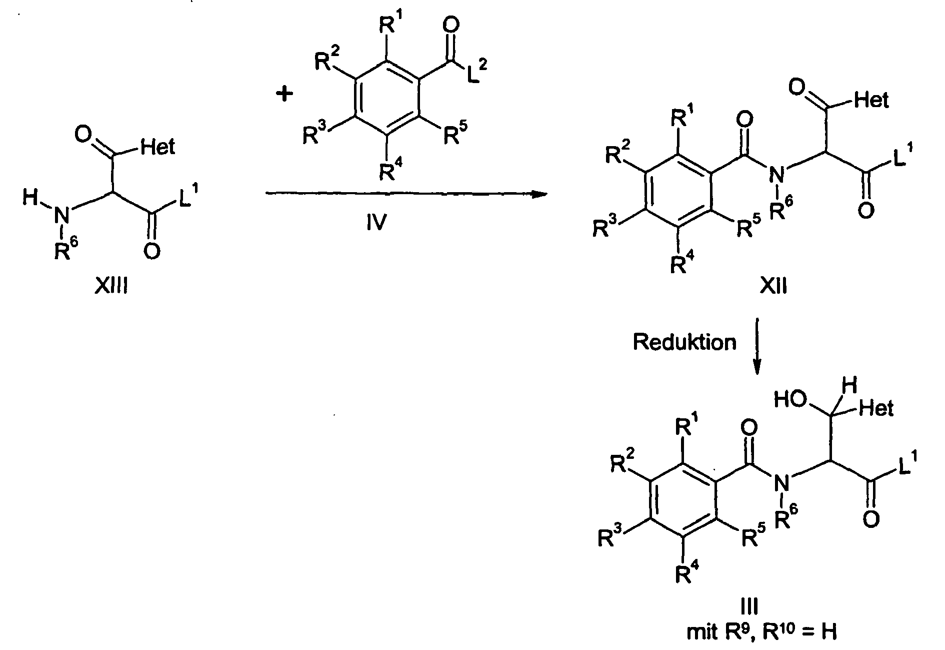

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₆-Alkylsulfonyl, Phosphoryl oder Isoureyl.

Die Acylierung der Ketoverbindungen der Formel XIII mit Benzoesäure(derivate)n der Formel IV zu N-Acyl-Ketoverbindungen der Formel XII erfolgt üblicherweise analog der unter Verfahren A genannten Umsetzung der Serinderivate der Formel V mit Benzoesäure(derivate)n der Formel IV zu den entsprechenden Benzoylderivaten der Formel III.

Die für die Herstellung der Benzoylderivate der Formel III mit R⁹ und R¹⁰ = Wasserstoff benötigten Ketoverbindungen der Formel XIII sind in der Literatur bekannt [WO 02/083111; Boto, A. et al., Tetrahedron Letters 39 (44), 8167-8170 (1988); von Geldern, T. et al., J. of Med. Chem. 39(4), 957-967 (1996); Singh, J. et al., TetrahedronLetters 34 (2), 211-214 (1993); ES 2021557; Maeda, S: et al., Chem. & Pharm. Bull. 32 (7), 2536-2543 (1984); Ito, S. et al., J. of Biol. Chem. 256 (15), 7834-4783 (1981); Vinograd, L. et al., Zhurnal Organicheskoi Khimii 16 (12), 2594-2599 (1980); Castro, A. et al., J. Org. Chem. 35 (8), 2815-2816 (1970); JP 02-172956; Suzuki, M. et al., J. Org. Chem. 38 (20), 3571-3575 (1973) ; Suzuki, M. et al, Synthetic Communications 2 (4), 237-242 (1972)] oder können gemäß der zitierten Literatur hergestellt werden.

Die Reduktion der N-Acyl-Ketoverbindungen der Formel XII zu Benzoylderivaten der Formel III mit R⁹ und R¹⁰ = Wasserstoff erfolgt üblicherweise bei Temperaturen von 0°C bis 100°C, vorzugsweise 20°C bis 80°C, in einem inerten organischen Lösungsmittel in Gegenwart eines Reduktionsmittels.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Toluol, Methylenchlorid oder tert.-Butylmethylether.

Es können auch Gemische der genannten Lösungsmittel verwendet werden.

Als Reduktionsmittel eignen sich z.B. Natriumborhydrid, Zinkborhydrid, Natriumcyanoborhydrid, Litium-triethylborhydrid (Superhydrid®), Litium-tri-sec.butylborhydrid (L-Seiectrid®), Litiumaluminiumhydrid oder Boran [vgl. z.B. WO 00/20424; Marchi, C. et al., Tetrahedron 58 (28), 5699 (2002); Blank, S. et al., Liebigs Ann. Chem. (8), 889-896 (1993); Kuwano, R. et al., J. Org Chem. 63 (10), 3499-3503 (1998); Clariana, J. et al., Tetrahedron 55 (23), 7331-7344 (1999)].

Weiterhin kann die Reduktion auch in Gegenwart von Wasserstoff und eines Katalysator erfolgen. Als Katalysatoren eignen sich z.B. [Ru(BINAP)Cl₂] oder Pd/C [vgl. Noyori, R. et al., J. Am. Chem. Soc. 111 (25), 9134-9135 (1989); Bolhofer, A. et al., J. Am. Chem. Soc. 75, 4469 (1953)].

Daneben kann die Reduktion auch Gegenwart eines Mikroorganismus erfolgen. Als Mikroorganismus eignet sich z.B. *Saccharomyces Rouxii* [vgl. Soukup, M. et al., Helv. Chim. Acta 70, 232 (1987)].

Die N-Acyl-Ketoverbindungen der Formel XII und das jeweilige Reduktionsmittel werden im allgemeinen in äquimolaren Mengen miteinander umgesetzt. Es kann vorteilhaft sein, das Reduktionsmittel in einem Überschuß bezogen auf XII einzusetzen.

Die Aufarbeitung und Isolierung der Produkte kann in an sich bekannter Weise erfolgen.

Die so erhaltenen Benzoylderivate der Formel III mit R⁹ und R¹⁰ = Wasserstoff können anschließend gemäß den voranstehend genannten Verfahren A und B zu den gewünschten benzoylsubstituierten Serin-Amiden der Formel I umgesetzt werden.

Benzoylderivate der Formel III 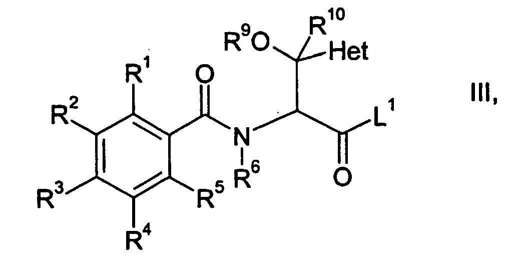 wobei Het, R¹ bis R⁶ sowie R⁹ und R¹⁰ die voranstehend genannten Bedeutungen haben und L¹ für Hydroxy oder C₁-C₆-Alkoxy steht, sind ebenfalls ein Gegenstand der vorliegenden Erfindung.

Die besonders bevorzugten Ausführungsformen der Zwischenprodukte in Bezug auf die Variablen entsprechen denen der Reste Het, R¹ bis R⁶ sowie R⁹ und R¹⁰ der Formel I.

Besonders bevorzugt werden Benzoylderivate der Formel III, in der
- Het: mono- oder bicyclisches Heteroaryl ausgewählt aus der Gruppe Thienyl, Thiazo-lyl, Tetrazolyl, Pyridyl und Indolyl,
wobei die genannten Heteroaryle partiell oder vollständig halogeniert sein können und/oder 1 bis 2 Reste aus der Gruppe Nitro, C₁-C₄-Alkyl, C₁-C₄-Halogenalkyl, Hydroxy, C₁-C₄-Alkoxy, C₁-C₄-Halogenalkoxy, Hydroxy-carbonyl, C₁-C₄-Alkoxycarbonyl, Hydroxycarbonyl-C₁-C₄-alkoxy, C₁-C₄-Alkoxycarbonyl-C₁-C₄-alkoxy, Amino, C₁-C₄-Alkylamino, Di(C₁-C₄-alkyl)-amino, C₁-C₄-Alkylsulfonylamino, C₁-C₄-Halogenalkylsulfonylamino, Ami-nocarbonylamino, (C₁-C₄-Alkylamino)carbonylamino, Di-(C₁-C₄-alkyl)-aminocarbonylamino tragen können;
- R¹: Fluor, Chlor oder CF₃;
- R² und R³: unabhängig voneinander Wasserstoff, Fluor oder Chlor;
- R⁴, R⁵ und R⁶: Wasserstoff;
- R⁹: Wasserstoff, C₁-C₄-Alkylcarbonyl, C₁-C₄-Alkylaminocarbonyl, Di-(C₁-C₄-Alkyl)-aminocarbonyl, Phenylaminocarbonyl, N-(C₁-C₄-alkyl)-N-(phenyl)-aminocarbonyl, SO₂CH₃, SO₂CF₃ oder SO₂(C₆H₅); und
- R¹⁰: Wasserstoff
bedeuten.

Die folgenden Beispiele dienen der Erläuterung der Erfindung.

### Herstellungsbeispiele

### Beispiel 1

### (2S, 3R)-threo-4-Fluor-N-(2-hydroxy-1-methylcarbamoyl-2-thiophen-3-yl-ethyl)-2-trifluoromethyl-benzamid (Tab. 3. Nr. 3.2)

### 1.1) rac-2-Amino-3-oxo-3-thiophen-3-yl-propionsäure-ethylester-Hydrochlorid

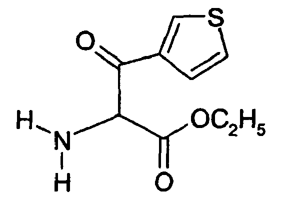

20.2 g (0.180 mol) Kalium-*tert*-butylat wurden unter Schutzgasatmosphäre in THF suspendiert und mit Aceton/Trockeneis-Mischung gekühlt. Anschließend tropfte man 21.8 g (0.082 mol) N-(Diphenylmethylen)-glycinethylester gelöst in THF zu. Nach 40 min wurde die Lösung in einen gekühlten Tropftrichter überführt und zu einer gekühlten Lösung von 11,9 g (0,082 mol)Thiophen-3-carbonylchlorid in THF getropft. Nach 1 h Rühren ließ man die Reaktionsmischung auf 0°C erwärmen. Es wurde mit 10%-iger Salzsäure hydrolysiert und nachgerührt. Anschließend entfernte man die Lösungsmittel, nahm den Rückstand in Wasser aufgenommen und mit wusch mit Methyl*tert-*butylether. Die Wasserphase wurde abgetrennt, eingeengt, der Rückstand mit Methanol versetzt und filtriert. Nach Einengen des Filtrats erhielt man 22.0 g (97 % der Theorie) der Titelverbindung als farbloses Öl.
¹H-NMR (DMSO): δ = 9,2 (br, 3H); 8,90 (s, 1H); 7,70 (m, 1H); 7,65 (m, 1H); 6,05 (s, 1H); 4,25 (m, 2H); 1,15 (t, 3H).

### 1.2) rac-2-(4-Fluor-2-trifluoromethyl-benzoylamino)-3-oxo-3-thiophen-3-yl-propionsäureethylester

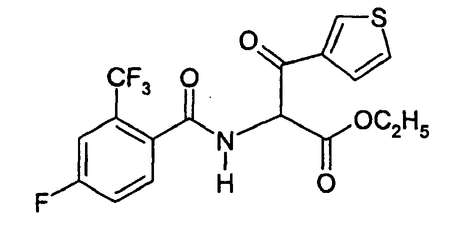

15.0 g (0.060 mol) *rac*-2-Amino-3-oxo-3-thiophen-3-yl-propionsäure-ethylester-Hydrochlorid wurden in Methylenchlorid gelöst und 18.2 g (0.096 mol) Triethylamin zugegeben. Hierzu wurden bei 0°C 13.6 g (0.060 mol) 4-Fluor-2-trifluormethylbenzoylchlorid gelöst in Methylenchlorid zugetropft. Es wurde 1 h bei Raumtemperatur gerührt und anschließend mit 5%iger Salzsäure versetzt. Die organische Phase wurde abgetrennt, gewaschen, getrocknet und das Lösungsmittel entfernt. Man erhielt 13.8 g (57% der Theorie) der Titelverbindung als farblose Kristalle.
¹H-NMR (DMSO): δ = 9,65 (d, 1H); 8,65 (s, 1H); 7,5-7,8 (m, 5H); 6,20 (d, 1H); 4,15 (m, 2H);1,15 (t, 3H).

### 1.3) (2S, 3R)-threo-2-(4-Fluor-2-trifluoromethyl-benzoylamino)-3-hydroxy-3-thiophen-3-yl-propionsäureethylester (Tab. 2, Nr. 2.2)

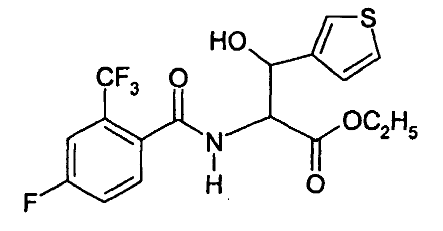

Aus 390 mg Dichloro(P-Cymene)ruthenium(II)-Dimer (RuCl₂Cy)und 690 mg *R*-BINAP in Methylenchlorid und Ethanol stellte man zunächst eine Katalysatormischung her, indem die Mischung 1h auf 50°C erwärmt und anschließend die Lösungsmittel entfernt wurden. Anschließend wurden 13.0 g (0.0322 mol) *rac*-2-(4-Fluor-2-trifluoromethyl-benzoylamino)-3-oxo-3-thiophen-3-yl-propionsäureethylester in Methylenchlorid gelöst. Die Lösung wurde im Ultraschallbad entgast und mit 1.0 g der Katalysatormischung versetzt. Man erhitzte die Reaktionsmischung 100 h bei 50°C unter 100 bar Wasserstoffdruck. Nach Entfernen der Lösungsmittel und chromatographischer Reinigung (Kieselgelsäule, Cyclohexan/Essigsäureethylester) erhielt man 9.5 g (73% der Theorie) der Titelverbindung als farblose Kristalle.
¹H-NMR (DMSO): δ = 8,80 (d, 1H); 7,6 (m, 2H); 7,40 (m, 1H); 7,35 (s, 1H); 7,25 (q, 1H); 7,15 (d, 1H); 5,80 (d, 1H); 5,25 (t, 1H); 4,70 (q, 1H); 4,2 (m, 2H); 1,20 (t, 3H).

### 1.4) (2S, 3R)-threo-4-Fluor-N-(2-hydroxy-1-methylcarbamoyl-2-thiophen-3-yl-ethyl)-2-trifluoromethyl-benzamid (Tab. 3, Nr. 3.2)

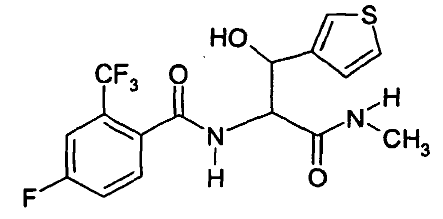

9.50 g (0.0234 mol) (2*S*, 3*R*)-threo-2-(4-Fluor-2-trifluoromethyl-benzoylamino)-3-hydroxy-3-thiophen-3-yl-propionsäureethylester wurden in 200 ml Methanol gelöst. Bei Raumtemperatur leitete man über 3h Methylamin-Gas ein. Nach Entfernen der Lösungsmittel und Verrühren des Rückstandes mit Diisopropylether/Pentan 1:1 erhielt man 8.0 g (88% der Theorie) der Titelverbindung als farblose Kristalle (Schmp. 190 °C).

### Beispiel 2

### (2S,3R)-threo-tert-Butoxycarbonylamino-essigsäure-2-(4-fluoro-2-trifluoromethyl-benzoylamino)-2-methylcarbamoyl-1-thiophen-3-yl-ethylester (Tab. 3, Nr. 3.12)

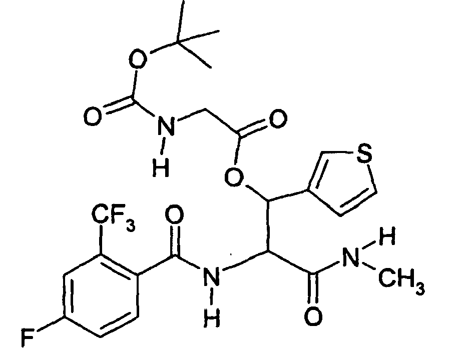

0.50 g (0.0013 mol) (2*S*, 3*R*)-threo-4-Fluor-N-(2-hydroxy-1-methylcarbamoyl-2-thiophen-3-yl-ethyl)-2-trifluoromethyl-benzamid wurden in Methylenchlorid gelöst. Dazu gab man bei 0°C 0.25 g (0.00144 mol) N-Boc-Glycin, 0.063 g (0.00052 mol) Dimethylaminopyridin und 0.28 g (0.0015 mol) 1-3-Dimethylaminopropyl)-3-ethylcarbodiimid-Hydrochlorid. Die Lösung wurde 14 h bei RT gerührt, und anschließend mit Wasser, 10%-iger Salzsäure und gesättigter NaHCO₃-Lösung gewaschen. Anschließend wurde die organische Phase getrocknet und eingeengt. Nach Rühren des Rückstandes mit Pentan/Diisopropylether erhielt man 0.17 g (24% der Theorie) der Titelverbindung als farbloses Pulver (Schmp. 100 °C).

### Beispiel 3

### (2S,3R)-threo-4-Fluoro-N-(1-methylcarbamoyl-2-thiophen-3-yl-2-triethylsilanyloxy-ethyl)-2-trifluoromethyl-benzamid (Tab. 3, Nr. 3.13)

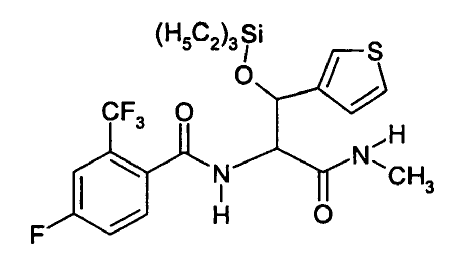

8.0 g (0.0205 mol) (2*S*, 3*R*)-threo-4-Fluor-N-(2-hydroxy-1-methylcarbamoyl-2-thiophen-3-yl-ethyl)-2-trifluoromethyl-benzamid wurden in Dimethylformamid gelöst, 3.60 g (0.0524 mol) Imidazol zugesetzt und 3.66 g (0.0243 mol) Triethylchlorsilan zugetropft. Die Lösung wurde 6 h bei 40°C gerührt, in Wasser eingerührt und mit MTBE/Essigester 1:1 extrahiert. Die organische Phase wurde getrocknet und eingeengt. Nach chromatographischer Reinigung (Kieselgelsäule, Cyclohexan/Essigsäureethylester) erhielt man 5.8 g (58% der Theorie) der Titelverbindung als farbloses Pulver (Schmp. 104 °C).

### Beispiel 4

### rac-erythro-4-Fluor-N-(2-hydroxy-1-methylcarbamoyl-2-pyridin-3-yl-ethyl)-2-trifluoromethyl-benzamid (Tab. 3, Nr. 3.4)

### 4.1) 2-(4-Fluoro-2-trifluoromethyl-benzoylamino)-malonsäure-diethylester

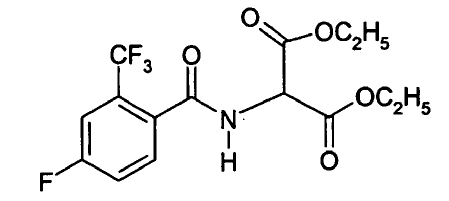

5.00 g (23.6 mmol) Aminomalonsäurediethylester-Hydrochlorid wurden in 2M NaHCO₃-Lösung und Dioxan gelöst. Anschließend tropfte man bei 0°C 6.30 g (28.0 mmol) 4-Fluor-2-Trifluoromethylbenzoylchlorid zugetropft. Es wurde 14 h bei RT gerührt, danach eingeengt und mit Essigester extrahiert. Die organische Phase wurde getrocknet und eingeengt. Man erhielt 9.50 g (99% der Theorie)der Titelverbindung als farbloses Pulver.
¹H-NMR (DMSO): δ = 9,55 (d, 1H); 7,7 (m, 3H); 5,30 (d, 1H); 4,20 (m, 4H); 1,20 (t, 6H).

### 4.2) rac-2-(4-Fluor-2-trifluoromethyl-benzoylamino)-malonsäuremonoethylester

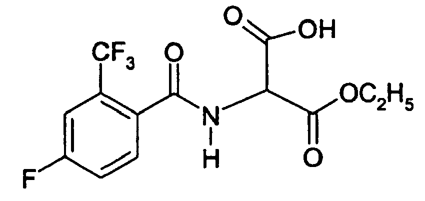

9.00 g (24.6 mmol) 2-(4-Fluor-2-trifluoromethyl-benzoylamino)- malonsäurediethylester wurden in Dioxan gelöst. Anschließend tropfte man 30ml 1M NaOH zu. Es wurde 14 h gerührt, dann die Lösung etwas eingeengt und mit Diethylether extrahiert. Dann gab man zur Wasserphase Essigsäureethylester und tropfte bei 0°C 15 ml 1 M H₂SO₄ zu. Die organische Phase wurde abgetrennt und die wässrige Phase extrahiert. Dann wurden die vereinigten organischen Phasen getrocknet und das Lösungsmittel entfernt. Man erhielt 7.80 g (94% der Theorie) der Titelverbindung als farbloses Pulver.
¹H-NMR (DMSO): 9,30 (d, 1H); 7,7 (m, 3H); 5,10 (d, 1H); 4,10 (m, 2H); 1,20 (t, 3H).

### 4.3) rac-erythro-2-(4-Fluor-2-trifluoromethyl-benzoylamino)-3-hydroxy-3-pyridin-3-yl-propionsäureethylester (Tab. 2, Nr. 2.4)

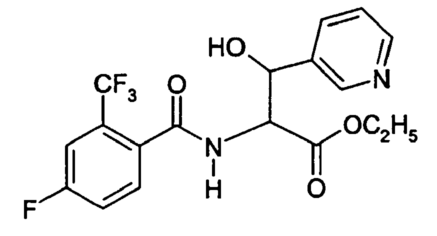

1.99 g (5.90 mmol) *rac*-2-(4-Fluor-2-trifluoromethyl-benzoylamino)-malonsäure-monoethylester wurden in THF gelöst und 0.63 g (5.90 mmol) Pyridin-3-aldehyd und 0.60 g (5.90 mmol) Triethylamin zugetropft. Anschließend wurde 14 h bei RT gerührt. Danach wurde das Lösungsmittel entfernt und der Rückstand in Methylenchlorid aufgenommen. Die Reaktionsmischung wurde mit gesättigter NaHCO₃-Lösung gewaschen, getrocknet und das Lösungsmittel entfernt. Man erhielt 0.31 g (13% der Theorie) der Titelverbindung als farbloses Pulver.
¹H-NMR (DMSO): δ = 9,05 (d, 1H); 8,55 (s, 1H); 8,50 (d, 1H); 7,80 (d, 1H); 7,70 (d, 1H); 7,60 (t, 1H); 7,40 (m, 1H); 7,10 (m, 1H); 6,05 (d, 1H); 4,95 (q, 1H); 4,65 (t, 1H); 4,15 (m, 2H); 1,15 (t, 3H).

### 4.4) rac-etythro-4-Fluor-N-(2-hydroxy-1-methylcarbamoyl-2-pyridin-3-yl-ethyl)-2-trifluoromethyl-benzamid (Tab. 3, Nr. 3.4)

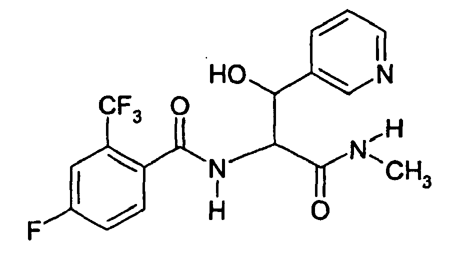

0.30 g (0.75 mmol) rac-erythro-2-(4-Fluor-2-trifluoromethyl-benzoylamino)-3-hydroxy-3-pyridin-3-yl-propionsäureethylester wurden in Methanol gelöst. Abnschließend leitete man über 3 h Methylamin in die Lösung. Danach wurde die Reaktionslösung am Rotationsverdampfer eingeengt. Man erhielt 0.25 g (87% der Theorie) der Titelverbindung als farbloses Pulver, welches ca. 25 % der threo-Verbindung enthielt (Schmp. 181°C).

In den nachfolgenden Tabellen 2 und 3 werden neben den voranstehenden Verbindungen noch weitere Benzoylderivate der Formel III sowie benzoylsubstituierte Serin-Amide der Formel I aufgeführt, die in analoger Weise nach den voranstehend beschriebenen Verfahren hergestellt wurden oder herstellbar sind.

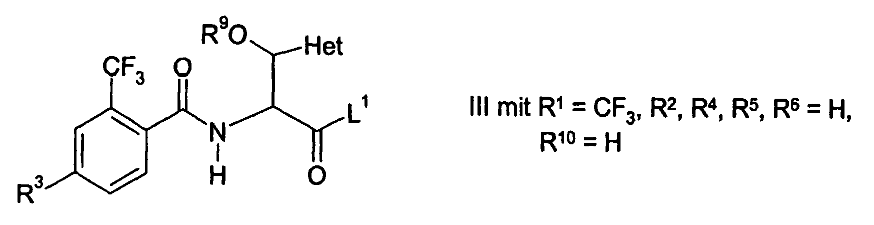

**Tabelle 2**

| Nr. | R³ | R⁹ | Het | L¹ | threo : erythro | Konfiguration | ¹H-NMR, 400 MHz, DMSO-d6, δ [ppm] |
|---|---|---|---|---|---|---|---|
| 2.1 | F | H | 2-Thienyl | OCH₃ | 3 : 2 | rac. | (CDCl₃) 7,5 (m, 1 H); 7,4 (m, 1 H); 7,3 (m, 2H); 7,0 (m, 1 H); 6,8 (m, 1 H); 5,6 (2d, 1H); 5,7 (2dd, 1 H); 3,8 (2s, 3H) |
| 2.2 | F | H | 3-Thienyl | OC₂H₅ | 9 : 1 | 2-*S* | 8,80 (d, 1 H); 7,6 (m, 2H); 7,40 (m, 1 H); 7,35 (s, 1H); 7,25 (q, 1H); 7,15 (d, 1H); 5,80 (d, 1H); 5,25 (t, 1 H); 4,70 (q, 1H); 4,2 (m, 2H); 1,20 (t, 3H) |
| 2.3 | F | H | 2-Pyridyl | OC₂H₅ | 0 : 1 | rac. | 8,9 (d,1H); 8,5 (d, 1H); 7,8 - 7,4 (m, 5H); 7,25 (q, 1H); 5,95 (d, 1H); 5,05 (q, 1 H); 4,95 (t, 1H); 4,0 (m, 2H); 1,05 (t, 3H) |
| 2.4 | F | H | 3-Pyridyl | OC₂H₅ | 1 : 3 | rac. | 9,05 (d, 1 H); 8,55 (s, 1 H); 8,50 (d, 1H); 7,80 (d, 1H); 7,70 (d, 1 H); 7,60 (t, 1 H); 7,40 (m, 1 H); 7,10 (m, 1H); 6,05 (d, 1H); 4,95 (q, 1 H); 4,65 (t, 1 H); 4,15 (m, 2H); 1,15 (t, 3H) |
| 2.5 | F | H | 4-Pyridyl | OC₂H₅ | 2 : 3 | rac. | 9,10 (d, 1H); 8,55 (d 2H); 7,70 (d,1H); 7,60 (t, 1H); 7,40 (d, 2H); 7,20 (q, 1 H); 6,15 (d, 1H); 4,90 (q, 1 H); 4,65 (t, 1H); 4,20 (q, 2H); 1,15 (t, 3H) |

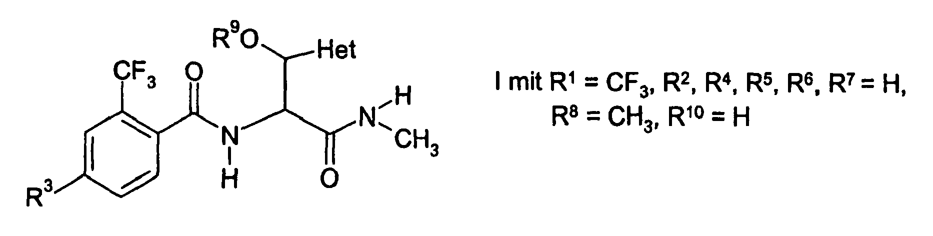

**Tabelle 3**

| Nr. | R³ | R⁹ | Het | threo : erythro | Konfiguration | Schmp. [°C] |
|---|---|---|---|---|---|---|
| 3.1 | F | H | 2-Thienyl | 3 : 2 | rac. | 209 |
| 3.2 | F | H | 3-Thienyl | 9 : 1 | 2-S | 190 |
| 3.3 | F | H | 2-Pyridyl | 0 : 1 | rac. | 180 |
| 3.4 | F | H | 3-Pyridyl | 1 : 3 | rac. | 181 |
| 3.5 | F | H | 4-Pyridyl | 2 : 3 | rac. | 192 |
| 3.6 | F | C(O)C(CH₃)₃ | 2-Thienyl | 4 : 1 | rac. | 204 |
| 3.7 | F | C(O)C(CH₃)₃ | 3-Thienyl | 5 : 1 | 2-*S* | 152 |
| 3.8 | F | C(O)CH₂OCH₃ | 3-Thienyl | 8 : 1 | 2-*S* | 185 |
| 3.9 | F | C(O)CH₂SCH₃ | 3-Thienyl | 1 : 0 | 2-*S* | 155 |
| 3.10 | F | C(O)N(CH₃)₂ | 2-Thienyl | 3 : 3 | rac. | 172 |
| 3.11 | F | C(O)N(CH₃)₂ | 3-Thienyl | 5 : 1 | 2-*S* | 155 |
| 3.12 | F | C(O)CH₂NHC(O)OC(CH₃)₃ | 3-Thienyl | 1 : 0 | 2-S | 100 |
| 3.13 | F | Si(C₂H₅)₃ | 3-Thienyl | 9 : 1 | 2-S | 104 |
| 3.14 | H | H | 1-CH₃-3-Pyrazolyl | 5:1 | rac | 115°C |
| 3.15 | H | H | 1-CH₃-3-Pyrazolyl | 1:1 | rac | m/z 370 |
| 3.16 | H | H | 1-CH₃-2-Imidazolyl | 5:1 | rac | 165°C |
| 3.17 | H | H | 2-Pyridyl | 1:1 | rac | 200°C |
| 3.18 | H | C(O)CH₃ | 1-CH₃-3-Pyrazolyl | 5:1 | rac | 179°C |
| 3.19 | H | C(O)CH₃ | 1-CH₃-3-Pyrazolyl | 1:1 | rac | 166°C |
| 3.20 | H | C(O)N(CH₃)₂ | 1-CH₃-3-Pyrazolyl | 5:1 | rac | m/z 441 |
| 3.21 | H | C(O)N(CH₃)₂ | 1-CH₃-3-Pyrazolyl | 1:1 | rac | m/z 441 |
| 3.22 | H | C(O)CH₂OCH₂CH₂OCH₂CH₂OCH₃ | 1-CH₃-3-Pyrazolyl | 1:1 | rac | m/z 530 |
| 3.23 | H | C(O)CH₃ | 1-CH₃-2-Imidazolyl | 5:1 | rac | m/z 412 |
| 3.24 | H | C(O)N(CH₃)₂ | 1-CH₃-2-Imidazolyl | 5:1 | rac | m/z 441 |
| 3.25 | H | C(O)N(CH₃)₂ | 2-Pyridyl | 1:1 | rac | m/z 438 |
| 3.26 | F | H | 1-(tert-C₄H₉)-Pyrrolyl | 1:1 | rac | 167°C |
| 3.27 | F | H | 2-Thienyl | 1:0 | 2-S | 171°C |
| 3.28 | F | H | 5-Cl-2-Thienyl | 4:1 | 2-S | 166°C |
| 3.29 | F | H | 5-Br-2-Furanyl | 0:1 | rac | 176°C |
| 3.30 | F | H | 5-Br-2-Furanyl | 1:1 | rac | 162°C |
| 3.31 | F | H | 4-Imidazolium-Trifluoracetat | 1:1 | rac | m/z 488 |
| 3.32 | F | H | 4-Imidazolium-Trifluoracetat | 3:2 | rac | m/z 488 |
| 3.33 | F | H | 4-CH₃-5-(1,2,4-Triazolyl) | 1:0 | rac | 247°C |
| 3.34 | F | H | 4-CH₃-5-(1,2,4-Triazolyl) | 0:1 | rac | 102°C |
| 3.35 | F | H | 3-CH₃-2-Thiazolyl | 1:0 | 2-S | m/z 405 |
| 3.36 | F | H | 2-Pyridyl | 1:1 | rac | 170°C |
| 3.37 | F | H | 2-F-3-Pyridyl | 1:0 | rac | 200°C |
| 3.38 | F | H | 2-F-3-Pyridyl | 1:4 | rac | m/z 403 |
| 3.39 | F | H | 2-Cl-3-Pyridyl | 3:1 | rac | m/z 419 |
| 3.40 | F | H | 2-Cl-3-Pyridyl | 9:1 | rac | 211°C |
| 3.41 | F | H | 2-Cl-3-Pyridyl-N-Oxid | 9:1 | rac | 220°C |
| 3.42 | F | H | 4-Cl-3-Pyridyl | 1:0 | rac | 179°C |
| 3.43 | F | H | 1-CH₃-2-Cl-3-Pyridinium-Triflat | 1:0 | rac | m/z 583 |
| 3.44 | F | H | 3-Cl-4-Pyridyl | 1:0 | rac | 232°C |
| 3.45 | F | H | 3-Cl-4-Pyridyl | 0:1 | rac | m/z 419 |
| 3.46 | F | H | 3-Indolyl | 1:0 | 2-S | 121°C |
| 3.47 | F | C(O)CH₃ | 2-Thienyl | 1:0 | 2-S | 220°C |
| 3.48 | F | C(O)CH₃ | 5-Cl-2-Thienyl | 9:1 | 2-S | 208°C |
| 3.49 | F | C(O)CH₃ | 5-Br-2-Furanyl | 0:1 | rac | 207°C |
| 3.50 | F | C(O)CH₃ | 5-Br-2-Furanyl | 1:1 | rac | 175°C |
| 3.51 | F | C(O)CH₃ | 4-CH₃-5-1,2,4-Triazolyl | 0:1 | rac | 129°C |
| 3.52 | F | C(O)CH₃ | 4-CH₃-5-1,2,4-Triazolyl | 1:0 | rac | 220°C |
| 3.53 | F | C(O)CH₃ | 2-Pyridyl | 1:1 | rac | 156°C |
| 3.54 | F | C(O)CH₃ | 2-F-3-Pyridyl | 1:0 | rac | 175°C |
| 3.55 | F | C(O)CH₃ | 2-F-3-Pyridyl | 0:1 | rac | m/z 445 |
| 3.56 | F | C(O)CH₃ | 2-Cl-3-Pyridyl | 1:0 | rac | 161°C |
| 3.57 | F | C(O)CH₃ | 4-Cl-3-Pyridyl | 1:0 | rac | 180°C |
| 3.58 | F | C(O)CH₃ | 3-Cl-4-Pyridyl | 0:1 | rac | 254°C |
| 3.59 | F | C(O)CH₃ | 3-Cl-4-Pyridyl | 1:0 | rac | 190°C |
| 3.60 | F | C(O)CH₂OCH₂CH₂OCH₂CH₂OCH₃ | 2-Pyridyl | 1:2 | rac | m/z 545 |
| 3.61 | F | C(O)CH₂Cl | 2-Thienyl | 1:0 | 2-S | 180°C |
| 3.62 | F | C(O)CH₂Cl | 5-Cl-2-Thienyl | 1:0 | 2-S | 182°C |
| 3.63 | F | C(O)CH₂SCH₃ | 2-Thienyl | 1:0 | 2-S | 177°C |
| 3.64 | F | C(O)CH₂SCH₃ | 5-Cl-2-Thienyl | 9:1 | 2-S | 152°C |
| 3.65 | F | C(O)N(CH₃)₂ | 2-Thienyl | 1:0 | 2-S | 121°C |
| 3.66 | F | C(O)N(CH₃)₂ | 5-Cl-2-Thienyl | 5:1 | 2-S | 142°C |
| 3.67 | F | C(O)N(CH₃)₂ | 5-Br-2-Furanyl | 1:1 | rac | 186°C |
| 3.68 | F | C(O)N(CH₃)₂ | 5-Br-2-Furanyl | 0:1 | rac | 194°C |
| 3.69 | F | C(O)N(CH₃)₂ | 2-Pyridyl | 1:1 | rac | 147°C |
| 3.70 | F | C(O)N(CH₃)₂ | 2-F-3-Pyridyl | 1:3 | rac | m/z 474 |
| 3.71 | F | C(O)N(CH₃)₂ | 2-F-3-Pyridyl | 1:0 | rac | 187°C |
| 3.72 | F | C(O)N(CH₃)₂ | 2-Cl-3-Pyridyl | 2:8 | rac | m/z 490 |
| 3.73 | F | C(O)N(CH₃)₂ | 2-Cl-3-Pyridyl | 9:1 | rac | 207°C |
| 3.74 | F | C(O)N(CH₃)₂ | 4-Cl-3-Pyridyl | 1:0 | rac | 180°C |
| 3.75 | F | C(O)N(CH₃)₂ | 2-Cl-3-Pyridyl-N-Oxid | 1:0 | rac | m/z 506 |
| 3.76 | F | C(O)N(CH₃)₂ | 1-CH₃-2-Cl-3-Pyridinium-Triflat | 1:0 | rac | m/z 654 |
| 3.77 | F | C(O)N(CH₃)₂ | 3-Cl-4-Pyridyl | 1:0 | rac | 202°C |
| 3.78 | F | C(O)N(CH₃)₂ | 3-Cl-4-Pyridyl | 0:1 | rac | m/z 490 |
| 3.79 | F | Si(C₂H₅)₃ | 5-Cl-2-Thienyl | 1:0 | 2-S | 132°C |
| 3.80 | F | Si(CH₃)₂(tert-C₄H₉) | 1-CH₃-2-Cl-3-Pyridinium-Triflat | 1:2 | rac | 132°C |
| 3.81 | F | Si(C₂H₅)₃ | 5-(p-CH₃-Phenyl)-2-thienyl | 1:0 | 2-S | m/z 594 |
| 3.82 | SCH₃ | Si(CH₃)₂(tert-C₄H₉) | 2-Cl-3-Pyridyl | 0:1 | rac | 141°C |

### Biologische Wirksamkeit

Die benzoylsubstituierten Serin-Amide der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht:
Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile eines Wirkstoffs der Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs der Formel I enthält.
II. 20 Gewichtsteile eines Wirkstoffs der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs der Formel I enthält.
III. 20 Gewichtsteile eines Wirkstoffs der Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs der Formel I enthält.
IV. 20 Gewichtsteile eines Wirkstoffs der Formel I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs der Formel I enthält.
V. 3 Gewichtsteile eines Wirkstoffs der Formel I werden mit 97 Gewichtsteilen feinteiligem Kaolin vermischt. Man erhält auf diese Weise ein Stäubemittel, das 3 Gew.-% des Wirkstoffs der Formel I enthält.
VI. 20 Gewichtsteile eines Wirkstoffs der Formel I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil eines Wirkstoffs der Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil eines Wirkstoffs der Formel I wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.).

Zur Verbreiterung des Wirkungsspektrums und zur Erzielung synergistischer Effekte können die benzoylsubstituierten Serin-Amide der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3- cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Emährungs- und Spurenelementmängeln eingesetzt werden. Es können auch nichtphytotoxische Öle und Ölkonzentrate zugesetzt werden.

### Anwendungsbeispiele

Die herbizide Wirkung der benzoylsubstituierten Serin-Amide der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 1,0 bzw. 0,5 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Amaranthus retroflexus | Fuchsschwanz | pig weed |
| Chenopodium album | Weißer Gänsefuß | lambsquaters |
| Setaria viridis | Grüne Borstenhirse | green foxtail |

Bei Aufwandmengen von 0,5 kg/ha zeigte die Verbindung 3.10 (Tabelle 3) im Nachauflauf eine sehr gute Wirkung gegen die unerwünschten Pflanzen Fuchsschwanz, weißer Gänsefuß und grüne Borstenhirse.

Weiterhin bekämpfte Verbindung 3.11 (Tabelle 3) im Nachauflauf bei Aufwandmengen von 1,00 kg/ha die Schadpflanzen Fuchsschwanz, weißer Gänsefuß und grüne Borstenhirse sehr gut.

Die Wirkung von Verbindung 3.13 (Tabelle 3) im Nachauflauf bei Aufwandmengen von 1,00 kg/ha auf die unerwünschten Pflanzen Fuchsschwanz, weißer Gänsefuß und grüne Borstenhirse war sehr gut.

Bei Aufwandmengen von 0,5 kg/ha zeigten die Verbindungen 3.21, 3.28, 3.36, 3.41, 3.53, 3.56, 3.57, 3.59, 3.63, 3.65, 3.66, 3.69, 3.71, 3.74 und 3.75 (Tabelle 3) im Nach-auflauf eine sehr gute Wirkung gegen die unerwünschten Pflanzen Fuchsschwanz, weißer Gänsefuß und grüne Borstenhirse.

Weiterhin bekämpften Verbindungen 3.14, 3.20, 3.35, 3.39, 3.40 und 3.79 (Tabelle 3) im Nachauflauf bei Aufwandsmengen von 1,0 kg/ha die Schadpflanzen Fuchsschwanz, weißer Gänsefuß und grüne Borstenhirse sehr gut.

## Patentansprüche

1. Benzoylsubstituierte Serin-Amide der Formel I 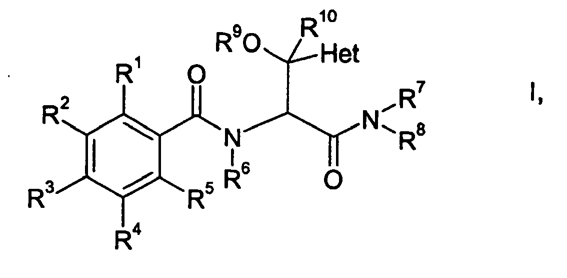 in der die Variablen die folgenden Bedeutungen haben:
Het mono- oder bicyclisches Heteroaryl mit 5 bis 10 Ringgliedern enthaltend 1 bis 4 Heteroatome aus der Gruppe Stickstoff, Sauerstoff und Schwefel, das partiell oder vollständig halogeniert sein kann und/oder 1 bis 3 Reste aus der Gruppe Cyano, Nitro, C₁-C₆-Alkyl, C₁-C₆-Halogenalkyl, Hydroxy, C₁-C₆-Alkoxy, C₁-C₆-Halogenalkoxy, Hydroxycarbonyl, C₁-C₆-Alkoxycarbonyl, Hydroxycarbonyl-C₁-C₆-alkoxy, C₁-C₆-Alkoxycarbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkylsulfonylamino, Aminocarbonylamino, (C₁-C₆-Alkylamino)-carbonylamino, Di-(C₁-C₆-alkyl)-aminocarbonylamino, Aryl und Aryl(C₁-C₆-alkyl) tragen kann;
R¹ Halogen, Cyano, C₁-C₆-Alkyl, C₁-C₆-Halogenalkyl oder C₁-C₆-Halogenalkoxy;
R², R³, R⁴, R⁵ Wasserstoff, Halogen, Cyano, C₁-C₆-Alkyl, C₁-C₆-Halogenalkyl, C₁-C₆-Alkoxy oder C₁-C₆-Halogenalkoxy;
R⁶, R⁷ Wasserstoff, Hydroxy oder C₁-C₆-Alkoxy;
R⁸ C₁-C₆-Alkyl, C₁-C₄-Cyanoalkyl oder C₁-C₆-Halogenalkyl;
R⁹ Wasserstoff, C₁-C₆-Alkyl, C₃-C₆-Cycloalkyl, C₃-C₆-Alkenyl, C₃-C₆-Alkinyl, C₃-C₆-Halogenalkenyl, C₃-C₆-Halogenalkinyl, Formyl, C₁-C₆-Alkylcarbonyl, C₃-C₆-Cycloalkylcarbonyl, C₂-C₆-Alkenylcarbonyl, C₂-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Alkylaminocarbonyl, C₃-C₆-Alkenylaminocarbonyl, C₃-C₆-Alkinylamino-carbonyl, C₁-C₆-Alkylsulfonylaminocarbonyl, Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkylamino)-imino-C₁-C₆-alkyl oder Tri-C₁-C₄-alkylsilyl,
wobei die genannten Alkyl-, Cycloalkyl- und Alkoxyreste partiell oder vollständig halogeniert sein können und/oder eine bis drei der folgen-den Gruppen tragen können: Cyano, Hydroxy, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Di-(C₁-C₄-alkyl)-amino, C₁-C₄-Alkyl-C₁-C₆-alkoxycarbonylamino, C₁-C₄-Alkylcarbonyl, Hydroxycarbonyl, C₁-C₄-Alkoxycarbonyl, Aminocarbonyl, C₁-C₄-Alkylaminocarbonyl, Di-(C₁-C₄-alkyl)-aminocarbonyl oder C₁-C₄-Alkylcarbonyloxy;
Phenyl, Phenyl-C₁-C₆-alkyl, Phenylcarbonyl, Phenylcarbohyl-C₁-C₆-alkyl, Phenoxycarbonyl, Phenylaminocarbonyl, Phenylsulfonylaminocarbonyl, N-(C₁-C₆-Alkyl)-N-(phenyl)-aminocarbonyl, Phenyl-C₁-C₆-alkylcarbonyl, Heterocyclyl, Heterocyclyl-C₁-C₆-alkyl, Heterocyclylcarbonyl, Heterocyclylcar-bonyl-C₁-C₆-alkyl, Heterocyclyloxycarbonyl, Heterocyclylaminocarbonyl, Heterocyclylsulfonylaminocarbonyl, N-(C₁-C₆-Alkyl)-N-(heterocyclyl)-aminocarbonyl, oder Heterocyclyl-C₁-C₆-alkylcarbonyl,
wobei der Phenyl- und der Heterocyclyl-Rest der 17 letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogen-alkoxy; oder
SO₂R¹¹;
R¹⁰ Wasserstoff oder C₁-C₆-Alkyl;
R¹¹ C₁-C₆-Alkyl, C₁-C₆-Halogenalkyl oder Phenyl,
wobei der Phenylrest partiell oder vollständig halogeniert sein kann und/oder eine bis drei der folgenden Gruppen tragen kann: C₁-C₆-Alkyl, C₁-C₆-Halogen-alkyl oder C₁-C₆-Alkoxy;
sowie deren landwirtschaftlich brauchbaren Salze.

2. Benzoylsubstituierte Serin-Amide der Formel I gemäß Anspruch 1, wobei Het für mono- oder bicyclisches Heteroaryl ausgewählt aus der Gruppe Furyl, Thienyl, Pyrazolyl, Imidazolyl, Thiazolyl, Triazolyl, Tetrazolyl, Pyridyl, Pyrimidinyl, Chinoliny und Indolyl,
wobei die genannten Heteroaryle partiell oder vollständig halogeniert sein können und/oder 1 bis 3 Reste aus der Gruppe Nitro, C₁-C₄-Alkyl, C₁-C₄-Halogenalkyl, Hydroxy, C₁-C₄-Alkoxy, C₁-C₄-Halogenalkoxy, Hydroxycarbonyl, C₁-C₆-Alkoxycarbonyl, Hydroxycarbonyl-C₁-C₆-alkoxy, C₁-C₆-Alkoxycarbonyl-C₁-C₆-alkoxy, Amino, C₁-C₆-Alkylamino, Di(C₁-C₆-alkyl)amino, C₁-C₄-Alkylsulfonylamino, C₁-C₄-Halogenalkylsulfonylamino, Aminocarbonylamino, (C₁-C₄-Alkylamino)carbonylamino und Di-(C₁-C₄-alkyl)-aminocarbonylamino tragen können,
steht.

3. Benzoylsubstituierte Serin-Amide der Formel I gemäß Anspruch 1 oder 2, wobei R¹ für Halogen oder C₁-C₆-Halogenalkyl steht.

4. Benzoylsubstituierte Serin-Amide der Formel I gemäß Ansprüchen 1 bis 3, wobei R² und R³ unabhängig voneinander für Wasserstoff, Halogen oder C₁-C₆-Halogenalkyl stehen.

5. Benzoylsubstituierte Serin-Amide der Formel I gemäß Ansprüchen 1 bis 4, wobei R⁴, R⁵, R⁶, R⁷ und R¹⁰ für Wasserstoff stehen.

6. Verfahren zur Herstellung von benzoylsubstituierten Serin-Amiden der Formel I gemäß Anspruch 1, **dadurch gekennzeichnet dass** Serinderivate der Formel V 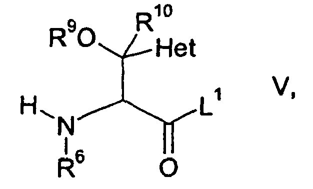 wobei Het sowie R⁶, R⁹ und R¹⁰ die unter Anspruch 1 genannten Bedeutungen haben und L¹ für eine nucleophil verdrängbare Abgangsgruppe steht,
mit Benzoesäure(derivate)n der Formel IV 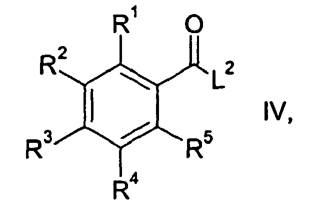 wobei R¹ bis R⁵ die unter Anspruch 1 genannten Bedeutungen haben und L² für eine nucleophil verdrängbare Abgangsgruppe steht,
zu entsprechenden Benzoylderivaten der Formel III 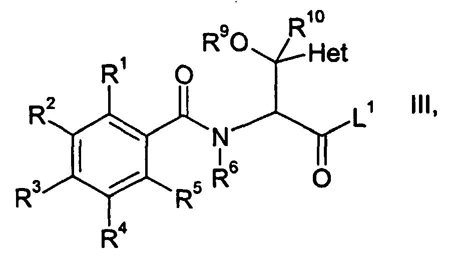 wobei Het, R¹ bis R⁶, R⁹ und R¹⁰ die unter Anspruch 1 genannten Bedeutungen haben und L¹ für eine nucleophil verdrängbare Abgangsgruppe steht,
und anschließend die erhaltenen Benzoylderivate der Formel III mit einem Amin der Formel II
HNR⁷R⁸ II,
wobei R⁷ und R⁸ die unter Anspruch 1 genannten Bedeutungen haben, umgesetzt werden.

7. Verfahren zur Herstellung von benzoylsubstituierten Serin-Amiden der Formel I gemäß Anspruch 6, wobei R⁹ und R¹⁰ für Wasserstoff stehen, **dadurch gekennzeichnet dass** Benzoylderivate der Formel III wobei R⁹ und R¹⁰ für Wasserstoff stehen, durch Acylierung von Ketoverbindungen der Formel XIII 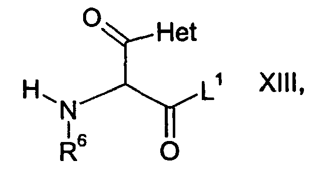 wobei Het und R⁶ die unter Anspruch 1 genannten Bedeutungen haben und L¹ für eine nucleophil verdrängbare Abgangsgruppe steht,
mit Benzoesäure(derivate)n der Formel IV zu N-Acyl-Ketoverbindungen der Formel XII 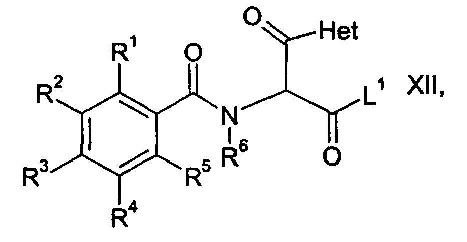 wobei Het sowie R¹ bis R⁶ die unter Anspruch 1 genannten Bedeutungen haben und L¹ für eine nucleophil verdrängbare Abgangsgruppe steht,
und anschließender Reduktion der Ketogruppe hergestellt werden.

8. Benzoylderivate der Formel III 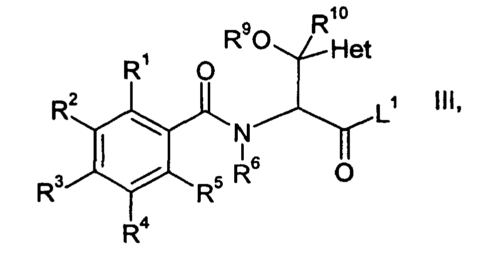 wobei Het, R¹ bis R⁶, R⁹ und R¹⁰ die unter Anspruch 1 genannten Bedeutungen haben und L¹ für eine nucleophil verdrängbare Abgangsgruppe steht.

9. Mittel, enthaltend eine herbizid wirksame Menge mindestens eines benzoylsubstituierten Serin-Amids der Formel I oder eines landwirtschaftlich brauchbaren Salzes von I gemäß den Ansprüchen 1 bis 5 und für die Formulierung von Pflanzenschutzmitteln übliche Hilfsmittel.

10. Verfahren zur Herstellung von Mitteln gemäß Anspruch 9, **dadurch gekennzeichnet, daß** man eine herbizid wirksame Menge mindestens eines benzoylsubstituierten Serin-Amids der Formel I oder eines landwirtschaftlich brauchbaren Salzes von I gemäß den Ansprüchen 1 bis 5 und für die Formulierung von Pflanzenschutzmitteln übliche Hilfsmittel mischt.

11. Verfahren zur Bekämpfung von unerwünschtem Pflanzenwuchs, **dadurch gekennzeichnet, daß** man eine herbizid wirksame Menge mindestens eines benzoylsubstituierten Serin-Amids der Formel I oder eines landwirtschaftlich brauchbaren Salzes von I gemäß den Ansprüchen 1 bis 5 auf Pflanzen, deren Lebensraum und/oder auf Samen einwirken läßt.

12. Verwendung der benzoylsubstituierten Serin-Amide der Formel I und deren landwirtschaftlich brauchbaren Salze gemäß den Ansprüchen 1 bis 5 als Herbizide.

## Claims

1. A benzoyl-substituted serinamide of the formula I 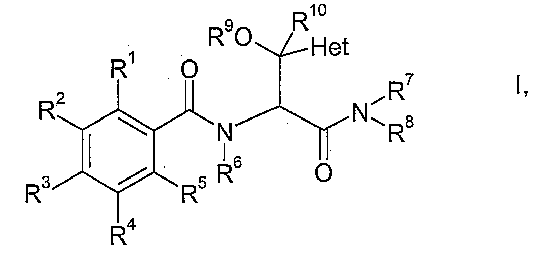 in which the variables are as defined below:
Het is mono- or bicyclic heteroaryl having 5 to 10 ring members comprising 1 to 4 heteroatoms from the group consisting of nitrogen, oxygen and sulfur, which heteroaryl may be partially or fully halogenated and/or may carry 1 to 3 radicals from the group consisting of cyano, nitro, C₁-C₆-alkyl, C₁-C₆-haloalkyl, hydroxyl, C₁-C₆-alkoxy, C₁-C₆-haloalkoxy, hydroxycarbonyl, C₁-C₆-alkoxycarbonyl, hydroxycarbonyl-C₁-C₆-alkoxy, C₁-C₆-alkoxycarbonyl-C₁-C₆-alkoxy, amino, C₁-C₆-alkylamino, di-(C₁-C₆-alkyl)amino, C₁-C₆-alkylsulfonylamino, C₁-C₆-haloalkylsulfonylamino, aminocarbonylamino, (C₁-C₆-alkylamino)carbonylamino, di-(C₁-C₆-alkyl)-aminocarbonylamino, aryl and aryl-(C₁-C₆-alkyl);
R¹ is halogen, cyano, C₁-C₆-alkyl, C₁-C₆-haloalkyl or C₁-C₆-haloalkoxy;
R², R³, R⁴, R⁵ are hydrogen, halogen, cyano, C₁-C₆-alkyl, C₁-C₆-haloalkyl, C₁-C₆-alkoxy or C₁-C₆-haloalkoxy;
R⁶, R⁷ are hydrogen, hydroxyl or C₁-C₆-alkoxy;
R⁸ is C₁-C₆-alkyl, C₁-C₄-cyanoalkyl or C₁-C₆-haloalkyl;
R⁹ is hydrogen, C₁-C₆-alkyl, C₃-C₆-cycloalkyl, C₃-C₆-alkenyl, C₃-C₆-alkynyl,
C₃-C₆-haloalkenyl, C₃-C₆-haloalkynyl, formyl, C₁-C₆-alkylcarbonyl, C₃-C₆-cycloalkylcarbonyl, C₂-C₆-alkenylcarbonyl, C₂-C₆-alkynylcarbonyl, C₁-C₆-alkoxycarbonyl, C₃-C₆-alkenyloxycarbonyl, C₃-C₆-alkynyloxycarbonyl, C₁-C₆-alkylaminocarbonyl, C₃-C₆-alkenylaminocarbonyl, C₃-C₆-alkynylamino-carbonyl, C₁-C₆-alkylsulfonylaminocarbonyl, di-(C₁-C₆-alkyl)aminocarbonyl, N-(C₃-C₆-alkenyl)-N-(C₁-C₆-alkyl)aminocarbonyl, N-(C₃-C₆-alkynyl)-N-(C₁-C₆-alkyl) aminocarbonyl, N-(C₁-C₆-alkoxy)-N-(C₁-C₆-alkyl)aminocarbonyl, N-(C₃-C₆-alkenyl)-N-(C₁-C₆-alkoxy) aminocarbonyl, N-(C₃-C₆-alkynyl) -N-(C₁-C₆-alkoxy) aminocarbonyl, di-(C₁-C₆-alkyl)aminothiocarbonyl, C₁-C₆-alkylcarbonyl-C₁-C₆-alkyl, C₁-C₆-alkoxyimino-C₁-C₆-alkyl, N-(C₁-C₆-alkylamino)imino-C₁-C₆-alkyl, N-(di-C₁-C₆-alkylamino) imino-C₁-C₆-alkyl or tri-C₁-C₄-alkylsilyl,
where the alkyl, cycloalkyl and alkoxy radicals mentioned may be partially or fully halogenated and/or may carry one to three of the following groups: cyano, hydroxyl, C₃-C₆-cycloalkyl, C₁-C₆-alkoxy-C₁-C₄-alkyl, C₁-C₄-alkoxy-C₁-C₄-alkoxy-C₁-C₄-alkyl, C₁-C₄-alkoxy, C₁-C₄-alkylthio, di-(C₁-C₄-alkyl)amino, C₁-C₄-alkyl-C₁-C₆-alkoxy-carbonylamino, C₁-C₄-alkylcarbonyl, hydroxycarbonyl, C₁-C₄-alkoxycarbonyl, aminocarbonyl, C₁-C₄-alkylaminocarbonyl, di-(C₁-C₄-alkyl)aminocarbonyl or C₁-C₄-alkylcarbonyloxy;
phenyl, phenyl-C₁-C₆-alkyl, phenylcarbonyl, phenylcarbonyl-C₁-C₆-alkyl, phenoxycarbonyl, phenylaminocarbonyl, phenylsulfonylaminocarbonyl, N-(C₁-C₆-alkyl)-N-(phenyl)aminocarbonyl, phenyl-C₁-C₆-alkylcarbonyl, heterocyclyl, heterocyclyl-C₁-C₆-alkyl, heterocyclylcarbonyl, heterocyclyl-carbonyl-C₁-C₆-alkyl, heterocyclyloxycarbonyl, heterocyclylaminocarbonyl, heterocyclylsulfonylaminocarbonyl, N-(C₁-C₆-alkyl)-N-(heterocyclyl)aminocarbonyl, or heterocyclyl-C₁-C₆-alkylcarbonyl,
where the phenyl and the heterocyclyl radical of the 17 last-mentioned substituents may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy; or
SO₂R¹¹;
R¹⁰ is hydrogen or C₁-C₆-alkyl;
R¹¹ is C₁-C₆-alkyl, C₁-C₆-haloalkyl or phenyl, where the phenyl radical may be partially or fully halogenated and/or may carry one to three of the following groups: C₁-C₆-alkyl, C₁-C₆-haloalkyl or C₁-C₆-alkoxy;
or an agriculturally useful salt thereof.

2. The benzoyl-substituted serinamide of the formula I according to claim 1 where Het is mono- or bicyclic heteroaryl selected from the group consisting of furyl, thienyl, pyrazolyl, imidazolyl, thiazolyl, triazolyl, tetrazolyl, pyridyl, pyrimidinyl, quinolinyl and indolyl,
where the heteroaryls mentioned may be partially or fully halogenated and/or may carry 1 to 3 radicals from the group consisting of nitro, C₁-C₄-alkyl, C₁-C₄-haloalkyl, hydroxyl, C₁-C₄-alkoxy, C₁-C₄-haloalkoxy, hydroxycarbonyl, C₁-C₆-alkoxycarbonyl, hydroxycarbonyl-C₁-C₆-alkoxy, C₁-C₆-alkoxycarbonyl-C₁-C₆-alkoxy, amino, C₁-C₆-alkylamino, di-(C₁-C₆-alkyl)amino, C₁-C₄-alkylsulfonylamino, C₁-C₄-haloalkyl-sulfonylamino, aminocarbonylamino, (C₁-C₄-alkylamino)carbonylamino and di-(C₁-C₄-alkyl)aminocarbonylamino.

3. The benzoyl-substituted serinamide of the formula I according to claim 1 or 2 where R¹ is halogen or C₁-C₆-haloalkyl.

4. The benzoyl-substituted serinamide of the formula I according to any of claims 1 to 3 where R² and R³ independently of one another are hydrogen, halogen or C₁-C₆-haloalkyl.

5. The benzoyl-substituted serinamide of the formula I according to any of claims 1 to 4 where R⁴, R⁵, R⁶, R⁷ and R¹⁰ are hydrogen.

6. A process for preparing benzoyl-substituted serinamides of the formula I according to claim 1, wherein serine derivatives of the formula V 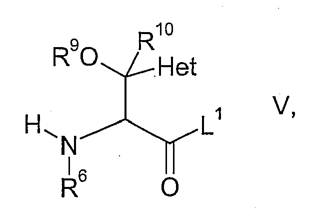 where Het and R⁶, R⁹ and R¹⁰ are as defined in claim 1 and L¹ is a nucleophilically displaceable leaving group
are reacted with benzoic acid (derivatives) of the formula IV 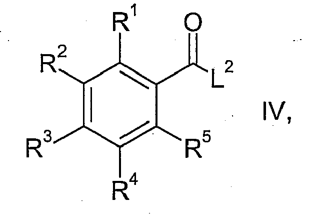 where R¹ to R⁵ are as defined in claim 1 and L² is a nucleophilically displaceable leaving group
to give the corresponding benzoyl derivatives of the formula III 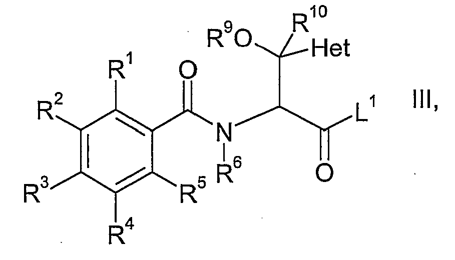 where Het, R¹ to R⁶, R⁹ and R¹⁰ are as defined in claim 1 and L¹ is a nucleophilically displaceable leaving group,
and the resulting benzoyl derivatives of the formula III are then reacted with an amine of the formula II
HNR⁷R⁸ II,
where R⁷ and R⁸ are as defined in claim 1.

7. A process for preparing benzoyl-substituted serinamides of the formula I according to claim 6 where R⁹ and R¹⁰ are hydrogen, wherein benzoyl derivatives of the formula III where R⁹ and R¹⁰ are hydrogen are prepared by acylation of keto compounds of the formula XIII 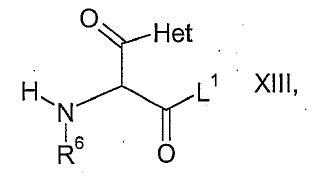 where Het and R⁶ are as defined in claim 1 and L¹ is a nucleophilically displaceable leaving group
with benzoic acid (derivatives) of the formula IV to give N-acyl keto compounds of the formula XII 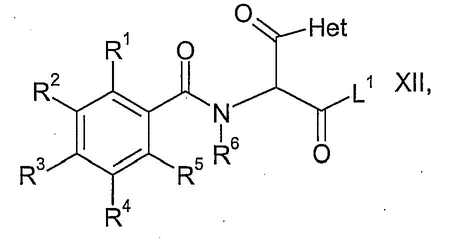 where Het and R¹ to R⁶ are as defined in claim 1 and L¹ is a nucleophilically displaceable leaving group
and subsequent reduction of the keto group.

8. A benzoyl derivative of the formula III 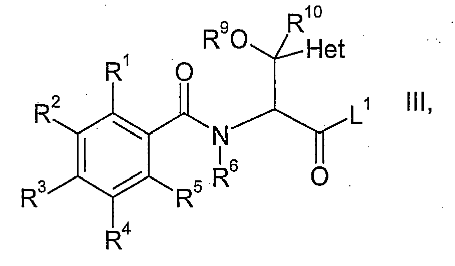 where Het, R¹ to R⁶, R⁹ and R¹⁰ are as defined in claim 1 and L¹ is a nucleophilically displaceable leaving group.

9. A composition, comprising a herbicidally effective amount of at least one benzoyl-substituted serinamide of the formula I or an agriculturally useful salt of I according to any of claims 1 to 5 and auxiliaries customary for formulating crop protection agents.

10. A process for preparing compositions according to claim 9, wherein a herbicidally effective amount of at least one benzoyl-substituted serinamide of the formula I or an agriculturally useful salt of I according to any of claims 1 to 5 and auxiliaries customary for formulating crop protection agents are mixed.

11. A method for controlling unwanted vegetation, wherein a herbicidally effective amount of at least one benzoyl-substituted serinamide of the formula I or an agriculturally useful salt of I according to any of claims 1 to 5 is allowed to act on plants, their habitat and/or on seed.

12. The use of a benzoyl-substituted serinamide of the formula I or an agriculturally useful salt thereof according to any of claims 1 to 5 as a herbicide.

## Revendications

1. Sérine-amides substitués par benzoyle, de formule I 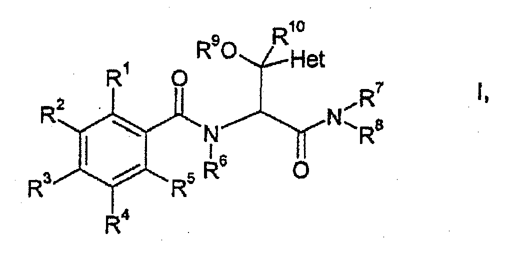 dans laquelle les variables ont les significations suivantes :
Het signifie un groupe hétéroaryle mono- ou bicyclique ayant de 5 à 10 chaînons formant le(s) cycle(s), contenant de 1 à 4 hétéroatomes choisis parmi les atomes d'azote, d'oxygène et de soufre, qui peut être partiellement ou totalement halogéné et/ou peut porter 1 à 3 radicaux choisis dans l'ensemble constitué par les groupes cyano, nitro, alkyle en C₁-C₆, halogénoalkyle en C₁-C₆, hydroxy, alcoxy en C₁-C₆, halogénoalcoxy en C₁-C₆, hydroxycarbonyle, alcoxy(C₁-C₆)carbonyle, hydroxycarbonyl-alcoxy(C₁-C₆), alcoxy(C₁-C₆)carbonyl-alcoxy(C₁-C₆), amino, alkyl(C₁-C₆)amino, dialkyl(C₁-C₆)amino, alkyl(C₁-C₆)sulfonyl-amino, halogénoalkyl(C₁-C₆)-sulfonylamino, aminocarbonylamino, [alkyl(C₁-C₆)amino]-carbonylamino, di [alkyl(C₁-C₆)]-amino-carbonylamino, aryle et aryl-alkyle(C₁-C₆) ;
R¹ représente un atome d'halogène ou un groupe cyano, alkyle en C₁-C₆, halogénoalkyle(C₁-C₆) ou halogénoalcoxy(C₁-C₆) ;
R², R³, R⁴, R⁵ représentent un atome d'hydrogène ou d'halogène, un groupe cyano, alkyle en C₁-C₆, hélogénoalkyle(C₁-C₆), alcoxy en C₁-C₆ ou halogénoalcoxy(C₁-C₆) ;
R⁶, R⁷ représentent un atome d'hydrogène, un groupe hydroxy ou alcoxy en C₁-C₆ ;
R⁸ représente un groupe alkyle en C₁-C₆, cyanoalkyle(C₁-C₄) ou halogénoalkyle(C₁-C₆) ;
R⁹ représente un atome d'hydrogène, un groupe alkyle en C₁-C₆, cycloalkyle en C₃-C₆, alcényle en C₃-C₆, alcynyle en C₃-C₆, halogénoalcényle(C₃-C₆), halogénoalcynyle-(C₃-C₆), formyle, alkyl(C₁-C₆)carbonyle, cycloalkyl(C₃-C₆)carbonyle, alcényl (C₂-C₆-)carbonyle, alcynyl(C₂-C₆)carbonyle, alcoxy(C₁-C₆)carbonyle, alcényloxy(C₃-C₆)-carbonyle, alcynyloxy(C₃-C₆)carbonyle, alkyl(C₁-C₆)amino-carbonyle, alcényl(C₃-C₆)-aminocarbonyle, alcynyl(C₃-C₆)-aminocarbonyle, alkyl(C₁-C₆)sulfonylaminocarbonyle, di[alkyl(C₁-C₆)]-aminocarbonyle, N-[alcényl(C₃-C₆)]-N-[alkyl(C₁-C₆)]-amino-carbonyle, N-[alcynyl(C₃-C₆)]-N-[alkyl-(C₁-C₆)]-aminocarbonyle, N-[alcoxy(C₁-C₆)]-N-[alkyl(C₁-C₆)]-aminocarbonyle, N-[alcényl-(C₃-C₆)]-N-[alcoxy(C₁-C₆)]-aminocarbonyle, N-[alcynyl(C₃-C₆)]-N-[alcoxy(C₁-C₆)]-amino-carbonyle, di[alkyl(C₁-C₆)]-aminothio-carbonyle, alkyl(C₁-C₆)carbonyl-alkyle(C₁-C₆), alcoxy(C₁-C₆)imino-alkyle(C₁-C₆), N-[alkyl(C₁-C₆)amino]-imino-alkyle(C₁-C₆), N-[dialkyl(C₁-C₆)amino]-imino-alkyle(C₁-C₆) ou trialkyl (C₁-C₄)silyle,
les radicaux alkyle, cycloalkyle et alcoxy cités pouvant être partiellement ou totalement halogénés et/ou pouvant porter un à trois des groupes suivants : cyano, hydroxy, cycloalkyle en C₃-C₆, alcoxy(C₁-C₆)-alkyle(C₁-C₄), alcoxy(C₁-C₄)-alcoxy(C₁-C₄)-alkyle(C₁-C₄), alcoxy en C₁-C₄, alkyl(C₁-C₄) thio, di[alkyl(C₁-C₄)]amino, alkyl(C₁-C₄)-alcoxy(C₁-C₆)carbonylamino, alkyl(C₁-C₄)-carbonyle, hydroxycarbonyle, alcoxy(C₁-C₄) carbonyle, aminocarbonyle, alkyl(C₁-C₄) aminocarbonyle, di[alkyl(C₁-C₄)]-aminocarbonyle ou alkyl(C₁-C₄)carbonyloxy ;
phényle, phényl-alkyle(C₁-C₆), phényl-carbonyle, phénylcarbonyl-alkyle(C₁-C₆), phénoxycarbonyle, phénylaminocarbonyle, phénylsulfonylaminocarbonyle, N-[alkyl(C₁-C₆)]-N-(phényl)-aminocarbonyle, phényl-alkyl(C₁-C₆)carbonyle, hétérocyclyle, hétérocyclyl-alkyle(C₁-C₆), hétérocyclyl-carbonyle, hétérocyclylcarbonyl-alkyle(C₁-C₆) hétérocyclyloxycarbonyle, hétérocyclylamino-carbonyle, hétérocyclylsulfonylamino-carbonyle, N-[alkyl(C₁-C₆)]-N-(hétérocyclyl)-aminocarbonyle ou hétérocyclyl-alkyl(C₁-C₆)-carbonyle,
le radical phényle et le radical hétérocyclyle des 17 substituants mentionnés en dernier pouvant être partiellement ou totalement halogénés et/ou pouvant porter un à trois des
substituants suivants : nitro, cyano, alkyle en C₁-C₄, halogénoalkyle(C₁-C₄), alcoxy en C₁-C₄ ou halogénoalcoxy(C₁-C₄) ; ou
SO₂R¹¹;
R¹⁰ représente un atome d'hydrogène ou un groupe alkyle en C₁-C₆ ;
R¹¹ représente un groupe alkyle en C₁-C₆, halogénoalkyle(C₁-C₆) ou phényle,
le radical phényle pouvant être partiellement ou totalement halogéné et/ou pouvant porter un à trois des groupes suivants : alkyle en C₁-C₆, halogénoalkyle en C₁-C₆ ou alcoxy en C₁-C₆: ;
ainsi que leurs sels utilisables en agriculture.

2. Sérine-amides substitués par benzoyle, de formule I selon la revendication 1, dans lesquels Het représente un radical hétéroaryle mono- ou bicyclique choisi dans l'ensemble constitué par les groupes furyle, thiényle, pyrazolyle imidazolyle, thiazolyle, triazolyle, tétrazolyle, pyridyle, pyrimidinyle, quinolinyle et indolyle, les radicaux hétéroaryle cités pouvant être partiellement ou totalement halogénés et/ou pouvant porter 1 à 3 radicaux choisis dans l'ensemble constitué par les groupes nitro, alkyle en C₁-C₄, halogénoalkyle(C₁-C₄), hydroxy, alcoxy en C₁-C₄, halogénoalcoxy(C₁-C₄), hydroxycarbonyle, alcoxy(C₁-C₆)carbonyle, hydroxycarbonyl-alcoxy(C₁-C₆), alcoxy(C₁-C₆)carbonyl-alcoxy(C₁-C₆), amino, alkyl(C₁-C₆)amino, di[alkyl(C₁-C₆)]amino, alkyl(C₁-C₄)-sulfonyl-amino, halogénoalkyl(C₁-C₄)-sulfonylamino, aminocarbonylamino, [alkyl(C₁-C₄)amino]-carbonylamino et di[alkyl(C₁-C₄)]-aminocarbonylamino.

3. Sérine-amides substitués par benzoyle, de formule I selon la revendication 1 ou 2, dans lesquels R¹ représente un atome d'halogène ou un groupe halogénoalkyle(C₁-C₆).

4. Sérine-amides substitués par benzoyle, de formule I selon les revendications 1 à 3, dans lesquels R² et R³ représentent, indépendamment l'un de l'autre, un atome d'hydrogène, un atome d'halogène ou un groupe halogénoalkyle(C₁-C₆).

5. Sérine-amides substitués par benzoyle, de formule I selon les revendications 1 à 4, dans lesquels R⁴, R⁵, R⁶, R⁷ et R¹⁰ représentent un atome d'hydrogène.

6. Procédé pour la préparation des sérine-amides substitués par benzoyle, de formule I selon la revendication 1, **caractérisé en ce qu'**on fait réagir des dérivés de sérine de formule V 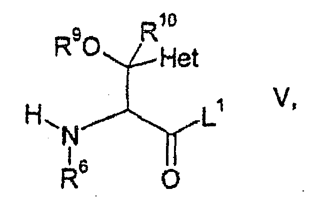 dans laquelle Het ainsi que R⁶, R⁹ et R¹⁰ ont les significations données dans la revendication 1 et L¹ représente un groupe partant séparable par substitution nucléophile,
avec un/des dérivé(s) d'acide benzoïque de formule IV 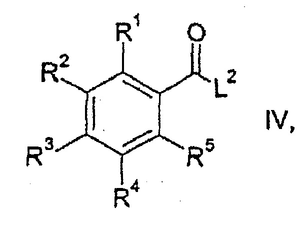 dans laquelle R¹ à R⁵ ont les significations données dans la revendication 1 et L² représente un groupe partant séparable par substitution nucléophile,
pour aboutir aux dérivés benzoyle correspondants de formule III 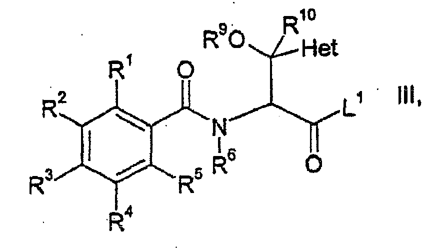 dans laquelle Het, R¹ à R⁶, R⁹ et R¹⁰ ont les significations données dans la revendication 1 et L¹ représente un groupe partant séparable par substitution nucléophile,
et on fait ensuite réagir les dérivés benzoyle de formule III obtenus avec une amine de formule II
HNR⁷R⁸ II,
Où R⁷ et R⁸ ont les significations données dans la revendication 1.

7. Procédé pour la préparation des sérine-amides substitués par benzoyle, de formule I selon la revendication 6, dans lesquels R⁹ et R¹⁰ représentent un atome d'hydrogène, **caractérisé en ce qu'**on prépare des dérivés benzoyle de formule III, dans laquelle R⁹ et R¹⁰ représentent un atome d'hydrogène, par acylation de composés céto de formule XIII 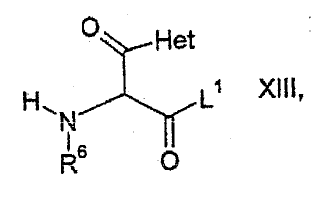 dans laquelle Het et R⁶ ont les significations données dans la revendication 1 et L¹ représente un groupe partant séparable par substitution nucléophile,
avec un/des dérivé(s) d'acide benzoïque de formule IV, pour aboutir aux composés N-acyl-céto de formule XII 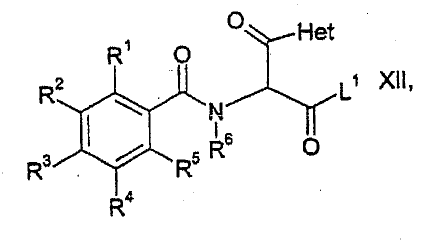 dans laquelle Het ainsi que R¹ à R⁶ ont les significations données dans la revendication 1 et L¹ représente un groupe partant séparable par substitution nucléophile,
et ensuite réduction du groupe céto.

8. Dérivés benzoyle de formule III 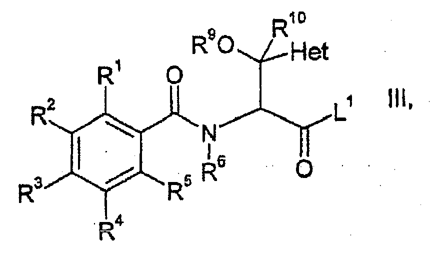 dans laquelle Het, R¹ à R⁶, R⁹ et R¹⁰ ont les significations données dans la revendication 1 et L¹ représente un groupe partant séparable par substitution nucléophile.

9. Compositions contenant une quantité à effet herbicide d'au moins un sérine-amide substituée par benzoyle, de formule I, ou d'un sel de I, utilisable en agriculture, selon les revendications 1 à 5, et des adjuvants usuels pour la formulation de produits phytosanitaires.

10. Procédé pour la préparation de compositions selon la revendication 9, **caractérisé en ce qu'**on mélange une quantité à effet herbicide d'au moins un sérine-amide substitué par benzoyle, de formule I, ou d'un sel de I, utilisable en agriculture, selon les revendications 1 à 5 et des adjuvants usuels pour la formulation de produits phytosanitaires.

11. Procédé pour la lutte contre une croissance indésirable de plantes, **caractérisé en ce qu'**on laisse agir sur des plantes, leur habitat et/ou sur des semences une quantité à effet herbicide d'au moins un sérine-amide substitué par benzoyle, de formule I, ou d'un sel de I, utilisable en agriculture, selon les revendications 1 à 5.

12. Utilisation des sérine-amides substitués par benzoyle, de formule I, et de leurs sels utilisables en agriculture, selon les revendication 1 à 5, en tant qu'herbicides.
